# EUROPÄISCHE PATENTSCHRIFT

(11) **EP 2 582 688 B1**
(45) Veröffentlichungstag und Bekanntmachung des Hinweises auf die Patenterteilung: **07.10.2015**
(21) Anmeldenummer: 11724637.1
(22) Anmeldetag: 10.06.2011
(51) Int. Cl.: C07D 401/04, C07D 401/14, C07D 413/14, C07D 417/14, A01N 43/56, A01N 43/713, A01N 43/76, A01N 43/78

(54) **ANTHRANILSÄUREDIAMID-DERIVATE**
ANTHRANILIC ACID DIAMIDE DERIVATIVES
DÉRIVÉS DE DIAMIDE D'ACIDE ANTHRANILIQUE

(30) Priorität: 15.06.2010 US 354905 P; 15.06.2010 EP 10166064
(43) Veröffentlichungstag der Anmeldung: 24.04.2013
(73) Patentinhaber: Bayer Intellectual Property GmbH, 40789 Monheim am Rhein (DE)
(72) Erfinder: FISCHER, Rüdiger, 50259 Pulheim (DE); GRONDAL, Christoph, 50937 Köln (DE); HEIL, Markus, 42799 Leichlingen (DE); WROBLOWSKY, Heinz-Juergen, 40764 Langenfeld (DE); GESING, Ernst, Rudolf, 40699 Erkrath (DE); FUNKE, Christian, 42799 Leichlingen (DE); MALSAM, Olga, 51503 Rösrath (DE); VOERSTE, Arnd, 50674 Köln (DE); GÖRGENS, Ulrich, 40882 Ratingen (DE); MURATA, Tetsuya, Osaka, 394-1156 (JP); FRANKEN, Eva-Maria, 51381 Leverkusen (DE)
(86) Internationale Anmeldenummer: PCT/EP2011/059698
(87) Internationale Veröffentlichungsnummer: WO 2011/157654

(56) Entgegenhaltungen:
- WO-A1-2007/144100
- WO-A1-2009/147188
- WO-A1-2010/020522
- WO-A2-2004/046129
- WO-A2-2006/033943
- JP-A- 5 202 096
- US-A- 4 877 881
- US-A- 5 234 946
- H. EL KHADEM, ZAKI EL-SHAFEI, LATIF RATEB, HASSAN MOKHTAR: "Synthesis of pyrazoles and oxyquinoxalines from 2,4-dioxohexenoales", JOURNAL OF HETEROCYCLIC CHEMISTRY, Bd. 10, Nr. 1, 1973, Seiten 15-19, XP002606820,
- MOKHTAR, H. ET AL.: "Synthesis of Nitrogenous Compounds from d-Unsaturated 1,3-Dicarbonyl Esters: Trisubstituted Pyrazoles of Possible Antimicrobial and Hypoglycemic Activities and Hydrazones with Antituberculosis Activity", INDIAN JOURNAL OF CHEMISTRY, SECTION B: ORGANIC CHEMISTRY INCLUDING MEDICINAL CHEMISTRY, Bd. 24B, Februar 1985 (1985-02), Seiten 188-192, XP009140504, ISSN: 0376-4699
- MOKHTAR H M: "SYNTHESIS OF TRISUBSTITUTED PYRAZOLES WITH POSSIBLE ANTIMICROBIAL ACTIVITY", PAKISTAN JOURNAL OF SCIENTIFIC AND INDUSTRIAL RESEARCH, PAKISTAN, Bd. 31, Nr. 11, 1. November 1988 (1988-11-01), Seiten 762-767, XP009000302, ISSN: 0030-9885
- MOKHTAR, H. M.; FARAHAT, O. O.: "Synthesis of nitrogenous compounds. Part-IV", PAKISTAN JOURNAL OF SCIENTIFIC AND INDUSTRIAL RESEARCH, Bd. 34, Nr. 1, 1991, Seiten 16-22, XP009140518, ISSN: 0030-9885
- MOKHTAR, HASSAN M.: "Synthesis of nitrogenous compounds. Part II", PAKISTAN JOURNAL OF SCIENTIFIC AND INDUSTRIAL RESEARCH, Bd. 33, Nr. 1-2, 1990, Seiten 30-36, XP009140519, ISSN: 0030-9885
- MOKHTAR, HASSAN M.: "Synthesis of nitrogeneous compounds from .delta.-unsaturated 1,3-dicarbonyl esters. Part I. Substituted pyrazoles, isoxazoles, and oxyquinoxalines", JOURNAL OF THE CHEMICAL SOCIETY OF PAKISTAN, Bd. 10, Nr. 4, 1988, Seiten 414-424, XP009140516, ISSN: 0253-5106
- MOKHTAR, HASSAN M.: "Synthesis of trisubstituted pyrazoles with possible antimicrobial activity", PAKISTAN JOURNAL OF SCIENTIFIC AND INDUSTRIAL RESEARCH, Bd. 28, Nr. 2, April 1985 (1985-04), Seiten 85-91, XP009140520, ISSN: 0030-9885

## Beschreibung

Die vorliegende Erfindung betrifft neue Anthranilsäurederivate, deren Anwendung als Insektizide und Akarizide zur Bekämpfung tierischer Schädlinge, auch in Kombination mit weiteren Mitteln zur Wirkungssteigerung, und mehre Verfahren zu ihrer Herstellung.

Anthranilsäurederivate mit insektiziden Eigenschaften sind in der Literatur bereits beschrieben, z.B. in WO 01/70671, WO 03/015519, WO 03/016284, WO 03/015518, WO 03/024222, WO 03/016282, WO 03/016283, WO 03/062226, WO 03/027099, WO 04/027042, WO 04/033468, WO 2004/046129, WO 2004/067528, WO 2005/118552, WO 2005/077934, WO 2005/085234, WO 2006/023783, WO 2006/000336, WO 2006/040113, WO 2006/111341, WO 2007/006670, WO 2007/024833, WO2007/020877, WO 2007/144100, WO2007/043677, WO2008/126889, WO2008/126890, WO2008/126933

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue Anthranilsäurederivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Anthranilsäurederivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Anthranilsäurederivate der Formel (I) 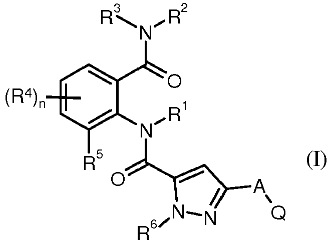 in welcher
- R¹: für Wasserstoff, Amino, Hydroxy oder für jeweils gegebenenfalls einfach oder mehrfach, gleich oder verschieden substituiertes C₁-C₆-Alkyl, C₂-C₆-Alkenyl, C₁-C₆-Alkinyl oder C₃-C₆-Cycloalkyl steht, wobei die Substituenten unabhängig voneinander ausgewählt sein können aus Halogen, Cyano, Nitro, Hydroxy, C₁-C₄-Alkoxy, C₁-C₄-Alkylthio, C₁-C₄-Alkylsulfinyl, C₁-C₄-Alkylsulfonyl, (C₁-C₄-Alkoxy)-carbonyl, C₁-C₄-Alkylamino, Di-(C₁-C₄-alkyl)amino, C₃-C₆-Cycloalkylamino oder (C₁-C₄-Alkyl)C₃-C₆-cycloalkylamino,
- R²: für Wasserstoff, C₁-C₆-Alkyl, C₂-C₆-Alkenyl, C₂-C₆-Alkinyl, C₃-C₆-Cycloalkyl, C₁-C₄-Alkoxy, C₁-C₄-Alkylamino, Di-(C₁-C₄-alkyl)amino, C₃-C₆-Cycloalkylamino, C₁-C₆-Alkoxycarbonyl oder C₁-C₆-Alkylcarbonyl steht,
- R³: für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkyl-sulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₁-C₆-Alkoxycarbonyl, C₁-C₆-Alkylcarbonyl oder C₃-C₆-Trialkylsilyl,
- R³: weiterhin für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Amino, C₃-C₆-Cycloalkylamino oder einem 5- oder 6-gliedrigen heteroaromatischen Ring,
- R³: ebenfalls weiterhin für C₃-C₁₂-Cycloalkyl, C₃-C₁₂-Cycloalkyl-C₁-C₆-Alkyl und C₄-C₁₂-Bicycloalkyl steht, wobei die Substituenten unabhängig voneinander ausgewählt sein können aus Halogen, Cyano, Nitro, Hydroxy, Amino, C₁-C₆-Alkyl, C₃-C₆-Cycloalkyl, C₃-C₆-Cycloalkylamino, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₁-C₆-Alkoxycarbonyl, C₁-C₆-Alkylcarbonyl, C₃-C₆-Trialkylsilyl oder einem 5- oder 6-gliedrigen heteroaromatischen Ring,
- R² und R³: miteinander über zwei bis sechs Kohlenstoffatome verbunden sein können und einen Ring ausbilden, der gegebenenfalls zusätzlich ein weiteres Stickstoff-, Schwefel- oder Sauerstoffatom enthält und gegebenenfalls einfach bis vierfach mit C₁-C₂-Alkyl, Halogen, Cyano, Amino oder C₁-C₂-Alkoxy substituiert sein kann,
- R², R³: weiterhin gemeinsam für =S(C₁-C₄-Alkyl)₂, =S(O)(C₁-C₄-Alkyl)₂, stehen,
- R⁴: für Wasserstoff, Halogen, Cyano, Nitro, Hydroxy, Amino, (C=O)OH, OCN, SCN, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Alkoxy-C₁-C₄-alkyl, C₁-C₄-Haloalkoxy, SF₅, C₁-C₄-Alkylthio, C₁-C₄-Alkylthio-C₁-C₄-alkyl, C₁-C₄-Alkylsulfinyl, C₁-C₄-Alkylsulfonyl, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, N-Methoxy-N-methylamino, C₁-C₄-Alkoxycarbonyl, C₁-C₄-Alkoxycarbonyl-C₁-C₄-alkyl, C₁-C₄-Alkylcarbonyl, C₁-C₄-Alkylcarbonyloxy, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₃-C₆-Cycloalkylamino, (C₁-C₄-Alkoxy)imino, (C₁-C₄-Alkyl)(C₁-C₄-Alkoxy)imino, (C₁-C₄-Haloalkyl)(C₁-C₄-Alkoxy)imino, C₁-C₄-Alkylsulfonylamino, Aminosulfonyl, C₁-C₄-Alkylaminosulfonyl, C₁-C₄-Dialkylaminosulfonyl, C₁-C₄-Alkylsulfoximino, C₃-C₆-Trialkylsilyl steht oder für einen 3- bis 6-gliedrigen gesättigten, teilgesättigten oder aromatischen Ring steht, der gegebenenfalls ein bis drei Heteroatome aus der Reihe O, S oder N enthatlen kann und der gegebenenfalls einfach oder mehrfach, gleich oder verschieden substituiert ist mit Halogen, Cyano, Nitro, Hydroxy, Amino, Carboxy, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, C₁-C₄-Alkoxycarbonyl, C₁-C₄-Alkylcarbonyl, C₁-C₄-Alkylcarbonyloxy, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₃-C₆-Cycloalkylamino, C₁-C₄-Alkylsulfonylamino, Aminosulfonyl, C₁-C₄-Alkylaminosulfonyl oder C₁-C₄-Dialkylaminosulfonyl,
zwei R⁴ über benachbarte Kohlenstoffatome einen Ring ausbilden, der für -(CH₂)₃-, -(CH₂)₄-, -(CH₂)₅-,-(CH=CH-)₂-, -OCH₂O-, -O(CH₂)₂O-, -OCF₂O-, -(CF₂)₂O-, -O(CF₂)₂O-, -(CH=CH-CH=N)- oder -(CH=CH-N=CH)- steht, oder
zwei R⁴ weiterhin über benachbarte Kohlenstoffatome die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Cyano, C₁-C₆-Alkyl, C₃-C₆-Cycloalkyl, C₁-C₆-Haloalkyl, C₃-C₆-Halocycloalkyl, Halogen, C₁-C₆-Alkoxy, C₁-C₄-Alkylthio(C₁-C₆-alkyl), C₁-C₄-Alkylsulfinyl(C₁-C₆-alkyl), C₁-C₄-Alkylsulfonyl(C₁-C₆-alkyl), C₁-C₄-Alkylamino, Di-(C₁-C₄-alkyl)amino oder C₃-C₆-Cycloalkylamino, 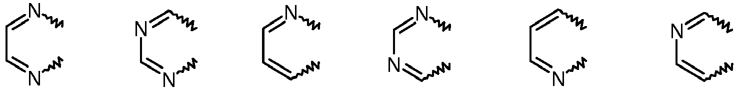 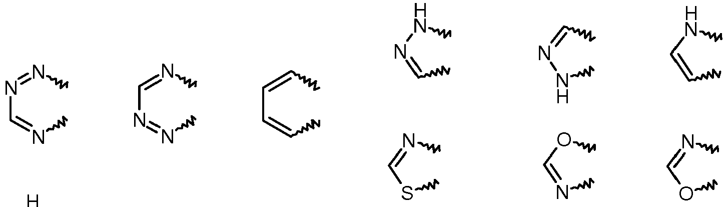 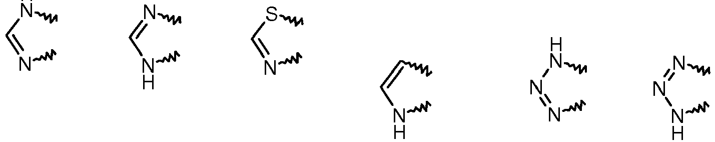 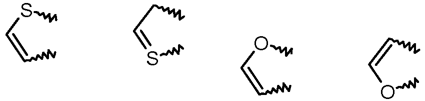
- n: für 0 bis 3 steht,
- R⁵: für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalkenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl steht,
- A: für - C(=O)-, -CH₂(CO)-, CH₂CH(OH)-, -C(=O)-C(=O)-, -CH= CH-, -C≡C-, Isoxazolin, Imidazolidon, -CH₂NHSO₂CH₂-, -CH₂NMeSO₂CH₂-, CH₂N(SO₂Me)CH₂-, -(CO)CH₂-, -CHCl-, -CCl₂-, -CHF-, -CF₂- steht,
- R⁸: für linear oder verzweigtes -(C₁-C₆-alkylen)- steht oder für eine direkte Bindung steht,
wobei mehrere R⁸ unabhängig voneinander für linear oder verzweigtes-(C₁-C₆-alkylen)- oder für eine direkte Bindung stehen,
beispielsweise steht R⁸-O-R⁸- für -(C₁-C₆-alkylen)-O-(C₁-C₆-alkylen)-, -(C₁-C₆-alkylen)-O-, -O-(C₁-C₆-alkylen)-, oder -O-,
- Qz: für einen 3- bis 4-gliedrigen, teilweise gesättigten oder gesättigten, oder für einen 5 bis 6-gliedrigen, teilweise gesättigten, gesättigten oder aromatischen Ring steht oder für ein 6 bis 10-gliedriges bizyklisches Ringsystem steht,
wobei der Ring oder das bizyklische Ringsystem gegebenenfalls 1-3 Heteroatome aus der Reihe N, S, O enthalten kann,
wobei der Ring oder das bizyklische Ringsystem gegebenenfalls einfach oder mehrfach, gleich oder verschieden substituiert ist, und wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₂-C₆-Haloalkyl, C₂-C₆-Haloalkenyl, C₂-C₆-Haloalkinyl, C₃-C₆-Halocycloalkyl, Halogen, CN, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, (=O) oder (=O)₂,
- Q: für Phenyl steht, welches einfach oder mehrfach durch R¹⁰ substituiert ist, oder für einen 5-oder 6-gliedrigen, teilweise gesättigten oder gesättigten heterozyklischen oder heteroaromatischen Ring, oder ein aromatisches 8-, 9- oder 10-gliedriges annelliertes heterobicyclisches Ringsystem steht, wobei der Ring oder das Ringsystem gegebenenfalls einfach oder mehrfach, gleich oder verschieden durch R¹⁰ substituiert ist,
- R¹⁰: für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,
wobei R¹⁰ nicht für Wasserstoff steht, falls A für -R⁸-C(=O)-R⁸ steht und Q für Phenyl steht,
- R⁶: für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₃-C₆-Cycloalkoxy oder 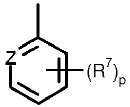 steht,
R⁷ unabhängig voneinander für Wasserstoff, C₁-C₆-Alkyl, C₃-C₆-Cycloalkyl, C₁-C₆-Haloalkyl, Halogen, Cyano, Nitro, C₁-C₄-Alkoxy, C₁-C₄-Haloalkoxy, C₁-C₄-Alkylthio oder C₁-C₄-Haloalkylthio steht,
p für 0 bis 4 steht,
Z für N, CH, CF, CCl, CBr oder CI steht,
die Verbindungen der allgemeinen Formel (I) außerdem N-Oxide und Salze umfassen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der Formel (I) umfassen gegebenenfalls Diastereomere oder Enantiomere.

Die erfindungsgemäßen Verbindungen sind durch die Formel (I) allgemein definiert. Bevorzugt, besonders bevorzugt und ganz besonders sind Verbindungen der Formel (I), in welcher
- R¹: bevorzugt für Wasserstoff, C₁-C₆-Alkyl, C₂-C₆-Alkenyl, C₂-C₆-Alkinyl, C₃-C₆-Cycloalkyl, Cyano(C₁-C₆-alkyl), C₁-C₆-Haloalkyl, C₂-C₆-Haloalkenyl, C₂-C₆-Haloalkinyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl oder C₁-C₄-Alkylsulfonyl-C₁-C₄-alkyl steht,
- R¹: besonders bevorzugt für Wasserstoff, Methyl, Cyclopropyl, Cyanomethyl, Methoxymethyl, Methylthiomethyl, Methylsulfinylmethyl oder Methylsulfonylmethyl steht,
- R¹: ganz besonders bevorzugt für Wasserstoff steht,
- R²: steht bevorzugt für Wasserstoff oder C₁-C₆-Alkyl.
- R²: steht besonders bevorzugt für Wasserstoff oder Methyl.
- R²: steht ganz besonders bevorzugt für Wasserstoff.
- R³: steht bevorzugt für Wasserstoff oder für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkyl-sulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht weiterhin bevorzugt für C₃-C₁₂-Cycloalkyl und C₄-C₁₀-Bicycloalkyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht besonders bevorzugt für Wasserstoff oder für jeweils gegebenenfalls einfach oder mehrfach, gleich oder verschieden substituiertes C₁-C₆-Alkyl, C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Tri-alkylsilyl,
- R³: steht weiterhin besonders bevorzugt für gegebenenfalls einfach oder mehrfach, gleich oder verschieden substituiertes C₃-C₆-Cycloalkyl wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
- R³: steht ganz besonders bevorzugt für C₁-C₄-Alkyl (Methyl, Ethyl, n-Propyl, iso-Propyl, n-Butyl, isoButyl, sec-Butyl oder tert-Butyl) oder Cyano-C₁-C₃-Alkyl (Cyanomethyl, 1-Cyanoethyl, 2-Cyanoethyl, 1-Cyano-n-Propyl, 2-Cyano-n-Propyl, 3-Cyano-n-Propyl, 1-Cyano-iso-Propyl, 2-Cyano-iso-Propyl).
- R³: steht insbesondere bevorzugt für Methyl, iso-Propyl, tert-Butyl oder Cyanomethyl.
- R⁴: bevorzugt für Wasserstoff, C₁-C₄-Alkyl, C₁-C₄-Haloalkyl, Halogen, Cyano, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, Nitro, Hydroxy, Amino, (C=O)OH, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, C₁-C₄-Alkoxycarbonyl, C₁-C₄-Alkylcarbonyl, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₁-C₄-Alkylsulfonylamino, Aminosulfonyl, C₁-C₄-Alkylaminosulfonyl oder C₁-C₄-Dialkylaminosulfonyl steht,
zwei benachbarte Reste R⁴ ebenfalls bevorzugt für -(CH₂)₃-, -(CH₂)₄-, -(CH₂)₅-, -(CH=CH-)₂-, -OCH₂O-, - O(CH₂)₂O-, -OCF₂O-, -(CF₂)₂O-, -O(CF₂)₂O-, -(CH=CH-CH=N)- oder -(CH=CH-N=CH)- stehen,
- R⁴: besonders bevorzugt für Wasserstoff, C₁-C₄-Alkyl, C₁-C₂-Haloalkyl, Halogen, Cyano oder C₁-C₂-Haloalkoxy. steht
zwei benachbarte Reste R⁴ besonders bevorzugt für -(CH₂)₄-, -(CH=CH-)₂-, -O(CH₂)₂O-, -O(CF₂)₂O-, - (CH=CH-CH=N)- oder -(CH=CH-N=CH)- stehen,
- R⁴: steht ganz besonders bevorzugt für Wasserstoff, Methyl, Trifluormethyl, Cyano, Fluor, Chlor, Brom, Iod oder Trifluormethoxy. Ganz besonders bevorzugt stehen ausserdem zwei benachbarte Reste R⁴ für -(CH₂)₄-, oder -(CH=CH-)₂-.
- R⁴: steht insbesondere bevorzugt für Chlor oder Brom,
- R⁴: steht weiterhin insbesondere bevorzugt für Iod oder Cyano.
zwei benachbarte Reste R⁴ stehen insbesondere bevorzugt für -(CH=CH-)₂
- R⁵: bevorzugt für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl, Halogen, Cyano, Nitro oder C₃-C₆-Trialkylsilyl steht,
- R⁵: besonders bevorzugt für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
- R⁵: ganz besonders bevorzugt für Methyl, Fluor, Chlor, Brom oder Iod steht,
- R⁵: insbesondere bevorzugt für Methyl oder Chlor steht,
- A: für -C(=O)-, -CH₂(CO)-, CH₂CH(OH)-, -C(=O)-C(=O)-, -CH=CH-,-C=C-, Isoxazolin, Imidazolidon, -CH₂NHSO₂CH₂-, -CH₂NMeSO₂CH₂-, CH₂N(SO₂Me)CH₂- ,-(CO)CH₂-, -CHCl-, -CCl₂-, -CHF-, -CF₂- steht,
- Qz: bevorzugt für einen 3- bis 4-gliedrigen, teilweise gesättigten oder gesättigten, oder für einen 5 bis 6-gliedrigen, teilweise gesättigten, gesättigten oder aromatischen Ring steht,wobei der Ring gegebenenfalls 1-3 Heteroatome aus der Reihe N,S,O enthalten kann,
wobei der Ring gegebenenfalls einfach oder mehrfach, gleich oder verschieden substituiert ist, und wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl,
- Qz: besonders bevorzugt für einen 3- bis 4-gliedrigen, teilweise gesättigten oder gesättigten, oder für einen 5 bis 6 gliedrigen, teilweise gesättigten, gesättigten oder aromatischen Ring steht, wobei der Ring gegebenenfalls 1-2 Heteroatome aus der Reihe N,S,O enthalten kann,
wobei der Ring gegebenenfalls einfach oder mehrfach, gleich oder verschieden substituiert ist, und wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl,
- Qz: ganz besonders bevorzugt für Phenyl, Furan, Thiophen, Imidazol, Thiazol, Oxazol, Pyridin, Pyrimidin, Azetidin, Oxetan, Thietan, Pyrrolidin, Pyrrolin, Pyrazolidin, Pyrazolin, Imidazolidin, Imidazolin, Isoxazolin, Piperidin, Piperazin, Pyrrolidon, Pyrrolidinon, Imidazolidon, Imidazolidinon, Triazolinon, Triazolidinon, Tetrazolinon, Tetrazolidinon, Thiazolon, Thiazolidinon, Oxazolon, Oxazolidinon steht,
- R⁶: bevorzugt für C₁-C₆-Alkyl steht oder für den Rest 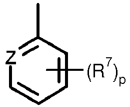 steht,
R⁶ weiterhin bevorzugt für C₃-C₆-Cycloalkoxy steht,
R⁶ besonders bevorzugt für Methyl steht oder für den Rest 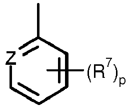 steht,
- R⁷: unabhängig voneinander bevorzugt für Wasserstoff, Halogen, Cyano, C₁-C₄-Alkyl, C₁-C₄-Alkoxy, C₁-C₄-Haloalkyl, C₁-C₄-Haloalkoxy, C₁-C₄-Haloalkylsulfonyl oder (C₁-C₄-Alkyl)C₁-C₄-Alkoxyimino steht,
- R⁷: unabhängig voneinander besonders bevorzugt für Wasserstoff, Halogen oder C₁-C₄-Haloalkyl steht,
- R⁷: ganz besonders bevorzugt für Fluor, Chlor oder Brom steht,
- R⁷: insbesondere bevorzugt für Chlor oder Brom steht,
- p: bevorzugt für 1, 2 oder 3 steht,
- p: besonders bevorzugt für 1 oder 2 steht,
- p: ganz besonders bevorzugt für 1 steht,
- Z: bevorzugt für N, CH, CF, CCl, CBr oder CI steht,
- Z: besonders bevorzugt für N, CH, CF, CCl oder CBr steht,
- Z: ganz besonders bevorzugt für N, CCl oder CH steht,
- R⁸: bevorzugt für linear oder verzweigtes -(C₁-C₄-alkylen)-oder für eine direkte Bindung steht
- R⁸: besonders bevorzugt für Methylen, Ethylen, Propylen, iso-Propylen, n-Butylen, sec-Butylen, oder iso-Butylen oder eine direkte Bindung steht
- R⁸: ganz besonders bevorzugt für Methylen oder Ethylen oder eine direkte Bindung steht
- R¹⁰: bevorzugt für Wasserstoff, C₁-C₃-Alkyl, C₁-C₃-Haloalkyl, C₁-C₂-Alkoxy, Halogen, Cyano, Hydroxy, Nitro, C₁-C₂-Haloalkoxy oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy oder C₁-C₄-Haloalkoxy substituiert sein können,
wobei R¹⁰ nicht für Wasserstoff steht, falls A für R⁹-O-R⁹- oder -R⁸-C(=O)-R⁸ steht und Q für Phenyl steht,
- R¹⁰: besonders bevorzugt für Wasserstoff, C₁-C₃-Alkyl, C₁-C₃-Haloalkyl, C₁-C₂-Alkoxy, Halogen, Cyano, Hydroxy, C₁-C₂-Haloalkoxy oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₃-Alkyl, C₂-C₄-Alkenyl, C₂-C₄-Alkinyl, C₃-C₆-Cycloalkyl, C₁-C₃-Haloalkyl, C₂-C₄-Haloalkenyl, C₂-C₄-Haloalkinyl, C₃-C₆-Halocycloalkyl, Halogen, Cyano, C₁-C₄-Alkoxy, C₁-C₄-Haloalkoxy substituiert sein können,
wobei R¹⁰ nicht für Wasserstoff steht, falls A für -R⁸-C(=O)-R⁸ steht und Q für Phenyl steht,
- R¹⁰: ganz besonders bevorzugt für Methyl, Ethyl, cyclo-Propyl, tert-Butyl, Chlor, Fluor, Iod, Brom, Cyano, Difluormethyl, Trifluormethyl, Pentafluorethyl., n-Heptafluorpropyl, iso-Heptafluorpropyl oder für Phenyl oder einen 5- oder 6-gliedrigen heteroaromatischen Ring steht, wobei die Substituenten unabhängig voneinander ausgewählt sein können aus Methyl, Ethyl, cyclo-Propyl, tert-Butyl, Chlor, Fluor, Iod, Brom, Cyano, Difluormethyl, Trifluormethyl, Pentafluorethyl., n-Heptafluorpropyl oder iso-Heptafluorpropyl,
- Q: bevorzugt für einfach oder mehrfach durch R¹⁰ substituiertes Phenyl oder für einen 5-oder 6-gliedrigen, teilweise gesättigten oder gesättigten heterozyklischen oder heteroaromatischen Ring oder ein aromatisches 8-, 9- oder 10-gliedriges annelliertes heterobicyclisches Ringsystem steht, wobei die Heteroatome ausgewählt sein können aus der Reihe N, S, O, wobei der Ring oder das Ringsystem gegebenenfalls einfach oder mehrfach, gleich oder verschieden durch R¹⁰ substituiert ist,
- Q: besonders bevorzugt für einfach oder mehrfach durch R¹⁰ substituiertes Phenyl oder für einen gegebenenfalls einfach oder mehrfach durch R¹⁰ substituierten 5- oder 6-gliedrigen heteroaromatischen Ring der Reihe Q-1 bis Q-53 und Q-58 bis Q-59, Q62 bis Q63, einen aromatisches 9-gliedriges annelliertes heterobicyclisches Ringsystem Q-54 bis Q-56 sowie für ein 5-gliedrigen heterocyclischen Ring Q-60 bis Q-61 steht,
- Q: ganz besonders bevorzugt für einfach oder mehrfach durch R¹⁰ substituiertes Phenyl oder für einen einfach oder mehrfach durch R¹⁰ substituierten heteroaromatischen Ring der Reihe Q- 6, Q 41, Q42, Q58, Q59, Q62 oder Q63 steht

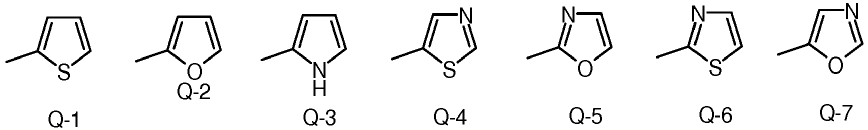

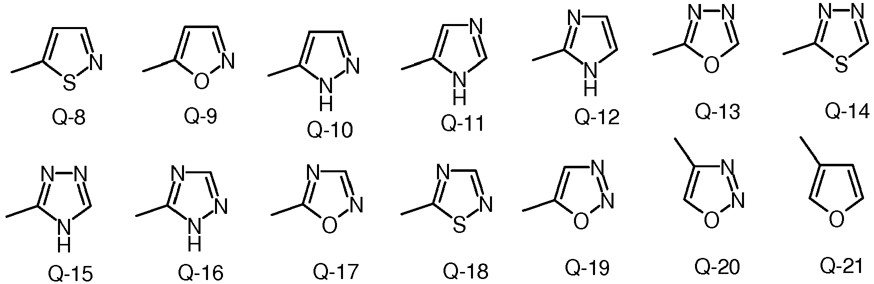

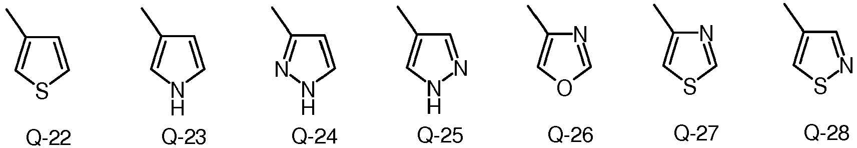

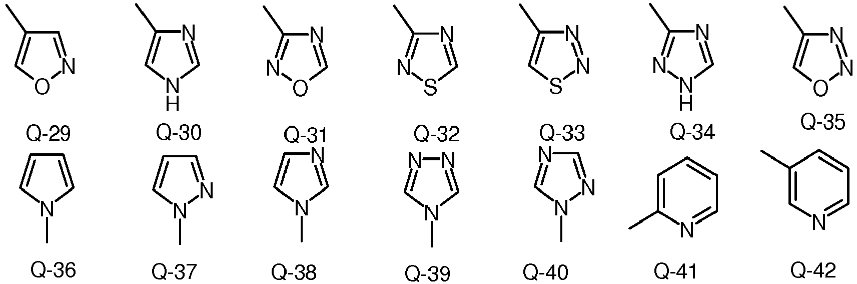

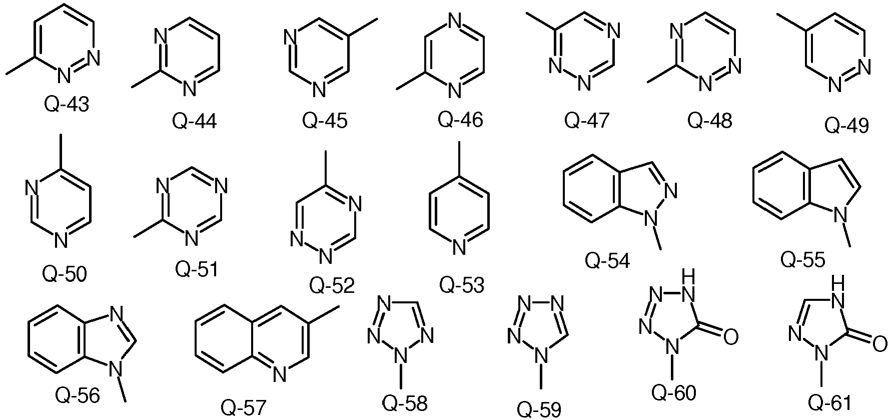

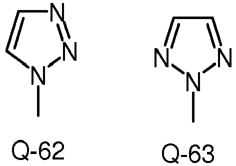

Die oben aufgeführten Ringe oder Ringsysteme können gegebenenfalls unabhängig voneinander zusätzlich durch Oxo, Thioxo, (=O)=NH, (=O)=N-CN, (=O)₂ substituiert sein. Beispielhaft seien genannt Tetrahydrothiophendioxid, Imidazolidon.

Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen -N(O)-, -S(O)- (auch kurz SO) und -S(O)₂- (auch kurz SO₂) im heterocyclischen Ring. Im Fall von -N(O)- und -S(O)-Gruppen sind jeweils beide Enantiomere umfasst.

Andere Substituenten als die Oxogruppe können an einem heterocyclischen Ring auch an einem Heteroatom gebunden sein, beispielsweise an einem Stickstoffatom, wenn dabei ein Wasserstoffatom am Stickstoffatom des Grundkörpers ersetzt wird. Im Falle des Stickstoffatoms und auch anderer Heteroatome wie z. B. des Schwefelatoms, kommt auch eine weitere Substitution unter Bildung von quartären Ammoniumverbindungen oder Sulfoniumverbindungen in Frage.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Insbesondere können die Verbindungen der Formeln (I) in Form verschiedener Regioisomere vorliegen. Beispielsweise in Form von Mischungen aus Verbindungen mit der Definition Q62 bzw. Q63 oder in Form von Mischungen aus Q58 und 59. Erfindungsgemäß umfasst sind daher auch Mischungen aus Verbindungen der Formeln (I), wobei Q die Bedeutungen Q62 und Q63, sowie Q58 und Q59 hat und die Verbindungen in verschiedenen Mischungsverhältnissen vorliegen können. Bevorzugt sind dabei Mischungsverhältnisse aus Verbindungen der Formel (I), worin der Rest Q für Q62 oder für Q58 steht zu Verbindungen der Formel (I) worin der Rest Q für Q63 oder für Q59 steht, von 60:40 bis 99:1, besonders bevorzugt von 70:30 bis 97:3, ganz besonders bevorzugt von 80:20 bis 95:5. Insbesonders bevorzugt sind die folgenden Mischungsverhältnisse einer Verbindung der Formel (I), wobei Q die Bedeutung Q62 oder Q58 hat zur Verbindung der Formel (I), wobei Q die Bedeutung Q63 oder Q59 hat: 80:20; 81:19; 82:18; 83:17; 84:16; 85:15, 86:14; 87:13; 88:12; 89:11; 90:10, 91:9; 92:8; 93:7; 96:6; 95;5.

### Herstellverfahren

Anthranilamide der Formel (I) 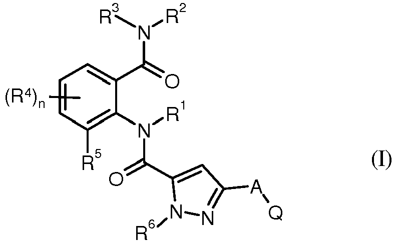 in welcher A, R¹, R², R³, R⁴, R⁵, R⁶, Q und n die oben angegebenen Bedeutungen haben, werden erhalten, indem man
(A) Aniline der Formel (II) 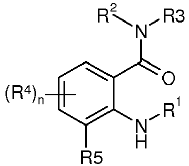 in welcher R¹, R², R³, R⁴, R⁵ und n die oben angegebenen Bedeutungen haben,
   beispielsweise mit einer Carbonsäure der Formel (III) 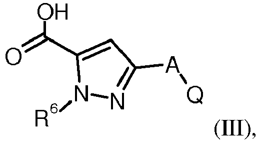 wobei
   Q, A und R⁶ die oben angegebenen Bedeutungen haben,
   in Gegenwart eines Kondensationsmittels umsetzt; oder
(B) Aniline der Formel (II) 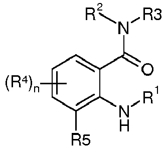 in welcher R¹, R², R³, R⁴, R⁵ und n die oben angegebenen Bedeutungen haben
   beispielsweise mit Carbonsäurechloriden der Formel (IIIA) 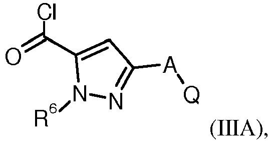 wobei

   Q, A und R⁶ die oben angegebenen Bedeutungen haben, in Gegenwart eines Säurebindungsmittels umsetzt; oder
(C) zur Synthese von Anthranilamiden der Formel (I), in welcher R¹ für Wasserstoff steht, beispielsweise Benzoxazinone der Formel (IIIB) 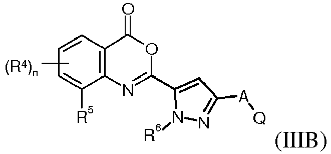 in welcher R⁴, R⁵, R⁶, A, Q und n die oben angegebenen Bedeutungen haben,
   mit einem Amin der Formel (IIIC) 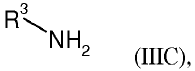 in welcher R³ die oben angegebenen Bedeutungen hat,
   in Gegenwart eines Verdünnungsmittels umsetzt zu erfindungsgemäßen Verbindungen der Formel (I).

### Erläuterung der Herstellverfahren und Zwischenprodukte

### Carbonsäuren der Formel (III-1)

Carbonsäuren der Formel (III-1) sind neu. Sie können nach dem folgenden Reaktionsschema, in welchem X für für eine Mesitylgruppe oder Halogen, R für Wasserstoff oder Alkyl und B für eine Methylengruppe oder eine direkte Bindung steht, hergestellt werden. Die dabei verwendeten Sulfonamide wurden nach bekannten Methoden hergestellt (z. B. WO2006/097292). Die Umsetzung der Verbindungen der Formel V zu Verbindungen der Formel III-1 kann nach bekannten Methoden durchgeführt werden (z. B. WO2007/144100).

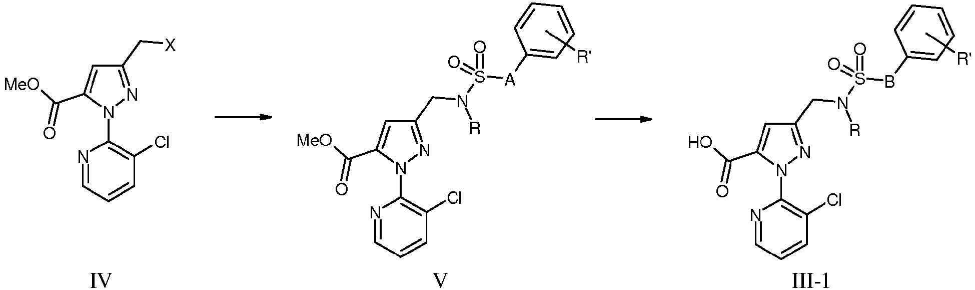

### Carbonsäuren der Formel (III-2) und (III-3)

Carbonsäuren der Formel III-2 und III-3 sind neu. Sie können nach dem folgenden Reaktionsschema, in welchem R⁶ und Q die oben angegebenen Bedeutungen haben und R für C₁-C₆ Alkyl steht aus Verbindungen der Formel VI hergestellt werden. Verbindungen der Formel VI sind bekannt (z.B. WO 2003016283). Die Umsetzung von VI zu VII kann nach bekannten Methoden durchgeführt werden wie z.B. mit einem geeigneten substituierten Alkin in Gegenwart von Kupfer(I)iodid und Tetrakis(triphenylphosphin)palladium (z.B. Heterocycles, 78(1), 71-91; 2009). Die weitere Umsetzung des Alkinderivates VII zum Diketon VIII erfolgt mit einem geeigneten Oxidationsmittel wie z.B. Kaliumpermanganat (vgl. J. Med. Chem. 48(7), 2270-2273; 2005). Die Verseifung zu III-2 und III-3 erfolgt nach bekannten Methoden (z.B. WO2006004903).

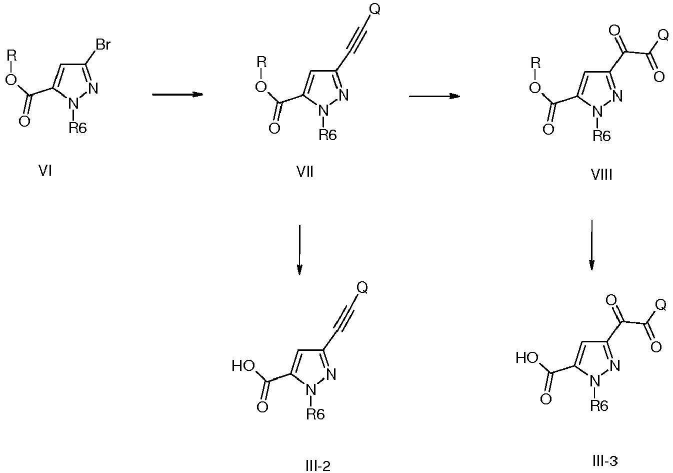

### Carbonsäuren der Formel III-4

Carbonsäuren der Formel III-4 sind neu. Sie können nach dem folgenden Reaktionsschema, in welchem R⁶ und Q die oben angegebenen Bedeutungen haben und R für C₁-C₆ Alkyl steht aus Verbindungen der Formel VI hergestellt werden. Verbindungen der Formel VI sind bekannt (z.B. WO 2003016283). Die Umsetzung von VI zu VII-a kann nach bekannten Methoden durchgeführt werden wie z.B. mit (1-Ethoxyvinyl)-tributylstannan unter Palladiumkatalyse (z.B. J. Med. Chem. 41, 1998, 3736). Die weitere Umsetzung zu VIII-a erfolgt mit einem geeigneten Halogenierungsmittel (z.B Bioorganic & Medicinal Chemistry Letters, 19(4), 1199-1205; 2009). Die Kupplung mit Q zu IX und die Verseifung zu III-4 erfolgen nach bekannten Methoden (z.B. Bioorganic & Medicinal Chemistry, 17(6), 2410-2422; 2009 und WO2006004903).

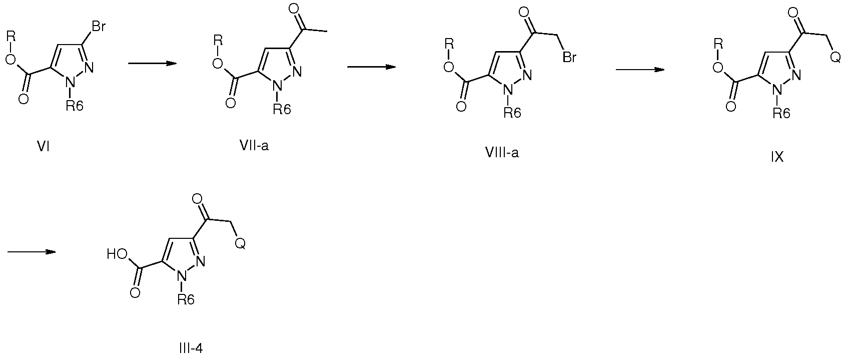

### Carbonsäuren der Formel III-5

Carbonsäuren der Formel III-5 sind neu. Sie können nach dem folgenden Reaktionsschema, in welchem R⁶ und Q die oben angegebenen Bedeutungen haben und R für C₁-C₆ Alkyl steht aus Verbindungen der Formel VI hergestellt werden. Verbindungen der Formel VI sind bekannt (z.B. WO 2003016283). Die Umsetzung von VI zu VII-b kann nach bekannten Methoden durchgeführt werden wie z.B. mit einem geeigneten Diamin Liganden unter Kupferkatalyse (z.B. J. Org. Chem. 69, 2004, 5578-5587). Die Verseifung zu III-5 erfolgt nach bekannten Methoden (z.B. WO2006004903).

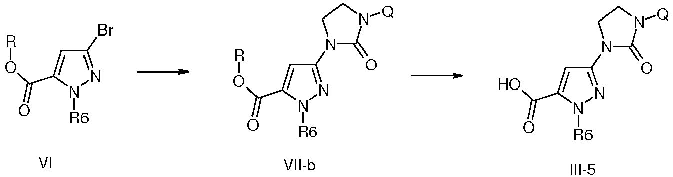

### Carbonsäuren der Formel III-6

Carbonsäuren der Formel III-6 sind neu. Sie können nach dem folgenden Reaktionsschema, in welchem R⁶ und Q die oben angegebenen Bedeutungen haben und R für C₁-C₆ Alkyl steht aus Verbindungen der Formel VI-c hergestellt werden. Verbindungen der Formel VI-c sind bekannt (z.B. WO 2004078732). Die Umsetzung von VI-c zu VII-c kann nach bekannten Methoden durchgeführt werden wie z.B. mit einem geigneten Zinn-Reagenz in Gegenwart eines Phoshin-Liganden und einem Palladium Katalysator (z.B. Tetrahedron Lett. 45, 2004, 3797-3801). Die Oxidation zu VIII-c erfolgt mit einem geeigneten Oxidationsmittel wie z.B. Mangandioxid (z.B WO 9800385). Die Verseifung zu III-6 erfolgt nach bekannten Methoden (z.B. WO2006004903).

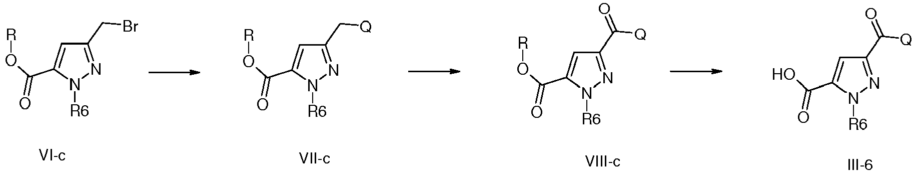

### Carbonsäuren der Formel III-7

Carbonsäuren der Formel III-7 sind neu. Sie können nach dem folgenden Reaktionsschema, in welchem R6 und Q die oben angegebenen Bedeutungen haben und R für C₁-C₆-Alkyl steht aus Verbindungen der Formel VI-d hergestellt werden. Verbindungen der Formel VI-d sind bekannt (z.B. WO 2003016283). Die Umsetzung von VI-d zu VII-d und die Cyclisierung zu VIII-d kann nach bekannten Methoden durchgeführt werden (z.B. WO 2004078732 und WO 2010020522). Die Verseifung zu III-7 erfolgt nach bekannten Methoden (WO2006004903).

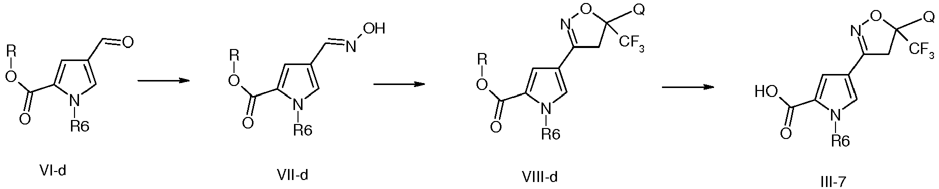

### Carbonsäuren der Formel (III-8)

Carbonsäuren der Formel (III-8) sind neu. Sie können nach dem folgenden Reaktionsschema, in welchem R⁶, Q die oben angegebenen Bedeutungen haben und R für C₁-C₆ Alkyl und R* für C₁-C₆ Alkyl steht, aus Verbindungen der Formel VI-e hergestellt werden.

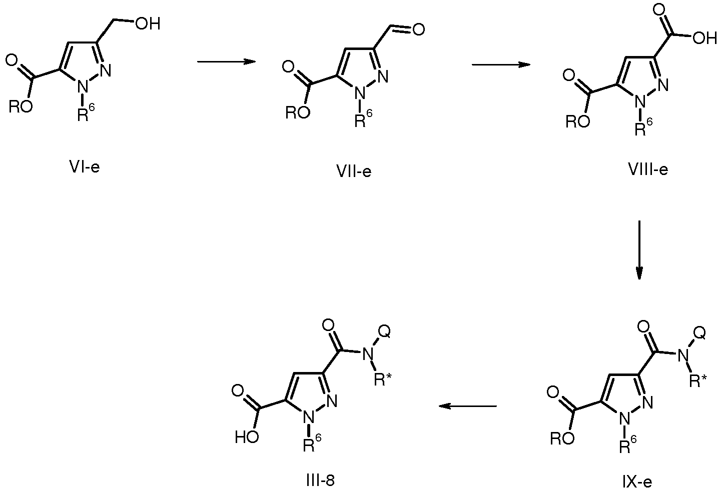

### Carbonsäuren der Formel III-9

Carbonsäuren der Formel (III-9) sind neu. Sie können nach dem folgenden Reaktionsschema, in welchem R⁶, Q die oben angegebenen Bedeutungen haben und R für C₁-C₆ Alkyl steht, aus Verbindungen der Formel (X) hergestellt werden. Verbindungen der Formel (X) sind bekannt (z.B WO 2006/000336).

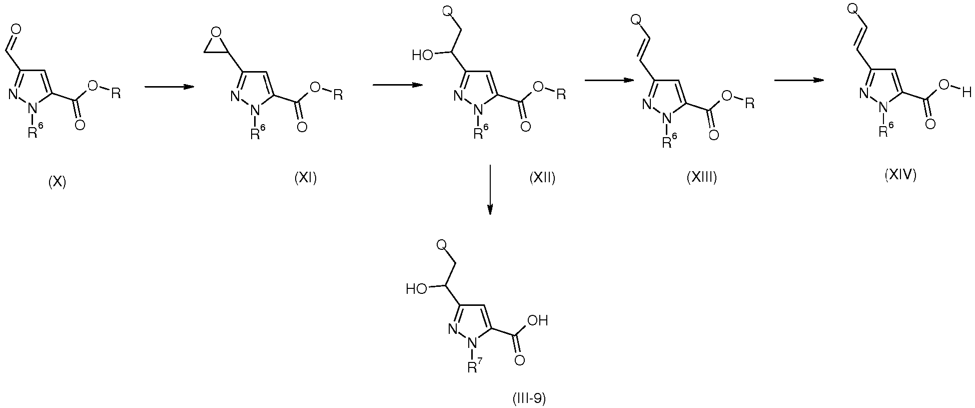

### Carbonsäuren der Formel III-10

Carbonsäuren der Formel (III-10) sind neu. Sie können nach dem folgenden Reaktionsschema, in welchem R⁶, Q die oben angegebenen Bedeutungen haben und R für C₁-C₆ Alkyl steht, aus kommerziell erhältlichem 3-Nitropyrazol der Formel (XV) hergestellt werden.

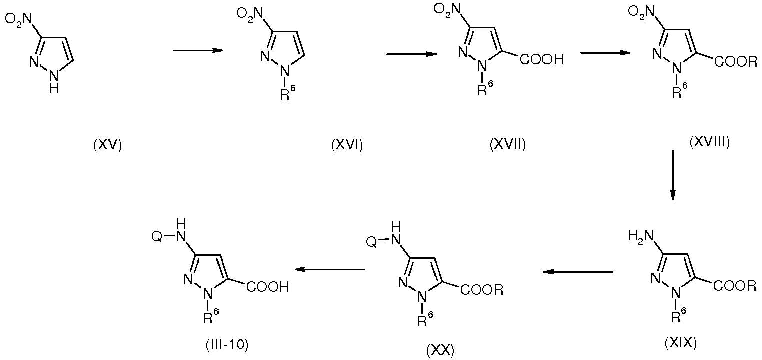

### Carbonsäuren der Formel (III-11)

Carbonsäuren der Formel (III-11) sind neu. Sie können nach dem folgenden Reaktionsschema aus Verbindungen der Formel (I) hergestellt werden. R steht dabei für C₁-C₆ Alkyl. Verbindungen der Formel (XXI) sind bekannt (z.B. WO2003/016283, WO2006/102025). Die Umsetzung von (XXI) mit (XXII) zu (XXIII) kann nach bekannten Methoden durchgeführt werden (z.B. WO2006/117370). Verbindungen der Formel (XXII) sind käuflich erhältlich oder können nach bekannten Methoden hergestellt werden (z.B. WO 2005/068460). Die weitere Umsetzung über Verbindungen der Formel (XXIII) zu Verbindungen der Formel (III-11) kann nach bekannten Methoden durchgeführt werden (z.B. WO2005/009344).

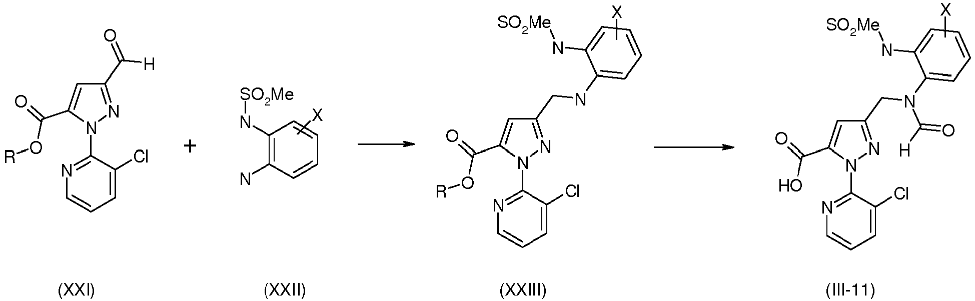

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Emteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Omithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lynmaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuellebomi, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die Wirksamkeit der Verbindungen der Formel (I) lässt sich durch die Zugabe von Ammonium- und Phosphoniumsalzen steigern. Die Ammonium- und Phosphoniumsalze werden durch Formel (XXIV) definiert 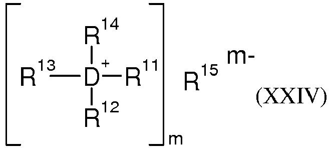 in welcher
- D: für Stickstoff oder Phosphor steht,
- D: bevorzugt für Stickstoff steht,
R¹¹, R¹², R¹³ and R¹⁴ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
R¹¹, R¹², R¹³ and R¹⁴ bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
R¹¹, R¹², R¹³ and R¹⁴ besonders bevorzugt unabhängig voneinander für Wasserstoff, Methyl, Ethyl, n-Propyl, i-Propyl, n-Butyl, i-Butyl, s-Butyl oder t-Butyl stehen,
R¹¹, R¹², R¹³ and R¹⁴ ganz besonders bevorzugt für Wasserstoff stehen,
- m: für 1, 2, 3 oder 4 steht,
- m: bevorzugt für 1 oder 2 steht,
- R¹⁵: für ein anorganisches oder organisches Anion steht,
- R¹⁵: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat, Citrat oder Oxalat steht,
- R¹⁵: besonders bevorzugt für Laktat, Sulfat, Monohydrogenphosphat, Dihydrogenphosphat, Nitrat, Thiosulfat, Thiocyanat, Citrat, Oxalat oder Formiat steht.
- R¹⁵: ganz besonders bevorzugt für Sulfat steht.

Die Ammonium- und Phosphoniumsalze der Formel (XXIV) können in einem breiten Konzentrationsbereich zur Steigerung der Wirkung von Pflanzenschutzmitteln enthaltend Verbindungen der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/l, bevorzugt 0,75 bis 37,5 mmol/l, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern ein Penetrationsförderer zugegeben. Selbst in diesen Fällen ist eine Wirkungssteigerung zu beobachten. Gegenstand der vorliegenden Erfindung ist also ebenfalls die Verwendung eines Penetrationsförders, sowie die Verwendung einer Kombination von Penetrationsförderer und Ammonium- und/oder Phosphoniumsalzen zur Wirkungssteigerung von Pflanzenschutzmitteln, die akarizid/insektizid wirksame Verbindungen der Formel (I) als Wirkstoff enthalten. Gegenstand der Erfindung ist schließlich weiterhin die Verwendung dieser Mittel zur Bekämpfung von Schadinsekten.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkoxylate in Betracht. Erfindungsgemäße Penetrationsförderer sind Alkanol-alkoxylate der Formel

R-O-(-AO)ᵥ-R' (XXV)

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (XXV-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (XXV-b)

in welcher
- R: die oben angegebene Bedeutung hat,
- R': die oben angegebene Bedeutung hat,
- EO: für -CH₂-CH₂-O- steht,
- PO: für 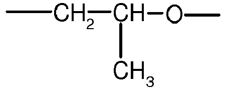 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (XXV-c)

in welcher
- R: die oben angegebene Bedeutung hat,
- R': die oben angegebene Bedeutung hat,
- EO: für -CH₂-CH₂-O- steht,
- PO: für 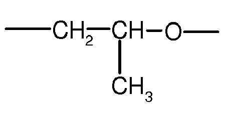 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (XXV-d)

in welcher
R und R' die oben angegebenen Bedeutungen haben,
- EO: für CH₂-CH₂-O- steht,
- BO: für 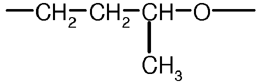 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (XXV-e)

in welcher
R und R' die oben angegebenen Bedeutungen haben,
- BO: für 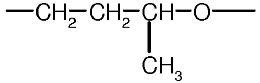 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (XXV-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (XXV-c) sei 2-Ethyl-hexyl-alkoxylat der Formel 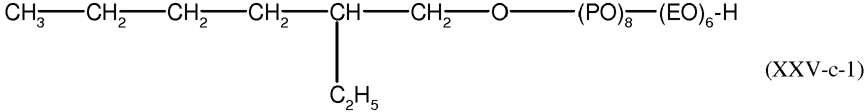 in welcher
- EO: für -CH₂-CH₂-O- steht,
- PO: für 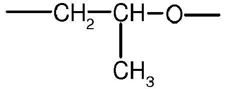 steht und
die Zahlen 8 und 6 Durchschnittswerte darstellen, genannt.

Als Beispiel für ein Alkanol-Alkoxylat der Formel (XII-d) sei die Formel

CH₃-(CH₂)₁₀-O-(-EO-)₆-(-BO-)₂-CH₃ (XXV-d-1)

in welcher
- EO: für CH₂-CH₂-O- steht,
- BO: für 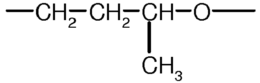 steht und
die Zahlen 10, 6 und 2 Durchschnittswerte darstellen, genannt.

Besonders bevorzugte Alkanol-Alkoxylate der Formel (XXV-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (XXV-f-1)

CH₃-(CH₂)ₜ-CH₂-O-(-CH₂-CH₂-O-)ᵤ-R' (XXV-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Löslichkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Erfindungsgemäß hervorgehobene Kombinationen von Wirkstoff, Salz und Penetrationsförderer sind in folgender Tabelle aufgeführt."Gemäß Test" bedeutet dabei, dass jede Verbindung geeignet ist, die in dem Test für die Kutikelpenetration (Baur et al., 1997, Pesticide Science 51, 131-152) als Penetrationsförderer wirkt:

| # | Wirkstoff | Salz | Penetrationsforderer |
|---|---|---|---|
| 1 | I | Ammoniumsulfat | Gemäß Test |
| 2 | I | Ammoniumlaktat | Gemäß Test |
| 3 | I | Ammoniumnitrat | Gemäß Test |
| 4 | I | Ammoniumthiosulfat | Gemäß Test |
| 5 | I | Ammoniumthiocyanat | Gemäß Test |
| 6 | I | Ammoniumcitrat | Gemäß Test |
| 7 | I | Ammoniumoxalat | Gemäß Test |
| 8 | I | Ammoniumformiat | Gemäß Test |
| 9 | I | Ammoniumhydrogenphosphat | Gemäß Test |
| 10 | I | Ammoniumdihydrogenphosphat | Gemäß Test |
| 11 | I | Ammoniumcarbonat | Gemäß Test |
| 12 | I | Ammoniumbenzoat | Gemäß Test |
| 13 | I | Ammoniumsulfit | Gemäß Test |
| 14 | I | Ammoniumbenzoat | Gemäß Test |
| 15 | I | Ammoniumhydrogenoxalat | Gemäß Test |
| 16 | I | Ammoniumhydrogencitrat | Gemäß Test |
| 17 | I | Ammoniumacetat | Gemäß Test |
| 18 | I | Tetramethylammoniumsulfat | Gemäß Test |
| 19 | I | Tetramethylammoniumlaktat | Gemäß Test |
| 20 | I | Tetramethylammoniumnitrat | Gemäß Test |
| 21 | I | Tetramethylammoniumthiosulfat | Gemäß Test |
| 22 | I | Tetramethylammoniumthiocyanat | Gemäß Test |
| 23 | I | Tetramethylammoniumcitrat | Gemäß Test |
| 24 | I | Tetramethylammoniumoxalat | Gemäß Test |
| 25 | I | Tetramethylammoniumformiat | Gemäß Test |
| 26 | I | Tetramethylammoniumhydrogenphosphat | Gemäß Test |
| 27 | I | Tetramethylammoniumdihydrogenphosphat | Gemäß Test |
| 28 | I | Tetraethylammioniumsulfat | Gemäß Test |
| 29 | I | Tetraethylammioniumlaktat | Gemäß Test |
| 30 | I | Tetraethylammioniumnitrat | Gemäß Test |
| 31 | I | Tetraethylammioniumthiosulfat | Gemäß Test |
| 32 | I | Tetraethylammioniumthiocyanat | Gemäß Test |
| 33 | I | Tetraethylammioniumcitrat | Gemäß Test |
| 34 | I | Tetraethylammioniumoxalat | Gemäß Test |
| 35 | I | Tetraethylammioniumformiat | Gemäß Test |
| 36 | I | Tetraethylammioniumhydrogenphosphat | Gemäß Test |
| 37 | I | Tetraethylammioniumdihydrogenphosphat | Gemäß Test |

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischten der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky-oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, kömige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemen, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticomis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Omithodorus moubat, Rhipicephalus sanguineus, Trombicula alfreddugesi, Neutrombicula autunmalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggem, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellbeispiele

### Synthese von Carbonsäuren der Formel III-1

### Beispiel Nr. V

### Synthese von Methyl-3-{[{[3,5-bis(trifluormethyl)benzyl]sulfonyl}(methyl)amino]methyl}-1-(3-chlorpyridin-2-yl)-1H-pyrazol-5-carboxylat

5.00 g (14.4 mmol) Methyl-1-(3-chlorpyridin-2-yl)-3-[(methylsulfonyl)methyl]-1H-pyrazol-5-carboxylat wurden in 50 mL Acetonitril gelöst und bei Raumtemperatur portionsweise mit 4.64g (14.4 mmol) 1-[3,5-Bis(trifluormethyl)phenyl]-N-methylmethansulfonamid versetzt. Anschließend wurden 2.40 g (17.35 mmol) Kaliumcarbonat zur Reaktionslösung gegeben und für 12 Stunden bei 50 °C gerührt. Nach Abkühlen auf Raumtemperatur wurde die Reaktionslösung im Vakuum vom Lösungsmittel befreit. Der Rückstand wurde mit 250 mL Wasser versetzt und mit Essigester (3 x 100 mL) extrahiert. Die vereinigten organischen Phasen wurden über Natriumsulfat getrocknet. Das Lösungsmittel wurde im Vakuum entfernt und der Rückstand chromatographisch aufgereinigt. (logP: 3,83; MH⁺: 571; ¹H-NMR (400 MHz, DMSO, δ, ppm): 2,79 (s, 3H), 3,74 (s, 3H), 4,43 (s, 2H), 4,80 (s, 2H), 7,06 (s, 1H), 7,70 (dd, 1H), 8,12 (s, 2H), 8,15 (s, 1H), 8,26 (d, 1H), 8,57 (d, 1H).

### Beispiel Nr. III-1

### Synthese von 3-{[{[3,5-Bis(trifluormethyl)benzyl]sulfonyl}(methyl)amino]methyl}-1-(3-chlorpyridin-2-yl)-1H-pyrazol-5-carbonsäure

3.30 g (5.78 mmol) Methyl-3-{[{[3,5-bis(trifluormethyl)benzyl]sulfonyl}(methyl)amino]methyl}-1-(3-chlorpyridin-2-yl)-1H-pyrazol-5-carboxylat wurden in 50 mL Methanol gelöst und bei 0 °C tropfenweise mit Natronlauge (0.28g Natriumhydroxid gelöst in 25ml Wasser; 6.94 mmol) versetzt. Die Reaktion wurde 12 Stunden bei Raumtemperatur nachgerührt. Anschließend wurde das Methanol unter vermindertem Druck entfernt und der wässrige Rückstand dreimal mit Methylenchlorid extrahiert. Die organische Phase wurde verworfen. Anschließend wurde die wässrige Phase mit 5%iger Salzsäure auf pH 3 eingestellt und mit Dichlormethan (2 x 100 mL) extrahiert. Die vereinigten organischen Phasen wurden über Natriumsulfat getrocknet. (logP: 3.07; MH⁺: 557; ¹H-NMR (400 MHz, DMSO, δ, ppm): 2,78 (s, 3H), 4,40 (s, 2 H), 4,78 (s, 2H), 6,97 (s, 1H), 7,62 (dd, 1H), 8,05 (, 1H), 8,09 (s, 2H), 8,15 (d, 1H), 8,51 (d, 1H).

### Synthese von Carbonsäuren der Formel III-2 und III-3

### Beispiel Nr. VII

### Synthese von Methyl 1-(3-chlorpyridin-2-yl)-3-{[4-(trilluormethoxy)phenyl]ethinyl}-1H-pyrazol-5-carboxylat

1,0 g (3,00 mmol) Methyl 4-brom-1-(3-chlorpyridin-2-yl)-1H-pyrrol-2-carboxylat, 1,12 g 4-Trifluormethoxy-ethinylbenzol, 0,15 g (0.78 mmol) Kupfer(I)iodid, 3,34 g (33,0 mmol) Triethylamin und 0,31 g (0,27 mmol) Tetrakis(triphenylphosphin)palladium wurden in DMF vorgelegt und unter Argon 30 min bei 80°C gerührt. Die Reaktionsmischung wurde abgesaugt, der Rückstand mit DMF gewaschen und die vereinigten Mutterlaugen eingeengt. Das gewünschte Produkt wurde durch chromatographische Reinigung erhalten.
(logP: 4,46; MH⁺: 422; ¹H-NMR (400 MHz, DMSO, δ, ppm): 3,74 (s, 3H), 7,27-7,43 (m, 3H), 7,66-7,74 (m, 3H), 8,21-8,26 (m, 1H), 8,54-8,58 (m, 1H).

Beispiel Nr. VIII:

### Synthese von Ethyl 1-(3-chlorpyridin-2-yl)-3-[(2,4-dichlorphenyl)(oxo)acetyl]-1H-pyrazol-5-carboxylat

600 mg (1,47 mmol) Ethyl 1-(3-chlorpyridin-2-yl)-3-[(2,4-dichlorphenyl)ethynyl]-1H-pyrazol-5-carboxylat wurden in einer Mischung aus 1,35 g (8,55 mmol) Kaliumpermanganat, 144 mg Aliquat 336 (0,35 mmol), 18 ml Wasser, 24 ml Dichlormethan und 1 ml Eisessig vorgelegt und 1h unter Rückfluß gerührt. Der Ansatz wurd mit 30 ml 20%iger Natruimhydrogensulfit Lösung versetzt und die abgetrennte organische Phase über Natriumsulfat getrocknet und eingeengt. Durch chromatographische Reinigung wurde das gewünschte Produkt isoliert.
(logP: 4,29; MH⁺: 453; ¹H-NMR (400 MHz, DMSO, δ, ppm): 1,12 (t, 3H), 4,20 (q, 2H), 7,64- 7,80 (m, 4H), 7,94 (d, 1H), 8,24 (d, 1H), 8,57 (dd, 1H).

### Beispiel Nr. III-2:

### Synthese von 1-(3-chlorpyridin-2-yl)-3-[(2,4-dichlorphenyl)(oxo)acetyl]-1H-pyrazol-5-carbonsäure

420 mg (0,74 mmol) Ethyl 1-(3-chlorpyridin-2-yl)-3-[(2,4-dichlorphenyl)(oxo)acetyl]-1H-pyrazol-5-carboxylat wurden in 8 ml Ethanol gelöst, mit 445 mg (3,26 mmol) 10%iger Natronlauge versetzt und 24h bei RT gerührt. Das Lösungsmittel wurde im Vakuum abdestilliert, der Rückstand in Wasser aufgenommern und einmal mit Methyl-tert.-butylether extrahiert. Anschließend wurde die wäßrige Phase mit 2N Salzsäure angesäuert und dreimal mit Methyl-tert.-butylether exthrahiert. Die vereinigten organischen Phasen wurden über Natriumsulfat getrocknet und eingeengt.
(logP: 2,78; MH⁺: 424; ¹H-NMR (400 MHz, DMSO, δ, ppm): 7,61- 7,69 (m, 3H), 7,79 (s, 1H), 7,93 (d, 1H), 8,21 (d, 1H), 8,54 (dd, 1H).

### Beispiel Nr. III-3:

### Synthese von 1-(3-Chlorpyridin-2-yl)-3-{[4-(trifluormethoxy)phenyl]ethinyl}-1H-pyrazole-5-carbonsäure

1,02 g (2,17 mmol) Methyl 1-(3-chlorpyridin-2-yl)-3-{[4-(trifluormethoxy)phenyl]ethynyl}-1H-pyrazol-5-carboxylat wurden in 20 ml Ethanol gelöst, mit 1,31 g (3,26 mmol) 10%iger Natronlauge versetzt und 2h bei RT gerührt. Das Lösungsmittel wurde im Vakuum abdestilliert, der Rückstand in Wasser aufgenommen und einmal mit Methyl-tert.-butylether extrahiert. Anschließend wurde die wäßrige Phase mit 2N Salzsäure angesäuert und dreimal mit Methyl-tert.-butylether exthrahiert. Die vereinigten organischen Phasen wurden über Natriumsulfat getrocknet und eingeengt.
(logP: 3,36; MH⁺: 408; ¹H-NMR (400 MHz, DMSO, δ, ppm): 7,28 (s, 1H), 7,40-7,43 (m, 2H), 7,63-7,74 (m, 3H), 8,18-8,22 (m, 1H), 8,52-8,56 (m, 1H).

### Synthese von Carbonsäuren der Formel III-4

### Beispiel Nr. VII-a

### Synthese von Ethyl 3-acetyl-1-(3-chlorpyridin-2-yl)-1H-pyrazol-5-carboxylat

1,4 g (15,6 mmol) Methyl 4-brom-1-(3-chlorpyridin-2-yl)-1H-pyrazl-2-carboxylat wurden unter Argon in 40 ml THF gelöst und bei Raumtemperatur 1,63 g (4,37 mmol) (1-Ethoxyvinyl)-tributylstannan, 0,54 g Lithiumchlorid und 0,24 g (0,2 mmol) Tetrakis(triphenylphosphin)palladium zugegeben und 48h unter Rückfluß gerührt. Anschließend wird das Lösungsmittel abdestilliert, der Rückstand in Methyl-tert.-butylether aufgenommen, jeweils einmal mit Wasser, 5%-iger Ammoniaklösung und gesättigter Natriumchlorid Lösung gewaschen, über Natriumsulfat getrocknet und einrotiert. Der Rückstand wurde in 40 ml THF gelöst und in Gegenwart von 3 ml 2N Salzsäure 24h bei RT gerührt. Das Lösungsmittel wurde erneut abdestilliert und der Rückstand in 60 ml Methyl-tert.-butylether gelöst und bei RT 1h mit 60 mL gesättigter Kaliumfluorid Lösung kräftig gerührt. Die organische Phase wurde abgetrennt und eingeengt. Durch chromatographische Reinigung wurde das gewünschte Produkt isoliert.
(logP: 2,31; MH⁺: 294; ¹H-NMR (400 MHz, DMSO, δ, ppm): 1,10 (t, 3H), 2,55 (s, 3H), 4,17 (q, 2H), 7,44 (s, 1H), 7,71-7,72 (m, 1H), 8,26 (dd, 1H), 8,59 (dd, 1H).

### Beispiel Nr. VIII-a

### Synthese von Ethyl 3-(bromacetyl)-1-(3-chlorpyridin-2-yl)-1H-pyrazol-5-carboxylat

1,20 g (4,40 mmol) Ethyl 3-acetyl-1-(3-chlorpyridin-2-yl)-1H-pyrazol-5-carboxylat wurden in je 30 ml Dioxan und Methyl-tert.-butylether vorgelegt und bei RT 776 mg (4,85 mmol) Brom bei RT zugetropft. Anschließend läßt man 6h unter Rückfluß rühren. Das Reaktionsgemisch wurde auf Wasser gegeben, die organische Phase abgetrennt, über Natriumsulfat getrocknet und eingeengt. Der Rückstand wurde aus Methyl-tert.-butylether umkristallisiert.
(logP: 2,87; MH⁺: 373)

### Beispiel Nr. IX

### Synthese von Ethyl 1-(3-chlorpyridin-2-yl)-3-{[5-(trifluormethyl)-2H-tetrazol-2-yl]acetyl}-1H-pyrazol-5-carboxylat

1,24 g (2,04 mmol) 5-(Trifluormethyl)-2H-tetrazol und 348 mg (2,52 mmol) Kaliumcarbonat wurden in 15 ml Acetonitril vorgelegt und 15 min bei RT gerührt. 693 mg (1,57 mmol) Ethyl 3-(bromacetyl)-1-(3-chlorpyridin-2-yl)-1H-pyrazol-5-carboxylat gelöst in 5 ml Acetonitril wurden zugegeben und die Reaktionsmischung 5 min bei 70°C gerührt. Das Lösungsmittel wurde zur Hälfte abdestilliert, der Rückstand mit 80 ml Wasser verdünnt und zweimal mit Dichlormethan exthrahiert. Die vereinigten organischen Phasen werden mit Natriumchlorid Lösung gewaschen, über Natriumsulfat getrocknet und eingeengt. Durch chromatographische Reinigung wurde das gewünschte Produkt isoliert.
(logP: 3,50; MH⁺: 430; ¹H-NMR (400 MHz, DMSO, δ, ppm): 1,12 (t, 3H), 4,21 (q, 2H), 6,72 (s, 2H), 7,72 (s, 1H), 7,80-7,82 (m, 1H), 8.39 (d, 1H), 8,65 (dd, 1H).

### Beispiel Nr. III-4

### Synthese von 1-(3-Chlorpyridin-2-yl)-3-{[5-(trifluormethyl)-2H-tetrazol-2-yl]acetyl}-1H-pyrazol-5-carbonsäure

300 mg (0,58 mmol) Ethyl 1-(3-chlorpyridin-2-yl)-3-{[5-(trifluormethyl)-2H-tetrazol-2-yl]acetyl}-1H-pyrazol-5-carboxylat wurden in 4 ml Ethanol gelöst, mit 282 mg (3,15 mmol) 2N Natronlauge versetzt und 5d bei RT gerührt. Das Lösungsmittel wurde im Vakuum abdestilliert, der Rückstand in Wasser aufgenommern und einmal mit Methyl-tert.-butylether extrahiert. Anschließend wurde die wäßrige Phase mit 2N Salzsäure angesäuert und dreimal mit Essigester exthrahiert. Die vereinigten organischen Phasen wurden über Natriumsulfat getrocknet und eingeengt.
(logP: 2,25; MH⁺: 402; ¹H-NMR (400 MHz, DMSO, δ, ppm): 6,69 (s, 2H), 7,60 (s, 1H), 7,75-7,78 (m, 1H), 8,32 (d, 1H), 8,61-8,63 (m, 1H).

### Synthese von Carbonsäuren der Formel III-5

### Beispiel Nr. VII-b

### Synthese von Ethyl 3-{3-[3,5-bis(trifluormethyl)phenyl]-2-oxoimidazolidin-1-yl}-1-(3-chlorpyridin-2-yl)-1H-pyrazol-5-carboxylat

600 mg (1,76 mmol) Ethyl 4-brom-1-(3-chlorpyridin-2-yl)-1H-pyrazl-2-carboxylat und 527 mg (1,76 mmol) 1-[3,5-Bis(trifluormethyl)phenyl]imidazolidin-2-on wurden in 30 ml Toluol vorgelegt und bei Raumtemperatur nacheinander 40,4 mg (0,21 mmol) Kupfer(I)iodid, 31,2 mg (0,35 mmol) N,N'-Dimethylethylendiamin und 489 mg (3,53 mmol) Kaliumcarbonat zugegeben. Das Reaktionsgemisch wurde 10h unter Rückfluß gerührt. Anschließend wurde das Lösungsmittel abdestilliert, der Rückstand in Essigester aufgenommen, jeweils einmal mit Wasser und gesättigter Natriumchlorid Lösung gewaschen, über Natriumsulfat getrocknet und einrotiert. Durch chromatographische Reinigung wurde das gewünschte Produkt isoliert.
(logP: 4,82; MH⁺: 548; ¹H-NMR (400 MHz, DMSO, δ, ppm): 1,12-1,19 (m, 3H), 3,99-4.05 (m, 2H), 4,15-4,20 (m, 4H), 7,40 (s, 1H), 7,76-7,71 (m, 1H), 7,77 (s, 1H), 8,24 (d, 1H), 8,30 (s, 2H), 8,57 (dd, 1H).

### Beispiel Nr. III-5

### Synthese von 3-{3-[3,5-Bis(trifluormethyl)phenyl]-2-oxoimidazolidin-1-yl}-1-(3-chlorpyridin-2-yl)-1H-pyrazol-5-carbonsäure

660 mg (1,20 mmol) Ethyl 3-{3-[3,5-bis(trifluormethyl)phenyl]-2-oxoimidazolidin-1-yl}-1-(3-chlorpyridin-2-yl)-1H-pyrazol-5-carboxylat wurden in 10 ml Ethanol gelöst, mit 72 mg (1,80 mmol) 2N Natronlauge versetzt und 1h bei RT gerührt. Das Lösungsmittel wurde im Vakuum abdestilliert, der Rückstand in Wasser aufgenommern und einmal mit Methyl-tert.-butylether extrahiert. Anschließend wurde die wäßrige Phase mit 2N Salzsäure angesäuert und dreimal mit Essigester exthrahiert. Die vereinigten organischen Phasen wurden über Natriumsulfat getrocknet und eingeengt.
(logP: 3,47; MH⁺: 420; ¹H-NMR (400 MHz, DMSO, δ, ppm): 3,98-4,04 (m, 2H), 4,14-4,18 (m, 1H), 7,32 (s, 1H), 7,64-7,67 (m, 1H), 7,77 (s, 1H), 8,21 (d, 1H), 8,30 (s, 2H), 8,56 (dd, 1H).

### Synthese von Carbonsäuren der Formel III-6

### Beispiel Nr. VII-c

### Synthese von Methyl 1-(3-Chlorpyridin-2-yl)-3-{[6-(trifluormethyl)pyridin-3-yl]methyl}-1H-pyrazol-5-carboxylat

0,8 g (2,36 mmol) Methyl 3-(brommethyl)-1-(3-chlorpyridin-2-yl)-1H-pyrazol-5-carboxylat, 1,18 g (2,60 mmol) 5-(Tributylstannyl)-2-(trifluoromethyl)pyridine, 0,11 g (0,47 mmol) Tri-2-furylphosphin und 0,07 g (0,07 mmol) Tri-(dibenzylidenaceton)-dipalladium wurden unter Argon in NMP für 2h bei 100°C gerührt. Das Lösungsmittel wurde im Vakuum abdestilliert, der Rückstand in 80 ml Essigester aufgenommen und 60 min mit 70 ml gesättigter Kaliumfluorid Lösung kräftig gerührt. Die organische Phase wurde abgetrennt, einmal mit gesättigter Natriumchlorid Lösung gewaschen, über Natriumsulfat getrocknet und einrotiert. Das gewünschte Produkt wurde durch chromatographische Reinigung erhalten.
(logP: 2,86; MH⁺: 397; ¹H-NMR (400 MHz, DMSO, δ, ppm): 3,70 (s, 3H), 4,22 (s, 2H), 7,06 (s, 1H), 7,67 (dd, 1H), 7,86 (d, 1H), 7,99 (d, 1H), 8,22 (d, 1H), 8,54 (dd, 1H), 8,74 (s, 1H).

### Beispiel Nr. VIII-c

### Synthese von Methyl 1-(3-chlorpyridin-2-yl)-3-{[6-(trifluormethyl)pyridin-3-yl]carbonyl}-1H-pyrazol-5-carboxylat

1,29 g (3,20 mmol) Methyl 1-(3-Chlorpyridin-2-yl)-3-{[6-(trifluormethyl)pyridin-3-yl]methyl}-1H-pyrazol-5-carboxylat wurden in 80 ml Dioxan gelöst, mit 16,72 g (192 mmol) Mangan(IV)oxid versetzt und 12h bei Rückfluß gerührt. Der Ansatz wurde abgesaugt und das Filtrat vom Lösungsmittel befreit. Das gewünschte Produkt wird durch chromatographische Reinigung erhalten.
(logP: 3,33; MH⁺: 411; ¹H-NMR (400 MHz, DMSO, δ, ppm): 3,79 (s, 3H), 7,75-7,78 (m, 2H), 8,12 (d, 1H), 8,32 (d, 1H), 8,62 (dd, 1H), 8,71 (dd, 1H), 9,36 (s, 1H).

### Beispiel Nr. III-6

### Synthese von 1-(3-Chlorpyridin-2-yl)-3-{[6-(trifluormethyl)pyridin-3-yl]carbonyl}-1H-pyrazol-5-carbonsäure

29 mg mg (0,07 mmol) Methyl 1-(3-chlorpyridin-2-yl)-3-{[6-(trifluormethyl)pyridin-3-yl]carbonyl}-1H-pyrazol-5-carboxylat wurden in 2 ml Ethanol gelöst, mit 4 mg (0,1 mmol) 2N Natronlauge versetzt und 30 min bei RT gerührt. Das Lösungsmittel wurde im Vakuum abdestilliert, der Rückstand in Wasser aufgenommern und einmal mit Methyl-tert.-butylether extrahiert. Anschließend wurde die wäßrige Phase mit 2N Salzsäure angesäuert und dreimal mit Essigester exthrahiert. Die vereinigten organischen Phasen wurden über Natriumsulfat getrocknet und eingeengt.
(logP: 2,33; MH⁺: 397; ¹H-NMR (400 MHz, DMSO, δ, ppm): 7,20 (s, 1H), 7,59 (dd, 1H), 8,09-8,15 (m 2H), 8,49 (dd, 1H), 8,69 (dd, 1H), 9,33 (s, 1H).

### Synthese von Carbonsäuren der Formel III-7

### Beispiel Nr. VII-d

### Synthese von Methyl 1-(3-chlorpyridin-2-yl)-3-[(hydroxyimino)methyl]

### -1H-pyrazol-5-carboxylat

1-(3-Chlorpyridin-2-yl)-3-formyl-1H-pyrazol-5-carboxylat (0.99g), Hydroxylaminhydrochlorid (0.39g) und Natriumacetat (0.61g) wurden in einer Mischung aus 5 ml Ethanol und 3 ml Wasser 30 min bei RT gerührt. Die Mischung wurde mit Methyl-tert-butylether verdünnt, mit gesättigter Natriumchlorid Lösung gewaschen und über Natriumsulfat getrocknet. Nach Abdestillieren des Lösungsmittel erhält man das gewünschte Zielprodukt.
¹H-NMR (CD3CN) δ: (E/Z mixture) 3.73-3.74 (d, 3H), 7.23 (s), 7.53-7.67 (m, 2H), 7.67(s), 8.02-8.05 (m, 1H), 8.14 (s), 8.49-8.51 (m, 1H), 9.07 (brs).

### Beispiel Nr. VIII-d

### Synthese von Methyl 1-(3-chlorpyridin-2-yl)-3-[5-(3,5-dichlorphenyl) -5-(trifluormethyl)-4,5-dihydro-1,2-oxazol-3-yl]-1H-pyrazol-5-carboxylat

N-Chlorosuccinimid (0.52g) wurde zu einer Lösung aus 1-(3-Chlorpyridin-2-yl)-3-[(hydroxyimino)methyl]-1H-pyrazol-5-carboxylat (0.99g) in 10 ml DMF gegeben und die Mischung 30 min bei 40°C gerührt. Nach Abkühlren auf RT werden 1,3-Dichlor-5-(3,3,3-trifluorprop-1-en-2-yl)benzol (0.56g) and Trimethylamin (0.3g) zu der Reaktionsmischung gegeben un 8 h bei RT gerührt. Die Mischung wurde mit Methyl-tert-butylether verdünnt, mit gesättigter Natriumchlorid Lösung gewaschen und über Natriumsulfat getrocknet. Nach Abdestillieren des Lösungsmittels wird das gewünschte Produkt durch chromatographische Reinigung erhalten.
¹H-NMR (CD3CN) δ: 3.75 (s, 3H), 3.91-3.96 (m, 1H), 4.20 (d, 1H), 7.39 (s, 1H), 7.54-7.59 (m, 4H), 8.04-8.07 (m, 1H), 8.51-8.52 (m, 1H).

### Beispiel Nr. III-7

### Synthese von 1-(3-chlorpyridin-2-yl)-3-[5-(3,5-dichlorphenyl)-5-(trifluormethyl)-4,5-dihydro-1,2-oxazol-3-yl]-1H-pyrazol-5-carbonsäure

0,9 g 1-(3-chlorpyridin-2-yl)-3-[5-(3,5-dichlorphenyl)-5-(trifluormethyl)-4,5-dihydro-1,2-oxazol-3-yl]-1H-pyrazol-5-carboxylat werden in einer Mischung aus 10 ml Wasser und 20 ml Ethanol gelöst, mit 0,2 g Natriumhydroxid versetzt und 3 h bei RT gerührt. Dert Ansatz wird mit 2N Salzsäure angesäuert, dreimal mit Methyl-tert-butylether extrahiert und über Magnesiumsulfat getrocknet. Das Produkt wird nach Abdestillieren des Lösungsmittels erhalten.
¹H-NMR (CD3CN) δ: 3.97 (d, 1H), 4.20 (d, 1H), 7.39 (s, 1H), 7.54-7.59 (m, 4H), 8.03-8.05 (m, 1H), 8.50-8.51 (m, 1H).

### Synthese von Carbonsäuren der Formel III-8

### Beispiel Nr. VII-e

### Synthese von Methyl-1-(3-chlorpyridin-2-yl)-3-formyl-1H-pyrazol-5-carboxylat

Zu einer Lösung von 3.00 g (11.2 mmol) Methyl-1-(3-chlorpyridin-2-yl)-3-(hydroxymethyl)-1H-pyrazol-5-carboxylat in 162 mg Dichlormethan wurden 3.90 g (44.9 mmol) Mangan(IV)oxid portionsweise gegeben und 4 Stunden bei 40°C gerührt. Nach dem Abkühlen auf 20°C wurden weitere 3.90 g (44.9 mmol) Mangan(IV)oxid hinzugegeben und die Mischung bei 40°C über Nacht erhitzt. Nach dem Abkühlen auf 20°C wurde über Celite filtriert und das Filtrat im Vakuum vom Lösungsmittel befreit.

### (logP: 1.82)

### Beispiel Nr. VIII-e

### Synthese von 1-(3-Chlorpyridin-2-yl)-5-(methoxycarbonyl)-1H-pyrazol-3-carbonsäure

2.00 g (7.53 mmol) Methyl-1-(3-chlorpyridin-2-yl)-3-formyl-1H-pyrazol-5-carboxylat wurden in 18 ml *tert-*Butanol gelöst und anschließend 5 ml Wasser zugegeben. Nach dem Abkühlen auf 0°C wurde nacheinander mit 2.35 g (15.1 mmol) Natriumdihydrogenphosphat-dihydrat, 2.73 g (30.1 mmol) Natriumchlorit und 3.17 g (45.2 mmol) 2-Methyl-2-buten versetzt. Nach 1 Stunde bei 20°C wurde mit Wasser versetzt und zweimal mit Ethylacetat extrahiert. Die vereinigten organischen Phasen wurden über Natriumsulfat getrocknet, und das Lösungsmittel wurde im Vakuum entfernt (logP: 1.38)

### Beispiel Nr. IX-e

### Synthese von Methyl-1-(3-chlorpyridin-2-yl)-3-{[4-(trifluormethyl)phenyl]carbamoyl}-1H-pyrazol-5-carboxylat

Eine Lösung von 1.00 g (3.55 mmol) 1-(3-Chlorpyridin-2-yl)-5-(methoxycarbonyl)-1H-pyrazol-3-carbonsäure in 13 ml Dichlormethan und 1.3 ml *N,N-*Dimethylformamid wurde auf 0 °C abgekühlt, eine Lösung von 0.86 g (5.33 mmol) 4-(Trifluormethyl)-anilin in 3 ml Dichloremethan zugetropft und portionsweise mit 0.68 g (3.55 mmol) N-Ethyl-N'-3-(dimethylaminopropyl)carbodiimid x HCl und 87 mg (710 µmol) 4-Dimethylaminopyridin versetzt. Man ließ 2 Stunden bei 0°C und 16 Stunden bei 20°C rühren. Anschließend wurde mit Dichlormethan verdünnt und nacheinander mit 1 N wäßriger Salzsäure und Wasser gewaschen. Die organische Phase wurde über Natriumsulfat getrocknet, das Lösungsmittel im Vakuum entfernt und der Rückstand an Kieselgel mit Cyclohexan/Ethylacetat = 3 : 1 → 2 : 1 gereinigt. (logP: 3.54).

### Beispiel Nr. III-8

### Synthese von 1-(3-Chlorpyridin-2-yl)-3-{[4-(trilluormethyl)phenyl]carbamoyl}-1H-pyrazol-5-carbonsäure

Eine Mischung aus 1.00 g (1.88 mmol) Methyl-1-(3-chlorpyridin-2-yl)-3-{[4-(trifluormethyl)phenyl]carbamoyl}-1H-pyrazol-5-carboxylat in 20 ml Ethanol wurde tropfenweise mit 0.98 g (2.45 mmol) 10 proz. Natronlauge versetzt und 2.5 Stunden bei 20°C gerührt. Anschließend wurde das Lösungsmittel im Vakuum entfernt und der Rückstand mit 30 ml Wasser und 30 ml Ethylacetat versetzt. Die wäßrige Phase wurde mit Salzsäure auf pH = 1 gestellt und gegen Ethylacetat extrahiert. Die vereinigten organischen Phasen wurden mit Natriumsulfat getrocknet und das Lösungsmittel im Vakuum entfernt (logP: 2.67)

### Synthese von Carbonsäuren der Formel (III-9

### Beispiel Nr. XI

### Methyl-1-(3-chlorpyridin-2-yl)-3-(oxiran-2-yl)-1H-pyrazol-5-carboxylat

5,00 g (18,8 mmol) Methyl-1-(3-chlorpyridin-2-yl)-3-formyl-1H-pyrazol-5-carboxylat (bekannt aus WO 2006/000336) und 5,76 g (28,2 mmol) Trimethylsulfoniumiodid wurden in 12,5 mL Dimethylsulfoxid vorgelegt und 2,64 g (23,5 mmol) t-BuOK in 12,5 mL Dimethylsulfoxid wurden bei Raumtemperatur zugetropft. Nach 1,5 Stunden Rühren bei Raumtemperatur wurden unter Eiskühlung vorsichtig 60 mL Wasser zugetropft, woraufhin das Reaktionsgemisch mehrfach mit Ethylacetat extrahiert wurde. Die vereinigten organischen Phasen wurden dreimal mit Wasser gewaschen und über Magnesiumsulfat getrocknet. Das Lösungsmittel wurde unter vermindertem Druck abdestilliert und der Rückstand an Kieselgel chromatographiert, wobei 1,10 g (21 % der Theorie) Methyl-1-(3-chlorpyridin-2-yl)-3-(oxiran-2-yl)-1H-pyrazol-5-carboxylat erhalten wurden. (logP: 1,67; MH⁺: 280,0; ¹H-NMR (400 MHz, CD3CN, δ, ppm): 3,10 (m, 2H), 3,71 (s, 3H), 3,98 (s, 1H), 6,92 (s, 1H), 7,56 (m, 1 H), 8,07 (m, 1H), 8,51 (m, 1H).)

### Beispiel Nr. XII

### Methyl-1-(3-chlorpyridin-2-yl)-3-{1-hydroxy-2-[5-(trifluormethyl)-2H-tetrazol-2-yl]ethyl}-1H-pyrazol-5-carboxylat / Methyl-1-(3-chlorpyridin-2-yl)-3-{1-hydroxy-2-[5-(trifluormethyl)-1H-tetrazol-1-yl]ethyl}-1H-pyrazol-5-carboxylat

51,5 mg (2,14 mmol) Natriumhydrid wurden in 15 mL Tetrahydrofuran vorgelegt und bei 0°C mit einer Lösung von 1380 mg (2,32 mmol) einer 25 prozentigen Lösung von 5-(Trifluormethyl)-2H-tetrazol in Tetrahydrofuran versetzt. Nach 20 Minuten Rühren bei 0°C wurden 500 mg (1,78 mmol) Methyl-1-(3-chlorpyridin-2-yl)-3-(oxiran-2-yl)-1H-pyrazol-5-carboxylat zugesetzt und 20 Stunden bei Rückfußtemperatur gerührt. Das Reaktionsgemisch wurde auf Wasser geschüttet, mit Eisessig angesäuert und anschließend mehrfach mit Ethylacetat extrahiert. Die vereinigten organischen Phasen wurden mit Wasser gewaschen und über Magnesiumsulfat getrocknet. Das Lösungsmittel wurde unter vermindertem Druck abdestilliert und der Rückstand an Kieselgel chromatographiert, wobei 286 mg (80% der Theorie) Methyl-1-(3-chlorpyridin-2-yl)-3-1{1-hydroxy-2-[5-(trifluormethyl)-2H-tetrazol-2-yl]ethyl}-1H-pyrazol-5 carboxylat und Methyl-1-(3-chlorpyridin-2-yl)-3-{1-hydroxy-2-[5-(trifluormethyl)-1H-tetrazol-1-yl]ethyl}-1H-pyrazol-5-carboxylat im Verhältnis 66:34 erhalten wurden. (logP: 2,64 /2,68; MH⁺: 418,0; ¹H-NMR (400 MHz, CD3CN, δ, ppm): 3,73 (s, 3H), 4,31 (m, 2H), 4,48 (m, 1H), 7,10 (m, 1 H), 7,54 (m, 1H), 8,05 (m, 1H), 8,50 (m, 1H).)

### Beispiel Nr. Nr. XIII

### Methyl-1-(3-chlorpyridin-2-yl)-3-{(E)-2-[5-(trifluormethyl)-2H-tetrazol-2-yl]vinyl}-1H-pyrazol-5-carboxylat / Methyl-1-(3-chlorpyridin-2-yl)-3-{(E)-2-[5-(trifluormethyl)-1H-tetrazol-1-yl]vinyl}-1H-pyrazol-5-carboxylat (Nr. XIII)

225 mg (0,54 mmol) Methyl-1-(3-chlorpyridin-2-yl)-3-{1-hydroxy-2-[5-(trifluormethyl)-2H-tetrazol-2-yl]ethyl}-1H-pyrazol-5 carboxylat und Methyl-1-(3-chlorpyridin-2-yl)-3-{1-hydroxy-2-[5-(trifluormethyl)-1H-tetrazol-1-yl]ethyl}-1H-pyrazol-5-carboxylat im Verhältnis 66:34 wurden in 7,0 mL Toluol vorgelegt, bei 0°C mit 75,3 mg (0,65 mmol) Methansulfonsäurechlorid 166 mg (1,64 mmol) Trimethylamin versetzt und 10 Stunden bei Rückfußtemperatur gerührt. Das Reaktionsgemisch wurde mit Toluol verdünnt und mit Natriumhydrogencarbonatlösung gewaschen. Die organischen Phasen wurden über Magnesiumsulfat getrocknet und das Lösungsmittel wurde unter vermindertem Druck abdestilliert. Der Rückstand wurde an Kieselgel chromatographiert, wobei 132 mg (45% der Theorie) Methyl-1-(3-chlorpyridin-2-yl)-3-{(E)-2-[5-(trifluormethyl)-2H-tetrazol-2-yl]vinyl}-1H-pyrazol-5-carboxylat und Methyl-1-(3-chlorpyridin-2-yl)-3-{(E)-2-[5-(trifluormethyl)-1H-tetrazol-1-yl]vinyl}-1H-pyrazol-5-carboxylat im Verhältnis 69:31 erhalten wurden.. (logP: 3,37 /3,49; MH⁺: 400,1; ¹H-NMR (400 MHz, CD3CN, δ, ppm): 3,78 (s, 3H), 7.35 (m, 1H), 7,60 (m, 1 H), 7,75 (m, 1H), 8,08 (m, 1H), 8,32 (m, 1H), 8,55 (m, 1H).)

### Beispiel Nr. XIV

### 1-(3-Chlorpyridin-2-yl)-3-{(E)-2-[5-(trifluormethyl)-2H-tetrazol-2-yl]vinyl}-1H-pyrazol-5-carbonsäure / 1-(3-Chlorpyridin-2-yl)-3-{(E)-2-[5-(trifluormethyl)-1H-tetrazol-1-yl]vinyl}-1H-pyrazol-5-carbonsäure

88,7 mg (0,23 mmol) Methyl-1-(3-chlorpyridin-2-yl)-3-{(E)-2-[5-(trifluormethyl)-2H-tetrazol-2-yl]vinyl}-1H-pyrazol-5-carboxylat und Methyl-1-(3-chlorpyridin-2-yl)-3-{(E)-2-[5-(trifluormethyl)-1H-tetrazol-1-yl]vinyl}-1H-pyrazol-5-carboxylat im Verhältnis 66:34 wurden in 5,0 mL Ethanol gelöst, und mit 26,6 mg (0,29 mmol) 45 prozentiger Natronlauge versetzt. Nach 1 Stunde Rühren bei Raumtemperatur wurde mit Wasser verdünnt und mit verdünnter Salzsäure angesäuert. Die wässrige Phase wurde mehrfach mit Ethylacetat extrahiert und über Magnesiumsulfat getrocknet. Das Lösungsmittel wurde unter vermindertem Druck abdestilliert, wobei 43 mg (48% der Theorie) 1-(3-Chlorpyridin-2-yl)-3-{(E)-2-[5-(trifluormethyl)-2H-tetrazol-2-yl]vinyl}-1H-pyrazol-5-carbonsäure und 1-(3-Chlorpyridin-2-yl)-3-{(E)-2-[5-(trifluormethyl)-1H-tetrazol-1-yl]vinyl}-1H-pyrazol-5-carbonsäure im Verhältnis 70:30 erhalten wurden.. (logP: 2,79 /2,86; MH⁺: 386,0/386,0; ¹H-NMR (400 MHz, CD3CN, δ, ppm): 7.40 (m, 1H), 7,59 (m, 1 H), 7,75 (m, 1H), 8,05 (m, 1H), 8,31 (m, 1H), 8,53 (m, 1H).)

### Beispiel Nr. III-9

### 1-(3-Chlorpyridin-2-yl)-3-{1-hydroxy-2-[5-(trifluormethyl)-2H-tetrazol-2-yl]ethyl}-1H-pyrazol-5-carbonsäure / 1-(3-Chlorpyridin-2-yl)-3-{1-hydroxy-2-[5-(trifluormethyl)-1H-tetrazol-1-yl]ethyl}-1H-pyrazol-5-carbonsäure

72,5 mg (0,18 mmol) Methyl-1-(3-chlorpyridin-2-yl)-3-{1-hydroxy-2-[5-(trifluormethyl)-2H-tetrazol-2-yl]ethyl}-1H-pyrazol-5 carboxylat und Methyl-1-(3-chlorpyridin-2-yl)-3-{1-hydroxy-2-[5-(trifluormethyl)-1H-tetrazol-1-yl]ethyl}-1H-pyrazol-5-carboxylat im Verhältnis 66:34 wurden in 5,0 mL Ethanol gelöst, und 21,0 mg (0,23 mmol) 45 prozentige Natronlauge zugesetzt. Nach 1 Stunde Rühren bei Raumtemperatur wurde mit Wasser verdünnt und mit verdünnter Salzsäure angesäuert. Die wässrige Phase wurde mehrfach mit Ethylacetat extrahiert und über Magnesiumsulfat getrocknet. Das Lösungsmittel wurde unter vermindertem Druck abdestilliert, wobei 43 mg (48% der Theorie) 1-(3-Chlorpyridin-2-yl)-3-{1-hydroxy-2-[5-(trifluormethyl)-2H-tetrazol-2-yl]ethyl}-1H-pyrazol-5-carbonsäure und 1-(3-Chlorpyridin-2-yl)-3-{1-hydroxy-2-[5-(trifluormethyl)-1H-tetrazol-1-yl]ethyl}-1H-pyrazol-5-carbonsäure im Verhältnis 73:27 erhalten wurden. (logP: 1,68 /1,37; MH⁺: 404,0 /404,0)

### Synthese von Carbonsäuren der Formel III-10

### Beispiel Nr. III-10

### 1-(3-Chlorpyridin-2-yl)-3-{[5-(trifluormethyl)pyridin-2-yl]amino}-1H-pyrazol-5-carbonsäure

### Beispiel Nr. XVI

### 3-Chlor-2-(3-nitro-1H-pyrazol-1-yl)pyridin

10,0 g (88,4 mmol) 3-Nitropyrazol und 14,4 g (97,2 mmol) 2,3-Dichlorpyridin wurden in 50 mL Dimethylformamid vorgelegt, 23,9 g (173 mmol) Kaliumcarbonat zugesetzt und das Reaktionsgemisch 18 Stunden bei 125 °C gerührt. Nach Abkühlen wurde auf Wasser geschüttet und der ausgefallene Feststoff abfiltriert. Nach Umkristallisieren aus Isopropanol/Wasser erhielt man 18,1 g (90 % der Theorie) 3-Chlor-2-(3-nitro-1H-pyrazol-1-yl)pyridin (logp: 1,83; MH⁺: 225,1; ¹H-NMR (400 MHz, CD3CN, δ, ppm): 7.15 (s,1H), 7,56(d,1H), 8,15 (d,1H), 8,23 (d, 1H), 8,53 (m, 1H).

### Beispiel Nr. XVII

### 1-(3-Chlorpyridin-2-yl)-3-nitro-1H-pyrazol-5-carbonsäure

8,65 g (38,5 mmol) 3-Chlor-2-(3-nitro-1H-pyrazol-1-yl)pyridin wurden in 196 mL Tetrahydrofuran gelöst und unter Argon auf -78 °C gekühlt. Daraufhin wurden 21,4 mL (42,7 mmol) einer 2 molaren Lösung von Lithiumdiisopropylamid in THF getropft. Man ließ eine Stunde bei -78 °C nachrühren und gab dann portionsweise insgesammt 1500 g gepulvertes Trockeneis zu. Es wurde solange bei -78°C gerührt, bis keine Kohlendioxidbildung mehr zu beobachten war und anschließend liess man bis Raumtemperatur erwärmen. Zunächst wurden nun 679 mL Wasser zugesetzt und anschließend mit 1N Natriumhydroxidlösung pH 11 eingestellt. Die wässrige Phase wurde mehrfach mit Ethylacetat extrahiert. Die vereinigten organischen Phasen wurden über Magnesiumsulfat getrocknet und das Lösungsmittel unter vermindertem Druck abdestilliert, wobei 5,0 g (47 % der Theorie) 1-(3-Chlorpyridin-2-yl)-3-nitro-1H-pyrazol-5-carbonsäure erhalten wurden logp: 0,90; MH⁺: 269,0; ¹H-NMR (400 MHz, CD3CN, δ, ppm): 7.77 (m,2H), 8,33 (m,1H) 8,62 (m, 1H).

### Beispiel Nr. XVIII

### Methyl-1-(3-chlorpyridin-2-yl)-3-nitro-1H-pyrazol-5-carboxylat

5,00 g (18,6 mmol) 1-(3-Chlorpyridin-2-yl)-3-nitro-1H-pyrazol-5-carbonsäure wurden in 66 mL Methylenchlorid vorgelegt, bei Raumtemperatur 6,64 g (55,8 mmol) Thionylchlorid zugegeben und das Reaktionsgemisch 24 Stunden unter Rückfluss erhitzt. Das Lösungsmittel wurde unter vermindertem Druck abdestilliert und der Rückstand direkt weiter umgesetzt.

Das Säurechlorid wurde unter Eiskühlung in 32 mL Methanol gelöst und 1,98 g (19,6 mmol) Trimethylamin zugesetzt. Nach 18 Stunden Rühren bei Raumtemperatur wurde das Lösungsmittel unter vermindertem Druck vollständig entfernt, der Rückstand in Methylenchlorid aufgenommen und mit 0,5 N HCl gewaschen. Die organische Phase wurde über Magnesiumsulfat getrocknet, das Lösungsmittel wurde unter vermindertem Druck abdestilliert und der Rückstand an Kieselgel chromatographiert wobei 3,20 g (68 % der Theorie) Methyl-1-(3-chlorpyridin-2-yl)-3-nitro-1H-pyrazol-5-carboxylat erhalten wurden.(logp: 2,18; MH⁺: 283,1); ; ¹H-NMR (400 MHz, CD3CN, δ, ppm): 3,79 (s, 3H), 7,80 (m,1H), 7,89 (s, 1H), 8,36 (m, 1H), 8,63 (d, 1H).)

### Beispiel Nr. XIX

### Methyl-3-amino-1-(3-chlorpyridin-2-yl)-1H-pyrazol-5-carboxylat

10,41 g (54,9 mmol) Zinn (II)chloride wurden in 80 mL Ethanol vorgelegt, 3,20 g (11,3 mmol) Methyl-1-(3-chlorpyridin-2-yl)-3-nitro-1H-pyrazol-5-carboxylat zugesetzt und 3 Stunden bei Rückflusstemperatur gerührt. Das Reaktionsgemisch wurde nach Abkühlen auf Eis gegossen und mit Natriumbicarbonat pH 12 eingestellt. Die wässrige Phase wurde mehrfach mit Ethylacetat extrahiert und über Magnesiumsulfat getrocknet. Das Lösungsmittel wurde unter vermindertem Druck abdestilliert und der Rückstand wurde an Kieselgel chromatographiert wobei 2,20 g (71% der Theorie) Methyl-3-amino-1-(3-chlorpyridin-2-yl)-1H-pyrazol-5-carboxylat erhalten wurden (logp: 1,09; MH⁺: 253,1); ; ¹H-NMR (400 MHz, CD3CN, δ, ppm): 3,66 (s, 3H), 5,20 (s, 2H), 6,25 (s, 1H), 7,56 (m, 1H), 8,15 (d, 1H), 8,47 (m,1H).)

### Beispiel Nr. XX

### Methyl-1-(3-chlorpyridin-2-yl)-3-{[5-(trifluormethyl)pyridin-2-yl]amino}-1H-pyrazol-5-carboxylat

1,90 g (7,52 mmol) Methyl-3-amino-1-(3-chlorpyridin-2-yl)-1H-pyrazol-5-carboxylat, 1,50 g (8,27 mmol) 2-Chlor-5-trifluormethylpyridin, 0,275 g (0,3 mmol) Tris(dibenzylaceton)dipalladium, 0,286 g (0,60 mmol) 2-Dicyclohexylphoshino-2,4,6-tri-isopropyl-1,1-biphenyl und 3,12 g (22,5mmol) Kaliumcarbonat wurden in 190 mL tert. Butanol vorgelegt und das Reaktionsgemisch 24 Stunden bei 100 °C gerührt. Nach Abkühlen wurde auf Wasser gegossen, mehrfach mit Ethylacetat extrahiert und über Magnesiumsulfat getrocknet. Das Lösungsmittel wurde unter vermindertem Druck abdestilliert und der Rückstand wurde an Kieselgel chromatographiert, wobei 950 mg (29% der Theorie) Methyl-1-(3-chlorpyridin-2-yl)-3-{[5-(trifluormethyl)pyridin-2-yl]amino}-1H-pyrazol-5-carboxylat erhalten wurden. (logp: 3,14; MH⁺: 398,0); ; ¹H-NMR (400 MHz, CD3CN, δ, ppm): 3,74(s, 3H), 7,26(m, 1H), 7,37 (s, 1H), 7,66 (m, 1H), 7,95 (m, 1H), 8,25 (d, 1H), 8,56 (m,2H).)

### Beispiel Nr. III-10

### 1-(3-Chlorpyridin-2-yl)-3-{[5-(trifluormethyl)pyridin-2-yl]amino}-1H-pyrazol-5-carbonsäure

855 mg (2,15 mmol) Methyl-1-(3-chlorpyridin-2-yl)-3-{[5-(trifluormethyl)pyridin-2-yl]amino}-1H-pyrazol-5-carboxylat wurden in 10,0 mL Ethanol gelöst, und mit 248 mg (2,79 mmol) 45 prozentiger Natronlauge versetzt. Nach 24 Stunden Rühren bei Raumtemperatur wurde mit Wasser verdünnt und mit verdünnter Salzsäure angesäuert. Die wässrige Phase wurde mehrfach mit Ethylacetat extrahiert und über Magnesiumsulfat getrocknet. Das Lösungsmittel wurde unter vermindertem Druck abdestilliert, wobei 758 mg (92% der Theorie) 1-(3-Chlorpyridin-2-yl)-3-{[5-(trifluormethyl)pyridin-2-yl]amino}-1H-pyrazol-5-carbonsäure erhalten wurden.. (logP: 2,79; MH⁺: 384,0; ¹H-NMR (400 MHz, CD3CN, δ, ppm): 7,28 (m, 1H), 7,38 (m, 1H), 7,55 (m, 1 H), 7,89 (m, 1H), 8,05 (m, 1H), 8,51 (m, 2H).

### Herstellungsbeispiele

Gemäß den oben beschriebenen Herstellverfahren lassen sich die Verbindungen der Formel (I) erhalten, die teils als Regiosiomere vorliegen können. Dabei sind in der folgenden Tabelle in Bezug auf die NMR-Daten jeweils die chemischen Verschiebungen und die dazugehörigen Signalintensitäten angegeben, beispielsweise steht für Verbindung 1:
Signal 1
   10.20; 2.040; für 10.20 ppm (chemische Verschiebung), 2.040 (Signalintensität);
Signal 2
   8.50; 2.770; für 8.50 ppm (chemische Verschiebung), 2.770 (Signalintensität);

| NR | Structure | Log p | MH+ | NMR |
|---|---|---|---|---|
| 1 | 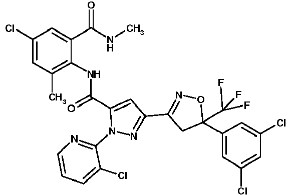 | | | (10.20;2.040),(8.50;2.770),(8.50;2.750),(8.49;2.870),(8.49;2.900),(8.15;2.480),(8.1 4;2.460),(8.13;2.840),(8.12;2.490),(8.02;1.140),(7.73;2.360),(7.72;4.230),(7.72;3.0 50),(7.67;7.660),(7.67;7.450),(7.62;2.640),(7.61;2.720),(7.60;2.650),(7.59;2.500),( 7.42;2.710),(7.42;3.000),(7.34;3.250),(7.34;2.890),(4.29;1.680),(4.25;4.120),(4.20; 2.890),(4.15;1.280),(3.65;0.880),(3.27;0.810),(3.26;0.860),(3.20;1.000),(3.19;1.33 0),(3.16;2.450),(3.15;2.980),(3.14;3.840),(3.11;1059.520),(3.08;8.370),(3.07;2.170 ),(2.68;9.930),(2.67;10.190),(2.66;3.210),(2.66;2.730),(2.65;2.200),(2.55;1.330),(2 .53;10.290),(2.50;15 8.700),(2.49;306.850),(2.49;417.820),(2.48;294.340),(2.48;14 3.670),(2.45;1.480),(2.32;1.250),(2.32;2.000),(2.31;2.490),(2.31;1.920),(2.16;16.0 00),(2.04;0.890),(1.90;0.910),(1.25;1.060),(1.11;14.530),(0.89;1.530),(0.01;6.080), (-0.00;123.470),(-0.01;5.520) |
| 2 | 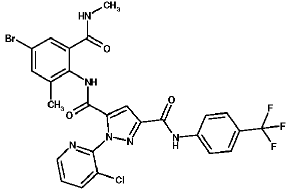 | 3,53 | | (10.56;0.920),(10.53;0.420),(8.54;2.480),(8.53;2.860),(8.52;2.930),(8.52;3.040),(8. 17;2.370),(8.16;2.610),(8.15;2.820),(8.14;2.810),(8.07;4.020),(8.05;4.560),(7.77;2. 040),(7.69;4.330),(7.67;4.070),(7.66;0.540),(7.64;2.730),(7.63;2.680),(7.62;2.550), (7.61;2.540),(7.56;2.550),(7.55;2.930),(7.48;3.100),(7.48;2.890),(7.46;0.460),(3.17 ;0.720),(3.16;0.860),(3.16;0.880),(3.11;611.470),(3.07;2.120),(3.06;1.130),(3.05;1. 310),(3.05;0.920),(3.02;0.440),(2.69;9.550),(2.68;9.500),(2.66;0.710),(2.66;0.840), (2.53;5.360),(2.50;42.200),(2.49;82.380),(2.49;112.620),(2.48;79.570),(2.48;38.94 0),(2.45;0.560),(2.32;0.510),(2.32;0.710),(2.17;16.000),(2.08;1.750),(2.04;1.260),( 1.97;1.420),(1.30;0.550),(1.28;0.420),(1.25;1.270),(1.20;0.540),(1.18;0.900),(1.16; 0.520),(0.88;0.800),(0.86;0.700),(0.86;0.530),(0.85;0.420),(0.01;1.000),(0.00;18.9 20),(-0.01;0.580) |
| 3 | 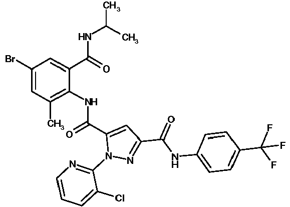 | 4,02 | | (10.58;0.280),(10.56;0.400),(8.52;2.120),(8.52;2.190),(8.51;2.240),(8.51;2.070),(8. 16;2.110),(8.15;2.000),(8.14;2.220),(8.13;2.050),(8.07;2.880),(8.05;3.170),(7.92;0. 360),(7.90;0.340),(7.89;0.290),(7.78;1.810),(7.69;3.360),(7.67;2.920),(7.64;0.320), (7.64;2.060),(7.62;2.080),(7.62;1.930),(7.60;1.840),(7.55;2.020),(7.54;2.180),(7.45 ;2.260),(7.45;1.990),(3.98;0.250),(3.96;0.640),(3.94;0.950),(3.93;0.910),(3.91;0.62 0),(3.89;0.260),(3.17;0.390),(3.17;0.290),(3.11;205.150),(3.07;0.370),(3.06;0.390), (3.05;0.380),(2.66;0.220),(2.66;0.350),(2.65;0.260),(2.63;0.260),(2.53;2.430),(2.50 ;19.490),(2.49;38.450),(2.49;53.040),(2.48;37.680),(2.48;18.670),(2.32;0.280),(2.3 1;0.290),(2.31;0.240),(2.21;0.380),(2.18;11.770),(2.08;1.420),(2.04;0.500),(1.40;0. 320),(1.25;0.250),(1.13;0.330),(1.12;0.350),(1.07;15.970),(1.05;16.000),(0.00;9.61 0),(-0.01;0.430) |
| 4 | 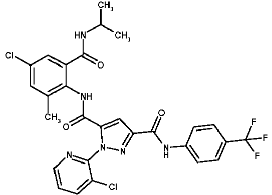 | 3,96 | | (10.57;2.380),(10.19;1.420),(8.53;2.150),(8.52;2.140),(8.51;2.270),(8.51;2.100),(8. 16;2.100),(8.16;2.100),(8.14;2.320),(8.14;2.160),(8.07;2.800),(8.05;3.070),(7.97;0. 500),(7.83;1.030),(7.81;2.630),(7.69;3.220),(7.67;2.840),(7.64;2.180),(7.63;2.060), (7.62;2.000),(7.61;1.920),(7.43;2.210),(7.42;2.350),(7.32;2.450),(7.31;2.120),(3.96 ;0.670),(3.95;1.000),(3.93;0.950),(3.91;0.670),(3.24;0.660),(3.22;0.670),(3.21;0.72 0),(3.20;0.930),(3.20;0.980),(3.19;1.070),(3.18;1.230),(3.18;1.330),(3.16;2.100),(3 .12;1048.820),(3.07;2.020),(3.06;1.020),(3.05;0.640),(2.67;0.500),(2.66;0.770),(2. 66;1.010),(2.65;0.710),(2.53; 8.730),(2.50;63.010),(2.49;123.740),(2.49;169.950),( 2.48;120.360),(2.48;59.100),(2.32;0.660),(2.32;1.000),(2.31;0.730),(2.19;11.940),( 2.04;2.030),(1.97;0.720),(1.25;0.660),(1.07;16.000),(1.05;15.770),(0.01;0.870),(-0.00;20.350),(-0.01;0.920) |
| 5 | 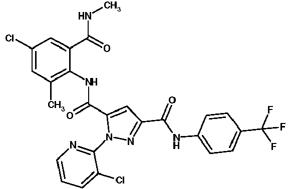 | 3,46 | | (10.57;2.810),(10.23;0.770),(8.54;2.720),(8.53;2.890),(8.53;2.900),(8.52;2.810),(8. 24;5.280),(8.17;2.650),(8.17;2.700),(8.15;2.980),(8.15;2.870),(8.08;3.600),(8.05;4. 370),(8.03;0.930),(7.97;0.410),(7.79;2.370),(7.69;4.150),(7.67;3.750),(7.64;2.820), (7.63;2.810),(7.62;2.620),(7.61;2.580),(7.43;2.600),(7.43;2.960),(7.35;3.150),(7.34 ;2.820),(3.62;0.410),(3.60;1.010),(3.60;0.440),(3.20;0.390),(3.18;0.560),(3.17;0.68 0),(3.17;1.040),(3.16;1.070),(3.12;525.300),(3.08;1.140),(3.07;0.760),(3.06;0.530), (3.05;0.490),(2.70;9.970),(2.68;9.810),(2.67;0.390),(2.66;0.510),(2.66;0.670),(2.65 ;0.450),(2.53;4.470),(2.50;32.490),(2.49;63.900),(2.49;87.960),(2.48;62.340),(2.48 ;30.770),(2.32;0.420),(2.32;0.530),(2.31;0.400),(2.18;16.000),(2.08;2.570),(2.04;0. 510),(1.78;0.450),(1.77;0.490),(1.76;1.210),(1.75;0.460),(1.75;0.400),(0.01;0.420), (0.00;8.750) |
| 6 | 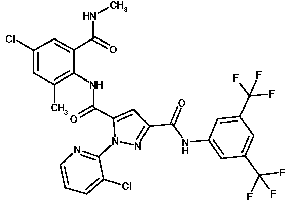 | 4,16 | | (10.93;1.410),(10.26;0.600),(8.61;6.940),(8.55;2.750),(8.54;2.860),(8.53;2.970),(8. 53;2.810),(8.18;2.660),(8.18;2.660),(8.16;2.970),(8.16;2.800),(8.05;0.810),(7.97;0. 380),(7.80;2.170),(7.74;3.030),(7.65;2.730),(7.64;2.720),(7.63;2.630),(7.62;2.510), (7.43;2.610),(7.43;2.910),(7.35;3.130),(7.35;2.770),(3.62;1.650),(3.62;1.220),(3.61 ;1.160),(3.61;1.600),(3.60;3.920),(3.60;1.570),(3.59;1.120),(3.59;1.240),(3.59;1.64 0),(3.40;0.940),(3.38;0.940),(3.18;0.560),(3.17;0.740),(3.16;1.220),(3.16;1.080),(3 .12;410.100),(3.07;0.530),(2.70;9.830),(2.68;9.710),(2.66;0.440),(2.66;0.530),(2.5 3 ;3.700),(2.51;1.790),(2.50;26.970),(2.49;53.120),(2.49;73.150),(2.48;52.040),(2.4 8;25.710),(2.32;0.430),(2.18;16.000),(2.04;0.570),(1.78;1.660),(1.77;1.760),(1.76; 4.610),(1.75;1.720),(1.75;1.550),(1.11;0.930),(1.09;1.860),(1.07;0.940),(0.01;0.41 0),(-0.00;8.710) |
| 7 | 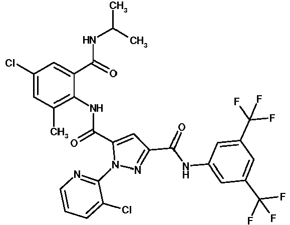 | 4,62 | | (10.93;0.320),(8.61;4.920),(8.54;1.860),(8.53;1.980),(8.52;2.030),(8.52;1.950),(8.1 7;1.860),(8.17;1.880),(8.15;2.090),(8.15;1.970),(7.85;0.600),(7.82;1.960),(7.74;2.1 60),(7.65;1.900),(7.64;1.920),(7.63:1.860),(7.62;1.780),(7.43;1.990),(7.42;2.210),( 7.32;2.200),(7.32;2.000),(3.97;0.580),(3.95;0.880),(3.93;0.850),(3.92;0.580),(3.62; 2.200),(3.62;1.650),(3.61;1.610),(3.61;2.180),(3.60;5.260),(3.60;2.180),(3.59;1.54 0),(3.59;2.180),(3.58;0.620),(3.42;0.970),(3.40;2.810),(3.39;2.840),(3.37;0.980),(3 .19;0.320),(3.18;0.290),(3.18;0.340),(3.16;0.760),(3.13;146.780),(3.08;0.310),(2.5 3;1.040),(2.50;7.750),(2.49;15.060),(2.49;20.530),(2.49;14.510),(2.48;7.110),(2.19 ;11.060),(2.04;0.280),(1.78;2.280),(1.77;2.440),(1.77;1.900),(1.76;6.210),(1.76;1.8 60),(1.75;2.320),(1.75;2.060),(1.11;3.080),(1.09;6.150),(1.07;16.000),(1.06;14.370 ),(0.00;1.420) |
| 8 | 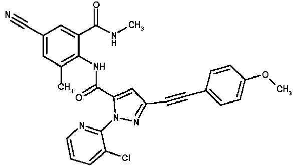 | 3,11 | 525 | (10.499;1.75),(8.513;1.32),(8.509;1.42),(8.501;1.44),(8.498;1.39),(8.347;0.68),(8.3 35;0.69),(8.188;1.32),(8.184;1.32),(8.167;1.49),(8.164;1.38),(7.872;1.44),(7.869;1. 54),(7.766;1.66),(7.762;1.56),(7.629;1.40),(7.618;1.36),(7.609;1.33),(7.597;1.34),( 7.585;0.37),(7.578;2.89),(7.573;0.96),(7.561;1.00),(7.556;3.19),(7.549;0.38),(7.46 2;2.05),(7.042;0.39),(7.035;3.11),(7.030;0.98),(7.018;0.95),(7.013;2.83),(7.005;0.3 0),(3.815;15.00),(3.295;242.65),(2.689;4.62),(2.678;4.68),(2.668;0.42),(2.538;0.40 ),(2.522;0.85),(2.517;1.42),(2.508;15.41),(2.504;31.19),(2.499;42.19),(2.495;29.65 ),(2.490;13.57),(2.224;7.06),(2.067;0.49),(-0.000;0.42) |
| 9 | 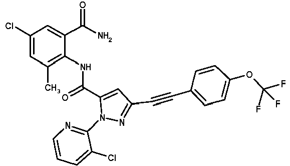 | 3,94 | 574 | (10.299;3.51),(8.523;2.80),(8.519;3.03),(8.5 11;3.07),(8.508;3.02),(8.192;2.69),(8.1 88;2.73),(8.172;3.01),(8.168;2.83),(7.792;0.74),(7.786;5.70),(7.780;2.00),(7.769;2. 18),(7.764;6.68),(7.757;0.91),(7.728;1.62),(7.635;2.97),(7.623;2.88),(7.615;2.80),( 7.603;2.81),(7.491;7.72),(7.476;7.01),(7.470;4.64),(7.457;3.54),(7.419;3.57),(7.41 3;3.00),(3.412;0.94),(3.394;2.82),(3.377;2.90),(3.359;1.03),(3.297;356.18),(3.273; 2.36),(2.669;0.38),(2.539;0.49),(2.522;1.07),(2.509;20.00),(2.504;40.68),(2.500;55 .33),(2.495;39.05),(2.490;18.02),(2.326;0.39),(2.322;0.31),(2.164;15.00),(2.067;0. 49),(1.109;2.92),(1.091;5.88),(1.074;2.81),(-0.000;0.57) |
| 10 | 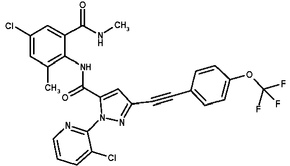 | 4,29 | 588 | (10.260;3.62),(8.516;2.72),(8.512;2.92),(8.504;2.93),(8.500;2.87),(8.227;1.44),(8.2 16;1.43),(8.205;0.53),(8.191;2.65),(8.187;2.66),(8.170;2.93),(8.167;2.74),(7.796;0. 72),(7.789;5.45),(7.784;1.92),(7.772;2.09),(7.767;6.29),(7.760;0.82),(7.634;2.84),( 7.623;2.72),(7.614;2.67),(7.602;2.67),(7.502;7.15),(7.477;4.03),(7.472;3.93),(7.46 5;3.88),(7.457;3.51),(7.352;3.25),(7.346;2.98),(3.412;0.52),(3.394;1.44),(3.377;1.5 0),(3.359;0.65),(3.336;0.36),(3.296;388.43),(3.273;2.27),(2.678;9.46),(2.667;9.46), (2.539;0.55),(2.522;1.22),(2.509;21.90),(2.504;44.19),(2.500;59.82),(2.495;42.06), (2.490;19.33),(2.331;0.34),(2.326;0.37),(2.173;15.00),(2.067;0.47),(1.109;1.44),(1.091;2.92),(1.074;1.43),(-0.000;0.64) |
| 11 | 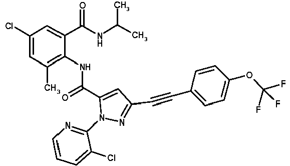 | 4,79 | 616 | (10.218;2.55),(8.502;2.02),(8.498;2.13),(8.490;2.19),(8.486;2.08),(8.177;1.91),(8.1 74;1.89),(8.157;2.14),(8.153;1.96),(8.060;1.02),(8.040;1.02),(7.796;0.59),(7.789;4. 12),(7.784;1.45),(7.773;1.66),(7.767;4.72),(7.761;0.63),(7.629;2.04),(7.617;2.00),( 7.609;1.92),(7.597;1.92),(7.517;5.01),(7.476;2.93),(7.461;3.23),(7.456;4.78),(7.31 2;2.40),(7.306;2.22),(3.948;0.58),(3.931;0.90),(3.912;0.87),(3.896;0.58),(3.301;17 5.99),(3.277;1.68),(2.539;0.44),(2.523;0.70),(2.509;10.95),(2.505;21.48),(2.500;28 .55),(2.496;20.02),(2.491;9.24),(2.178;11.07),(1.055;15.00),(1.038;14.81),(-0.000;0.48) |
| 12 | 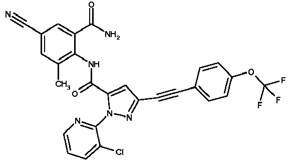 | 3,58 | 565 | (10.593;2.79),(8.528;2.87),(8.524;3.01),(8.516;3.11),(8.512;2.96),(8.197;2.75),(8.1 93;2.76),(8.177;3.10),(8.173;2.86),(7.872;3.85),(7.844;4.04),(7.840;3.32),(7.795;0. 87),(7.788;6.27),(7.783;2.12),(7.771;2.43),(7.766;7.18),(7.759;0.92),(7.640;2.93),( 7.628;2.88),(7.620;2.80),(7.608;2.95),(7.588;1.81),(7.511;3.61),(7.480;3.75),(7.47 8;4.13),(7.458;3.65),(3.301;260.31),(3.278;2.37),(2.539;0.61),(2.523;1.02),(2.510; 15.65),(2.505;30.88),(2.500;41.22),(2.496;29.01),(2.491;13.42),(2.210;15.00),(2.0 68;1.64),(-0.000;0.61) |
| 13 | 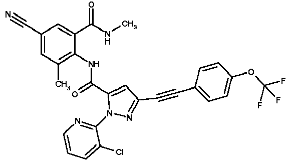 | 3,88 | 579 | (10.529;2.62),(8.520;2.86),(8.516;3.02),(8.508;3.10),(8.505;2.97),(8.360;0.72),(8.1 96;2.71),(8.192;2.70),(8.176;3.03),(8.172;2.81),(7.867;2.85),(7.798;0.84),(7.791;6. 18),(7.786;2.22),(7.769;10.05),(7.639;2.82),(7.627;2.75),(7.619;2.65),(7.607;2.62), (7.523;3.05),(7.479;4.14),(7.459;3.67),(3.299;249.99),(3.276;2.46),(2.691;10.00),( 2.679;10.01),(2.665;0.39),(2.539;0.63),(2.523;1.03),(2.509;16.11),(2.505;31.86),(2 .500;42.61),(2.496;30.03),(2.491;14.04),(2.224;15.00),(2.068;0.34),(-0.000;0.43) |
| 14 | 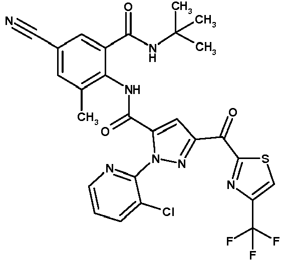 | 3,57 | 616 | (10.676;0.76),(8.975;1.15),(8.548;0.62),(8.545;0.65),(8.537;0.67),(8.533;0.64),(8.2 45;0.60),(8.242;0.59),(8.225;0.67),(8.222;0.63),(8.193;1.06),(7.846;0.74),(7.843;0. 77),(7.766;0.95),(7.712;0.83),(7.707;0.79),(7.691;0.63),(7.679;0.60),(7.671;0.58),( 7.659;0.57),(3.290;61.94),(2.539;0.46),(2.522;0.42),(2.509;5.91),(2.504;11.51),(2. 500;15.28),(2.495;10.83),(2.491;5.11),(2.234;3.36),(1.260;15.00),(-0.000;1.04) |
| 15 | 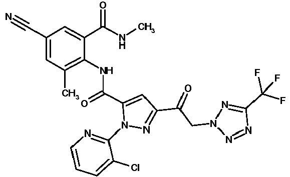 | 2,89 | 573 | (10.637;0.74),(8.568;3.45),(8.565;3.56),(8.557;3.59),(8.553;3.30),(8.533;0.43),(8.5 24;0.38),(8.343;0.92),(8.335;0.85),(8.317;0.62),(8.238;1.91),(8.219;1.95),(7.869;1. 86),(7.770;1.75),(7.692;1.57),(7.681;1.75),(7.673;1.69),(7.662;1.41),(7.229;0.55),( 7.210;0.76),(7.190;0.59),(6.694;13.03),(6.489;0.66),(6.327;0.97),(6.306;0.86),(5.7 46;6.51),(5.344;1.14),(3.755;0.48),(3.451;0.49),(3.393;1.00),(3.379;1.36),(3.308;1 853.88),(3.284;52.64),(3.225;0.83),(3.222;0.77),(3.192;0.50),(3.184;0.42),(3.177;0 .42),(2.859;2.41),(2.731;0.44),(2.677;8.46),(2.669;9.78),(2.621;0.60),(2.581;1.67), (2.565;15.00),(2.539;3.75),(2.522;8.30),(2.509;118.29),(2.505;222.13),(2.500;290. 90),(2.496;201.03),(2.491;96.93),(2.405;0.94),(2.384;0.42),(2.358;0.41),(2.332;1.6 2),(2.327;2.10),(2.322;1.72),(2.290;0.42),(2.276;0.78),(2.214;6.07),(2.132;0.84),(2 .096;0.44),(2.069;0.86),(2.060;0.60),(2.056;0.68),(1.987;1.06),(1.979;3.77),(1.974; 3.78),(1.942;1.04),(1.937;1.21),(1.908;0.68),(1.398;1.10),(1.235;0.89),(1.193;0.36) ,(1.175;0.50),(0.890;0.79),(0.859;2.38),(-0.000;18.75),(-0.008;0.80) |
| 16 | 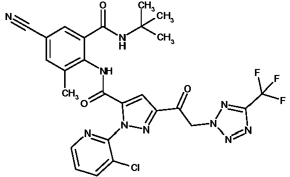 | 3,66 | 615 | (10.559;0.88),(8.557;0.59),(8.554;0.62),(8.546;0.68),(8.542;0.60),(8.245;0.56),(8.2 41;0.59),(8.224;0.62),(8.220;0.57),(7.891;1.23),(7.841;0.78),(7.793;0.93),(7.699;1. 35),(7.687;0.62),(7.678;0.62),(7.666;0.58),(6.698;2.39),(3.382;0.61),(3.303;675.53 ),(3.280;8.35),(2.673;0.79),(2.669;1.03),(2.664;0.81),(2.539;2.14),(2.508;62.10),(2 .504;110.51),(2.500;139.90),(2.495;95.96),(2.491;46.52),(2.331;0.78),(2.327;0.98) ,(2.322;0.74),(2.220;3.38),(2.069;1.19),(1.241;15.00),(1.222;0.51),(-0.000;18.33),(-0.008;0.76) |
| 17 | 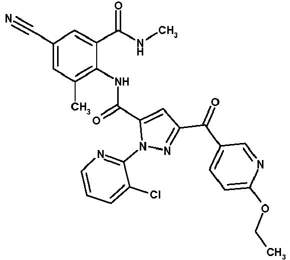 | 2,99 | 544 | (10.639;4.11),(10.175;0.52),(9.119;4.43),(9.113;4.56),(8.551;3.44),(8.542;3.34),(8. 539;3.45),(8.462;2.82),(8.456;2.78),(8.440;2.96),(8.434;2.98),(8.412;0.37),(8.339; 1.38),(8.235;2.93),(8.215;3.10),(7.994;0.66),(7.911;2.56),(7.877;3.03),(7.801;0.76) ,(7.798;0.77),(7.764;4.04),(7.677;2.51),(7.666;2.59),(7.657;2.53),(7.646;2.30),(7.5 12;0.60),(6.983;4.84),(6.962;4.97),(5.747;0.42),(4.445;2.46),(4.428;7.40),(4.410;7. 46),(4.392;2.45),(3.957;0.69),(3.427;0.47),(3.304;457.99),(3.234;0.43),(3.231;0.39 ),(2.859;0.35),(2.742;0.41),(2.732;0.61),(2.686;13.10),(2.675;12.24),(2.634;0.47),( 2.619;0.45),(2.580;0.79),(2.539;6.39),(2.501;135.51),(2.327;0.95),(2.277;2.77),(2. 239;14.53),(2.132;2.49),(2.070;1.41),(2.049;0.30),(1.987;0.51),(1.360;7.59),(1.342 ;15.00),(1.325;7.28),(1.293;0.41),(1.236;1.22),(1.175;0.40),(-0.000;6.60) |
| 18 | 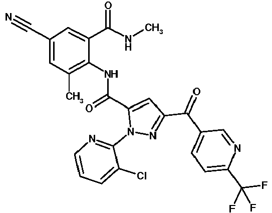 | 3,01 | 568 | (10.676;3.73),(9.398;2.95),(8.763;1.83),(8.744;1.84),(8.739;1.68),(8.548;2.82),(8.5 44;2.84),(8.536;2.89),(8.532;2.58),(8.354;1.76),(8.342;1.72),(8.239;2.72),(8.235;2. 54),(8.219;2.95),(8.215;2.55),(8.156;3.18),(8.136;2.88),(8.005;4.39),(7.889;3.43),( 7.886;3.40),(7.769;3.80),(7.766;3.38),(7.679;2.62),(7.668;2.56),(7.659;2.42),(7.64 7;2.32),(5.746;1.06),(3.305;946.34),(2.687;9.85),(2.676;10.12),(2.628;0.31),(2.539 ;2.95),(2.505;136.08),(2.500;169.46),(2.496;119.21),(2.383;0.37),(2.332;1.05),(2.3 27;1.31),(2.322;1.04),(2.291;0.33),(2.278;0.86),(2.245;15.00),(2.132;0.66),(2.069; 0.59),(1.987;0.73),(1.398;1.26),(1.236;1.18),(1.175;0.43),(0.008;0.44),(-0.000;8.71) |
| 19 | 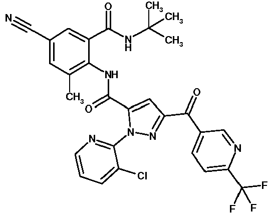 | 3,85 | 610 | (10.616;0.74),(9.388;0.64),(8.757;0.35),(8.754;0.36),(8.744;0.38),(8.741;0.39),(8.5 32;0.45),(8.530;0.51),(8.524;0.50),(8.522;0.51),(8.239;0.33),(8.225;0.36),(8.156;0. 69),(8.143;0.66),(8.026;0.66),(7.867;0.83),(7.715;0.53),(7.669;0.28),(7.663;0.29),( 5.761;0.43),(3.568;0.45),(3.386;0.93),(3.360;342.69),(3.336;5.04),(3.329;0.30),(2. 616;0.34),(2.525;0.94),(2.522;1.17),(2.519;1.27),(2.510;17.53),(2.507;37.51),(2.50 4;51.34),(2.501;37.25),(2.498;17.32),(2.388;0.32),(2.240;1.86),(2.077;0.46),(1.910 ;0.32),(1.255;13.50),(-0.000;0.77) |
| 20 | 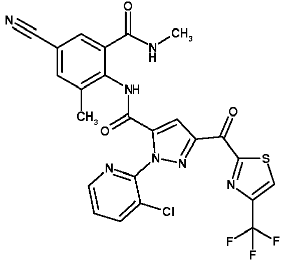 | 2,77 | 574 | (10.775;2.80),(8.973;4.95),(8.972;4.87),(8.563;2.74),(8.560;3.00),(8.552;3.00),(8.5 48;2.89),(8.341;1.39),(8.329;1.40),(8.318;0.49),(8.252;2.75),(8.248;2.77),(8.231;3. 09),(8.228;2.87),(8.168;3.59),(7.882;3.15),(7.879;3.31),(7.765;3.57),(7.761;3.27),( 7.694;2.91),(7.682;2.81),(7.673;2.74),(7.661;2.72),(4.058;0.70),(4.040;2.11),(4.02 2;2.12),(4.004;0.74),(3.622;0.34),(3.340;0.35),(3.290;320.52),(2.691;9.32),(2.679; 9.81),(2.668;0.96),(2.664;0.62),(2.538;0.71),(2.522;2.52),(2.517;4.04),(2.508;38.3 7),(2.504;76.37),(2.499;102.94),(2.495;72.38),(2.490;33.42),(2.330;0.50),(2.326;0. 66),(2.321;0.45),(2.241;15.00),(2.068;0.83),(1.986;9.51),(1.399;3.66),(1.237;0.41), (1.193;2.64),(1.175;5.37),(1.157;2.56),(-0.000;6.67) |
| 21 | 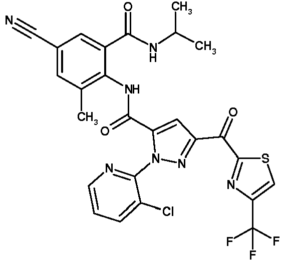 | 3,24 | 602 | (10.727;2.06),(8.974;3.48),(8.973;3.39),(8.545;1.91),(8.542;2.06),(8.534;2.10),(8.5 30;2.02),(8.236;1.94),(8.232;1.93),(8.215;2.32),(8.212;2.32),(8.203;2.91),(8.173;1. 28),(8.154;1.28),(7.874;2.25),(7.871;2.36),(7.731;2.50),(7.727;2.31),(7.686;2.01),( 7.675;1.96),(7.666;1.87),(7.654;1.88),(4.040;0.49),(4.022;0.49),(3.948;0.57),(3.93 2;0.87),(3.913;0.83),(3.897;0.59),(3.300;108.65),(2.673;0.33),(2.668;0.44),(2.664; 0.31),(2.538;0.43),(2.522;1.59),(2.517;2.52),(2.508;24.74),(2.504;49.28),(2.499;66 .35),(2.495;46.51),(2.490;21.53),(2.326;0.42),(2.249;10.36),(2.068;0.52),(1.986;2. 16),(1.399;3.12),(1.193;0.63),(1.175;1.21),(1.158;0.61),(1.061;15.00),(1.044;14.84 ),(-0.000;5.15) |
| 22 | 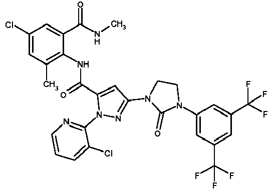 | 4,43 | 700 | (10.271;1.47),(8.493;3.21),(8.489;3.38),(8.481;3.52),(8.477;3.35),(8.340;7.59),(8.1 85;1.40),(8.174;1.38),(8.137;3.12),(8.133;3.08),(8.117;3.47),(8.113;3.16),(7.771;3. 64),(7.758;5.94),(7.585;3.27),(7.573;3.10),(7.564;2.99),(7.553;3.09),(7.455;2.82),( 7.450;3.09),(7.332;3.39),(7.326;3.14),(4.208;1.45),(4.190;2.64),(4.181;1.84),(4.16 8;2.44),(4.051;2.36),(4.037;2.09),(4.028;2.63),(4.022;1.03),(4.010;1.32),(3.303;94 3.61),(3.282;3.11),(2.679;11.18),(2.668;11.68),(2.539;2.21),(2.522;4.95),(2.517;7. 51),(2.509;75.70),(2.504;144.01),(2.500;190.84),(2.495;129.18),(2.491;59.60),(2.3 36;0.44),(2.331;0.95),(2.327;1.34),(2.322;0.90),(2.318;0.41),(2.169;16.00),(2.069; 1.06),(1.987;1.20),(1.398;0.41),(1.237;2.53),(1.193;0.36),(1.175;0.70),(1.157;0.37) ,(0.008;0.91),(-0.000;24.12),(-0.009;0.76) |
| 23 | 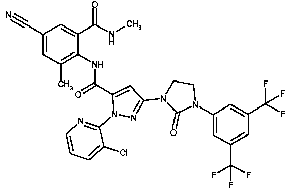 | 4,08 | 691 | (10.535;4.53),(8.503;0.50),(8.497;3.16),(8.493;3.40),(8.485;3.42),(8.481;3.22),(8.3 41;8.02),(8.324;1.36),(8.315;1.91),(8.302;1.79),(8.142;3.22),(8.139;3.20),(8.122;3. 57),(8.118;3.16),(7.860;3.47),(7.857;3.63),(7.787;7.64),(7.773;3.75),(7.749;3.99),( 7.745;3.71),(7.590;3.33),(7.578;3.12),(7.570;3.15),(7.558;3.09),(7.552;1.12),(4.21 1;1.59),(4.194;3.01),(4.184;1.97),(4.171;2.52),(4.140;0.68),(4.134;0.55),(4.056;2.8 2),(4.039;4.96),(4.032;2.87),(4.021;3.81),(4.013;1.62),(4.003;1.30),(3.756;0.67),(3 .361;0.68),(3.354;0.84),(3.300;1105.50),(2.689;10.86),(2.677;11.26),(2.669;2.75),( 2.664;2.45),(2.652;1.02),(2.539;3.57),(2.522;8.46),(2.517;13.57),(2.509;123.25),(2 .504;229.19),(2.500;299.50),(2.495;201.66),(2.491;92.17),(2.336;0.74),(2.331;1.51 ),(2.326;1.93),(2.322;1.39),(2.317;0.60),(2.221;16.00),(2.069;1.67),(1.987;13.51),( 1.837;0.50),(1.831;0.93),(1.398;1.03),(1.237;1.32),(1.193;3.86),(1.175;7.67),(1.15 7;3.84),(0.008;1.21),(-0.000;28.43),(-0.008;0.85) |
| 24 | 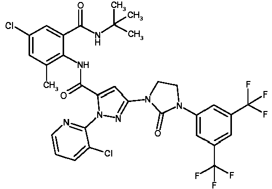 | 5,29 | 742 | (10.218;0.94),(8.483;0.67),(8.479;0.70),(8.471;0.73),(8.467;0.71),(8.336;1.74),(8.1 39;0.67),(8.135;0.67),(8.118;0.75),(8.115;0.68),(7.801;2.10),(7.769;0.78),(7.587;0. 69),(7.575;0.66),(7.566;0.66),(7.555;0.65),(7.489;1.02),(7.426;0.70),(7.420;0.76),( 7.254;0.79),(7.247;0.74),(4.202;0.33),(4.185;0.60),(4.176;0.42),(4.163;0.55),(4.04 9:0.52),(4.036:0.48),(4.027:0.60),(3.305:134.81),(2.522:0.90),(2.518:1.40),(2.509: 13.53),(2.505;25.66),(2.500;33.89),(2.496;23.38),(2.491;11.06),(2.165;3.53),(1.98 7;0.55),(1.254;16.00),(1.175;0.32),(-0.000;2.12) |
| 25 | 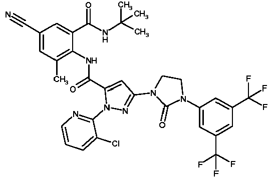 | 4,84 | 733 | (10.4433:1.00),(8.484;0.64),(8.480;0.69),(8.472;0.70),(8.468;0.67),(8.338;1.84),(8.1 38;0.62),(8.134;0.61),(8.118;0.67),(8.114;0.64),(7.820;1.89),(7.770;0.82),(7.689;1. 86),(7.587;0.62),(7.575;0.60),(7.566;0.59),(7.555;0.59),(4.205;0.35),(4.188;0.62),( 4.179;0.46),(4.165;0.58),(4.052;0.56),(4.039;0.62),(4.030;0.64),(4.021;0.33),(4.01 2;0.33),(3.304;203.13),(3.280;2.47),(2.539;0.36),(2.522;1.18),(2.518;1.85),(2.509; 17.61),(2.505;33.34),(2.500;44.02),(2.496;30.12),(2.491;14.11),(2.222;3.43),(1.98 7;0.59),(1.290;0.66),(1.259;16.00),(1.175;0.35),(1.107;0.57),(-0.000;3.10) |
| 26 | 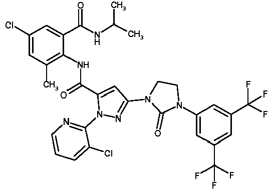 | 4,97 | 728 | (10.236;2.96),(8.480;2.20),(8.476;2.35),(8.469;2.41),(8.465;2.30),(8.340;5.46),(8.1 27;2.19),(8.123;2.17),(8.107;2.44),(8.103;2.25),(7.984;1.56),(7.965;1.58),(7.788;6. 09),(7.770;2.51),(7.582;2.29),(7.570;2.16),(7.562;2.10),(7.550;2.16),(7.449;2.21),( 7.447;2.23),(7.443;2.40),(7.292;2.54),(7.286;2.37),(5.747;0.91),(4.206;1.04),(4.18 8;1.89),(4.179;1.31),(4.165;1.71),(4.049;1.66),(4.036;1.49),(4.027;1.87),(4.009;0.9 1),(3.950;0.64),(3.933;0.96),(3.914;0.93),(3.898;0.63),(3.310;241.62),(2.540;0.36), (2.523;1.14),(2.510;15.41),(2.505;28.56),(2.501;37.18),(2.496;25.31),(2.492;11.74 ),(2.176;11.11),(1.236;0.83),(1.059;16.00),(1.043;15.80),(-0.000;1.92) |
| 27 | 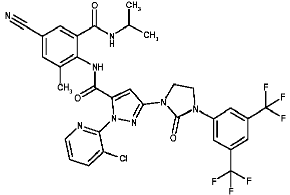 | 4,55 | 719 | (10.493;3.34),(8.482;2.09),(8.478;2.18),(8.470;2.26),(8.467;2.14),(8.341;6.10),(8.1 29;2.88),(8.126;3.63),(8.109;3.35),(8.106;3.55),(7.853;2.59),(7.850;2.68),(7.814;5. 39),(7.772;2.78),(7.715;2.84),(7.711;2.71),(7.584;2.08),(7.573;2.00),(7.564;1.90),( 7.552;1.90),(5.747;3.75),(4.209;1.12),(4.191;2.05),(4.182;1.45),(4.169;1.91),(4.05 3;1.82),(4.039;1.62),(4.031;2.04),(4.012;1.03),(3.948;0.70),(3.931;1.07),(3.913;1.0 5),(3.896;0.68),(3.305;178.70),(2.670;0.33),(2.540;0.41),(2.523;1.29),(2.509;18.63 ),(2.505;34.66),(2.501;45.04),(2.496;31.34),(2.492;14.92),(2.233;11.39),(1.477;0.4 0),(1.264;0.44),(1.248;0.51),(1.236;0.69),(1.120;0.32),(1.103;0.34),( 1.063;16.00),( 1.046;15.82),(-0.000;2.14) |
| 28 | 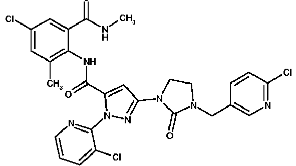 | 2,64 | 613 | (10.262;4.03),(8.466;3.17),(8.462;3.37),(8.455;3.48),(8.451;3.32),(8.415;3.19),(8.4 10;3.24),(8.188;0.62),(8.177;1.74),(8.166;1.72),(8.155;0.59),(8.108;3.14),(8.105;3. 16),(8.088;3.55),(8.084;3.28),(7.847;2.12),(7.841;2.04),(7.827;2.39),(7.820;2.33),( 7.586;7.90),(7.554;3.45),(7.543;6.99),(7.534;3.31),(7.522;6.54),(7.447;3.07),(7.44 6;3.11),(7.441;3.43),(7.326;3.68),(7.319;3.39),(5.747;1.72),(4.482;8.54),(4.039;0.5 1),(4.021;0.55),(3.850;1.87),(3.832;2.94),(3.826;2.24),(3.810;2.25),(3.485;2.35),(3 .464;2.88),(3.4455:1.84),(3.305;325.95),(2.678;11.00),(2.666;10.90),(2.539;0.66),(2 .522;1.96),(2.517;3.05),(2.509;28.85),(2.504;54.37),(2.500;71.65),(2.496;48.90),(2 .491;22.92),(2.331;0.37),(2.327;0.50),(2.322;0.37),(2.158;16.00),(2.070;0.39),(1.9 87;2.37),(1.193;0.69),(1.175;1.37),(1.157;0.67),(-0.000;3.04) |
| 29 | 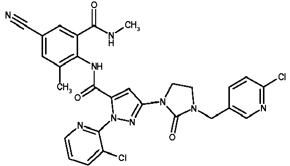 | 2,31 | 604 | (10.538;4.58),(8.470;2.83),(8.466;2.98),(8.458;3.06),(8.455;2.94),(8.416;3.57),(8.4 10;3.59),(8.322;0.72),(8.311;1.83),(8.300;1.84),(8.113;2.74),(8.110;2.72),(8.093;3. 04),(8.090;2.82),(7.848;5.79),(7.827;2.46),(7.821;2.34),(7.743;3.98),(7.739;3.78),( 7.613;7.31),(7.559;2.91),(7.548;3.33),(7.543;4.87),(7.527;3.11),(7.523;3.94),(7.44 5;0.70),(5.747;2.64),(4.484;9.31),(3.854;2.00),(3.836;3.18),(3.814;2.43),(3.714;1.3 4),(3.518;0.33),(3.488;2.60),(3.468;3.25),(3.449;2.08),(3.420;0.33),(3.401;0.38),(3 .309;952.42),(3.252;0.56),(2.687;10.28),(2.675;10.79),(2.539;1.35),(2.523;4.50),(2 .509;64.64),(2.505;120.25),(2.500;156.42),(2.496;108.56),(2.492;51.43),(2.332;0.7 8),(2.327;1.03),(2.322;0.75),(2.210;16.00),(1.987;0.53),(1.941;0.96),(1.236;0.74),( 1.175;0.36),(-0.000;7.18) |
| 30 | 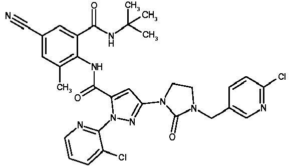 | 3,02 | 646 | (10.424;1.01),(8.458;0.70),(8.455;0.75),(8.447;0.77),(8.443;0.74),(8.413;0.74),(8.4 08;0.74),(8.109;0.69),(8.105;0.70),(8.089;0.78),(8.085;0.72),(7.846;0.49),(7.840;0. 48),(7.825;0.64),(7.819;1.24),(7.815;0.88),(7.682;1.71),(7.638;1.81),(7.556;0.74),( 7.544;0.85),(7.541;1.00),(7.536;0.77),(7.524;0.79),(7.521;0.83),(4.481;1.94),(3.85 2;0.43),(3.834;0.67),(3.828;0.51),(3.812;0.51),(3.483;0.53),(3.463;0.65),(3.443;0.4 2),(3.305;88.33),(2.522;0.49),(2.518;0.78),(2.509;7.18),(2.505;13.53),(2.500;17.82 ),(2.496;12.20),(2.491;5.72),(2.209;3.49),(1.987;1.20),(1.257;16.00),(1.193;0.36),( 1.175;0.70),(1.157;0.34),(-0.000;0.53) |
| 31 | 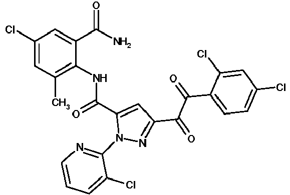 | 3,51 | 591 | (7.96;0.180),(7.94;0.240),(7.81;0.160),(7.61;0.180),(7.43;0.180),(7.42;0.210),(5.69 ;0.310),(3.16;0.540),(3.11;332.660),(3.06;0.260),(3.05;0.190),(2.66;0.230),(2.66;0. 310),(2.65;0.190),(2.53;0.700),(2.51;0.940),(2.50;16.140),(2.49;32.390),(2.49;45.1 70),(2.48;32.230),(2.48;16.000),(2.32;0.200),(2.31;0.300),(2.31;0.190),(2.17;0.750 ),(1.36;0.250),(1.24;0.300),(0.01;0.190),(-0.00;5.170),(-0.01;0.220) |
| 32 | 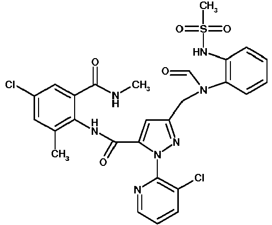 | 2,09 | 630 | (8.68;2.520),(8.54;1.080),(8.54;1.150),(8.54;1.170),(8.53;1.150),(8.52;2.450),(8.52 ;2.530),(8.51;2.640),(8.51;2.530),(8.30;7.120),(8.27;1.120),(8.26;1.120),(8.19;1.08 0),(8.19;1.150),(8.18;1.120),(8.17;1.060),(8.16;2.440),(8.16;2.410),(8.15;2.640),(8 .15;2.480),(8.03;15.520),(7.71;0.950),(7.70;0.940),(7.69;0.990),(7.69;0.950),(7.66; 1.980),(7.66;1.990),(7.64;2.440),(7.64;3.110),(7.63;1.080),(7.63;1.080),(7.62;1.10 0),(7.62;2.460),(7.61;2.410),(7.60;2.350),(7.60;2.330),(7.47;3.870),(7.47;3.790),(7 .47;3.430),(7.46;1.550),(7.45;1.230),(7.45;3.650),(7.44;2.960),(7.43;0.940),(7.43;0 .960),(7.42;1.730),(7.40;0.950),(7.40;0.940),(7.37;1.080),(7.37;2.310),(7.32;4.160) ,(7.32;1.790),(7.32;1.700),(7.30;2.090),(7.30;1.970),(7.21;1.210),(7.21;0.930),(7.2 0;1.740),(5.03:5.430),(3.52:13.060),(3.17:15.000),(3.08:9.160),(2.95:17.820),(2.92 ;9.200),(2.92;18.680),(2.92;25.870),(2.91;18.280),(2.91;8.810),(2.81;12.810),(2.81 ;12.790),(2.79;17.540),(2.75;10.300),(2.75;20.340),(2.75 ;28.540),(2.74;20.370),(2. 74;9.890),(2.40;1.820),(2.25;16.000),(2.24;6.720),(0.00;7.860) |
| 33 | 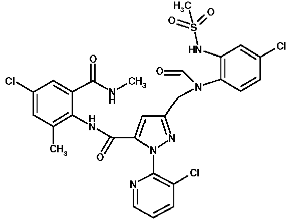 | 2,49 | 664 | |
| 34 | 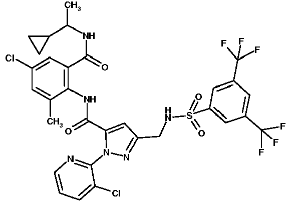 | 4,38 | 763 | (10.03;0.330),(8.40;1.120),(8.40;1.120),(8.39;1.150),(8.38;1.070),(8.36;2.910),(8.2 9;1.240),(8.03;1.010),(8.03;0.930),(8.01;1.080),(8.01;1.010),(7.97;0.280),(7.95;0.5 70),(7.93;0.540),(7.53;1.030),(7.52;0.990),(7.51;0.940),(7.49;0.880),(7.41;1.140),( 7.41;1.210),(7.32;1.240),(7.31;1.050),(7.14;1.840),(4.26;4.280),(3.39;0.340),(3.38; 0.550),(3.36;0.540),(3.34;0.330),(3.21;0.320),(3.19;0.530),(3.12;195.600),(3.07;0. 390),(2.66;0.300),(2.53;2.450),(2.50;17.530),(2.49;33.800),(2.49;45.830),(2.48;32. 500),(2.48;16.000),(2.32;0.280),(2.31;0.320),(2.13;6.220),(2.04;0.260),(1.36;0.280 ),(1.23;1.040),(1.22;1.010),(1.10;3.630),(1.08;3.580),(0.89;0.300),(0.88;0.320),(0. 87;0.600),(0.86;0.430),(0.85;0.560),(0.84;0.340),(0.83;0.260),(0.54;0.330),(0.52;0. 330),(0.40;0.330),(0.39;0.520),(0.38;0.510),(0.37;0.530),(0.36;0.330),(0.35;0.230), (0.31;0.260),(0.30;0.410),(0.28;0.440),(0.28;0.520),(0.26;0.510),(0.26;0.250),(0.24 ;0.320),(0.23;0.320),(0.22;0.460),(0.21;0.580),(0.19;0.470),(0.17;0.290),(0.16;0.50 0),(0.15;0.570),(0.13;0.440),(0.12;0.270),(-0.00;5.860) |
| 35 | 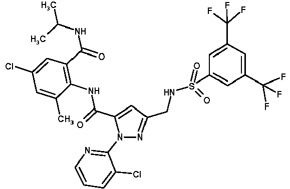 | 4,1 | 737 | (10.03;0.800),(8.56;0.170),(8.40;1.620),(8.39;1.620),(8.39;1.630),(8.38;1.490),(8.3 5;4.440),(8.29;1.850),(8.03;1.470),(8.03;1.400),(8.01;1.560),(8.01;1.430),(7.88;0.1 90),(7.87;0.820),(7.85;0.810),(7.53;1.550),(7.51;1.490),(7.51;1.430),(7.49;1.310),( 7.40;1.790),(7.40;1.900),(7.31;2.020),(7.31;1.670),(7.14;2.720),(4.27;6.340),(4.20; 0.170),(3.97;0.180),(3.96;0.530),(3.94;0.770),(3.92;0.760),(3.90;0.520),(3.89;0.19 0),(3.29;0.160),(3.16;0.600),(3.12;134.430),(2.66;0.280),(2.53;2.240),(2.52;0.540), (2.50;17.440),(2.49;33.400),(2.49;45.180),(2.48;32.220),(2.48;16.000),(2.31;0.330 ),(2.29;0.290),(2.13;9.990),(1.36;0.770),(1.24;0.240),(1.19;0.860),(1.17;0.810),(1. 11;0.170),(1.10;0.330),(1.07;12.150),(1.05;12.140),(0.86;0.260),(0.84;0.260),(0.83 ;0.260),(0.81;0.200),(-0.00;7.970) |
| 36 | 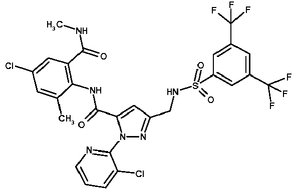 | 3,62 | 709 | (10.07;0.570),(8.41;0.690),(8.40;0.720),(8.40;0.750),(8.39;0.710),(8.35;1.880),(8.2 9;0.800),(8.04;0.960),(8.04;0.880),(8.02;0.810),(8.02;0.780),(7.53;0.730),(7.52;0.7 50),(7.51;0.690),(7.50;0.680),(7.41;0.720),(7.40;0.830),(7.34;0.870),(7.34;0.790),( 7.12;1.220),(4.27;2.120),(3.25;0.170),(3.18;0.500),(3.17;0.690),(3.12;238.920),(3. 08;0.270),(3.08;0.300),(3.06;0.200),(2.69;2.760),(2.68;2.700),(2.66;0.270),(2.66;0. 330),(2.53;2.650),(2.51;1.200),(2.50;17.760),(2.49;34.530),(2.49;47.050),(2.48;33. 050),(2.48;16.000),(2.32;0.200),(2.32;0.260),(2.31;0.320),(2.13;4.390),(1.36;0.300 ),(1.24;0.170),(-0.00;5.680) |
| 37 | 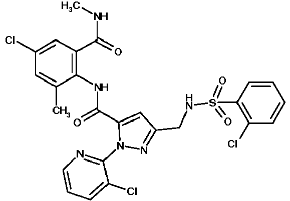 | 2,58 | 607 | (8.44;0.850),(8.44;0.870),(8.43;0.910),(8.42;0.810),(8.06;0.960),(8.06;0.960),(8.04 ;1.140),(8.04;1.110),(7.99;0.830),(7.99;0.790),(7.98;0.900),(7.97;0.890),(7.94;0.21 0),(7.61;0.320),(7.60;0.440),(7.59;1.270),(7.58;1.380),(7.58;0.780),(7.56;1.090),(7 .56;0.850),(7.54;0.510),(7.54;1.020),(7.52;0.960),(7.51;0.860),(7.50;0.930),(7.50;0 .720),(7.50;0.590),(7.48;0.800),(7.46;0.470),(7.46;0.470),(7.41;0.980),(7.40;1.050) ,(7.35;1.210),(7.34;1.000),(7.09;1.670),(6.87;0.830),(6.39;0.320),(4.22;3.860),(4.1 8;0.490),(3.60;0.190),(3.31;0.160),(3.29;0.180),(3.27;0.210),(3.22;0.340),(3.11;12 5.070),(2.73;0.240),(2.71;3.270),(2.70;3.260),(2.66;0.400),(2.65;0.300),(2.53;1.07 .0),(2.50;20.220),(2.49;36.300),(2.49;46.540),(2.48;32.590),(2.48;16.000),(2.45;0.2 10),(2.38;3.210),(2.32;0.250),(2.31;0.280),(2.29;0.930),(2.18;1.360),(2.14;5.700),( 2.04;0.190),(1.76;0.180),(1.36;10.740),(1.25;0.200),(1.19;0.210),(1.17;0.190),(-0.00;9.080) |
| 38 | 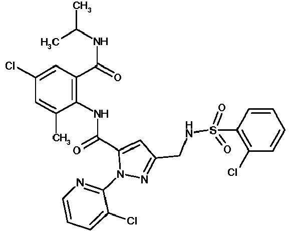 | 3,06 | 635 | (8.43;1.010),(8.42;1.000),(8.41;1.030),(8.41;0.960),(8.05;0.970),(8.05;0.900),(8.03 ;1.010),(8.03;0.950),(8.02;0.260),(7.99;1.000),(7.97;1.290),(7.97;1.060),(7.96;0.29 0),(7.84;0.590),(7.82;0.570),(7.60;0.420),(7.59;1.520),(7.58;2.010),(7.56;1.050),(7 .56;0.870),(7.55;0.420),(7.54;0.420),(7.53;1.000),(7.52;1.000),(7.51;0.960),(7.50;1 .390),(7.49;0.680),(7.48;0.850),(7.48;0.910),(7.46;0.570),(7.46;0.460),(7.40;1.190) ,(7.40;1.210),(7.32;1.250),(7.31;1.090),(7.12;1.750),(6.87;1.000),(6.39;0.380),(4.2 2;4.450),(4.14;0.600),(3.98;0.330),(3.96;0.500),(3.94;0.470),(3.93;0.380),(3.93;0.3 40),(3.31;0.240),(3.31;0.210),(3.30;0.490),(3.28;0.570),(3.26;0.510),(3.25;0.350),( 3.11;127.470),(3.01;0.320),(2.99;0.230),(2.66;0.330),(2.53;1.020),(2.50;21.310),(2 .49;37.380),(2.49;46.790),(2.48;32.700),(2.48;16.000),(2.32;0.260),(2.31;0.310),(2 .30;1.120),(2.18;1.580),(2.17;0.370),(2.15;6.170),(2.04;0.310),(1.36;12.100),(1.31; 0.270),(1.25;0.300),(1.19;4.470),(1.17;4.250),(1.12;0.190),(1.11;0.230),(1.08;7.30 0),(1.06;7.150),(0.86;0.200),(0.84;0.240),(-0.00;3.990) |
| 39 | 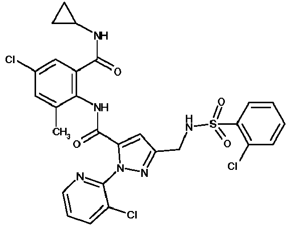 | 2,8 | 633 | (10.01;0.620),(8.44;1.540),(8.44;1.350),(8.43;1.570),(8.42;1.230),(8.10;0.440),(8.0 8;0.910),(8.06;1.570),(8.06;1.290),(8.04;1.530),(8.04;1.270),(8.00;1.310),(7.99;1.2 80),(7.98;1.430),(7.97;1.250),(7.97;1.310),(7.61;0.730),(7.59;2.140),(7.59;2.450),( 7.58;1.520),(7.57;1.660),(7.57;1.550),(7.55;0.760),(7.54;1.500),(7.52;1.450),(7.52; 1.400),(7.50;2.050),(7.48;1.190),(7.47;0.630),(7.46;0.630),(7.40;1.470),(7.40;1.53 0),(7.30;1.780),(7.29;1.550),(7.12;2.430),(7.07;0.430),(6.76;0.510),(4.25;0.610),(4 .23;5.170),(4.20;1.100),(3.81;0.430),(3.80;0.530),(3.47;0.470),(3.12;36.230),(2.75; 0.530),(2.74;0.690),(2.73;0.650),(2.73;0.510),(2.57;0.440),(2.55;0.480),(2.54;0.58 0),(2.53;1.200),(2.50;20.470),(2.49;36.240),(2.49;46.040),(2.48;32.380),(2.48;16.0 00),(2.41;0.430),(2.30;2.070),(2.18;0.520),(2.14;8.360),(2.12;1.510),(1.74;0.450),( 1.36;1.120),(1.19;1.010),(1.17;0.610),(0.70;0.580),(0.69;0.560),(0.68;0.890),(0.66; 1.090),(0.66;1.100),(0.64;1.620),(0.63;1.600),(0.61;0.630),(0.50;0.700),(0.49;1.78 0),(0.48;1.790),(0.48;1.440),(0.46;0.520),(-0.00;1.720) |
| 40 | 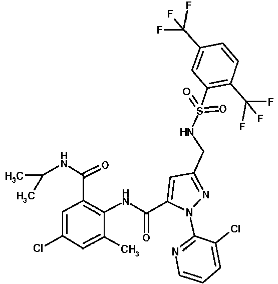 | 3,84 | 737 | (8.41;2.35),(8.41;2.93),(8.40;4.77),(8.40;3.73),(8.38;0.47),(8.38;0.45),(8.37;0.42),( 8.37;0.41),(8.14;0.60),(8.12;3.82),(8.12;2.77),(8.10;0.85),(8.10;0.87),(8.04;2.26),( 8.04;2.29),(8.02;2.42),(8.02;2.31),(7.96;0.23),(7.96;0.22),(7.94;0.25),(7.94;0.26),( 7.85;0.92),(7.83;0.95),(7.53;2.37),(7.52;2.34),(7.51;2.22),(7.50;2.16),(7.46;0.24),( 7.45;0.23),(7.44;0.23),(7.43;0.22),(7.40;2.01),(7.40;2.07),(7.39;2.40),(7.39;2.27),( 7.31;2.30),(7.31;2.11),(7.14;3.27),(6.87;0.57),(6.46;0.24),(4.57;0.24),(4.33;0.32),( 4.31;8.42),(4.20;1.24),(3.96;0.61),(3.95;0.93),(3.93;0.93),(3.91;0.65),(3.89;0.26),( 3.28;0.32),(3.26;0.45),(3.24;0.37),(3.11;6.59),(2.86;0.46),(2.53;0.70),(2.51;0.57),( 2.50;9.40),(2.49;18.80),(2.49;26.12),(2.48;18.49),(2.48;9.13),(2.30;0.60),(2.18;0.8 9),(2.13;12.54),(1.36;7.82),(1.19;0.27),(1.17;4.37),(1.15;4.29),(1.13;0.49),(1.12;0. 49),(1.10;0.28),(1.10;0.31),(1.07;16.00),(1.05;15.86),(1.02;0.63),(1.01;0.57),(0.84; 0.80),(0.83;0.27),(0.81;0.25),(-0.00;4.34) |
| 41 | 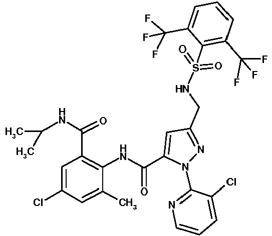 | 3,63 | 737 | (8.41;2.11),(8.41;2.24),(8.40;2.23),(8.40;2.17),(8.19;4.54),(8.17;5.37),(8.16;0.67),( 8.14;0.21),(8.04;2.36),(8.04;2.45),(8.02;2.69),(8.02;2.58),(8.00;0.42),(7.99;0.40),( 7.97;0.44),(7.97;0.43),(7.96;0.43),(7.95;0.43),(7.93;1.21),(7.91;1.72),(7.89;0.87),( 7.82;1.00),(7.81;1.00),(7.53;2.36),(7.51;2.33),(7.51;2.30),(7.49;2.19),(7.40;1.98),( 7.40;2.05),(7.39;2.31),(7.39;2.16),(7.30;2.29),(7.30;2.05),(7.13;3.15),(6.88;0.27),( 4.31;7.85),(4.24;0.53),(3.98;0.23),(3.96;0.59),(3.94;0.92),(3.92;0.89),(3.91;0.63),( 3.89;0.22),(3.68;0.90),(3.31;0.49),(3.30;1.20),(3.28;1.63),(3.26;1.27),(3.25;0.57),( 3.11;18.81),(2.86;0.43),(2.66;0.22),(2.53;0.93),(2.51;0.76),(2.50;12.11),(2.49;24.2 2),(2.49;33.58),(2.48;23.79),(2.48;11.70),(2.31;0.22),(2.30;0.64),(2.18;0.24),(2.14; 12.16),(1.36;1.61),(1.22;0.22),(1.20;15.72),(1.18;15.43),(1.13;0.45),(1.12;0.46),(1. 10;0.28),(1.09;0.41),(1.07;16.00),(1.05;15.80),(0.86;0.52),(0.84;0.74),(0.84;0.53),( 0.83;0.30),(0.83;0.31),(0.81;0.29),(-0.00;3.99) |
| 42 | 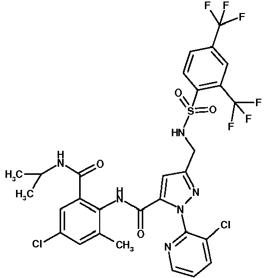 | 3,94 | 737 | (8.41;2.09),(8.41;2.27),(8.40;2.42),(8.40;2.44),(8.39;0.39),(8.39;0.34),(8.37;0.17),( 8.34;1.43),(8.32;1.91),(8.28;0.32),(8.19;1.37),(8.17;1.08),(8.14;0.21),(8.12;2.26),( 8.10;0.38),(8.05;2.13),(8.04;2.19),(8.03;2.38),(8.02;2.25),(7.97;0.23),(7.97;0.20),( 7.85;0.91),(7.83;0.95),(7.53;2.32),(7.52;2.28),(7.51;2.20),(7.50;2.17),(7.49;0.17),( 7.40;1.97),(7.40;2.07),(7.40;2.37),(7.40;2.22),(7.32;2.26),(7.31;2.12),(7.14;3.07),( 6.87;0.51),(6.50;0.17),(6.40;0.21),(4.33;8.28),(4.23;0.88),(3.98;0.21),(3.96;0.60),( 3.95;0.91),(3.93;0.92),(3.91;0.62),(3.89;0.24),(3.29;0.27),(3.27;0.35),(3.26;0.29),( 3.11;6.39),(3.03;0.18),(2.86;0.56),(2.53;0.64),(2.51;0.50),(2.50;8.44),(2.49;16.91), (2.49;23.45),(2.48;16.58),(2.48;8.16),(2.30;0.62),(2.18;0.92),(2.13;12.32),(1.36;8. 50),(1.21;0.20),(1.19;0.39),(1.18;3.60),(1.16;3.53),(1.13;0.57),(1.12;0.59),(1.10;0. 28),(1.07;16.00),(1.05;15.84),(1.03;0.25),(1.01;0.20),(0.86;0.35),(0.84;0.81),(0.83; 0.23),(0.83;0.29),(0.81;0.25),(-0.00;4.06) |
| 43 | 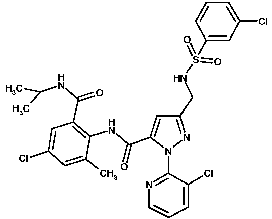 | 3,25 | 635 | (10.01;0.39),(8.43;0.50),(8.43;0.51),(8.42;0.52),(8.41;0.52),(8.06;0.51),(8.06;0.52) ,(8.04;0.54),(8.04;0.54),(7.84;0.59),(7.83;0.86),(7.83;0.58),(7.82;0.25),(7.80;0.33), (7.79;0.41),(7.79;0.27),(7.78;0.37),(7.77;0.44),(7.77;0.31),(7.67;0.25),(7.66;0.20),( 7.65;0.36),(7.65;0.41),(7.65;0.41),(7.64;0.36),(7.61;0.54),(7.59;0.69),(7.57;0.26),( 7.54;0.53),(7.52;0.54),(7.52;0.52),(7.50;0.49),(7.40;0.52),(7.40;0.55),(7.31;0.57),( 7.31;0.52),(7.14;0.78),(4.15;1.12),(3.95;0.23),(3.93;0.25),(3.11;127.21),(2.86;0.18 ),(2.66;0.24),(2.66;0.29),(2.65;0.22),(2.56;0.17),(2.53;0.58),(2.51;1.33),(2.50;16.8 5),(2.49;33.02),(2.49;45.40),(2.48;32.33),(2.48;16.00),(2.33;0.17),(2.32;0.21),(2.3 1;0.29),(2.31;0.22),(2.29;0.19),(2.18;0.17),(2.15;2.92),(1.36;1.49),(1.19;0.36),(1.1 7;0.33),(1.13;0.17),(1.11;0.21),(1.07;3.82),(1.06;3.73),(0.01;0.24),(-0.00;4.95),(-0.01;0.28) |
| 44 | 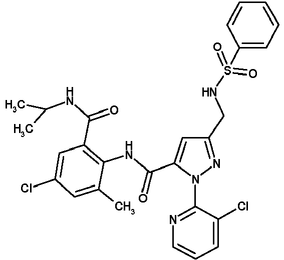 | 2,84 | 601 | (10.01;0.35),(8.43;0.35),(8.43;0.39),(8.42;0.34),(8.42;0.35),(8.13;0.21),(8.06;0.38) ,(8.06;0.38),(8.04;0.42),(8.04;0.40),(7.86;0.50),(7.85;0.54),(7.84;0.27),(7.84;0.66), (7.83;0.69),(7.81;0.20),(7.61;0.26),(7.60;0.30),(7.60;0.52),(7.59;0.66),(7.59;0.33),( 7.57;0.61),(7.56;0.19),(7.55;0.18),(7.54;0.39),(7.52;0.39),(7.52;0.38),(7.50;0.37),( 7.41;0.37),(7.40;0.39),(7.31;0.41),(7.31;0.39),(7.13;0.57),(6.87;0.18),(4.11;0.57),( 4.09;0.55),(3.96;0.18),(3.94;0.18),(3.19;0.26),(3.11;146.20),(3.05;0.17),(2.66;0.21 ),(2.66;0.29),(2.65;0.22),(2.53;0.54),(2.51;1.22),(2.50;16.79),(2.49;32.94),(2.49;45 .26),(2.48;32.20),(2.48;16.00),(2.32;0.20),(2.31;0.29),(2.31;0.20),(2.18;0.31),(2.15 ;2.13),(1.36;2.74),(1.19;0.17),(1.08;2.78),(1.06;2.75),(0.01;0.20),(-0.00;5.00) |
| 45 | 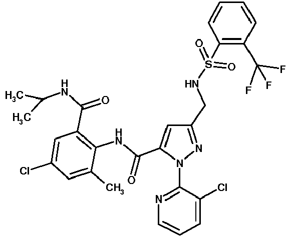 | 3,26 | 669 | (10.02;0.45),(8.45;0.19),(8.44;0.63),(8.43;0.63),(8.42;0.61),(8.42;0.62),(8.14;0.40) ,(8.11;0.46),(8.06;0.52),(8.06;0.56),(8.04;0.59),(8.04;0.52),(7.93;0.36),(7.91;0.45), (7.85;0.22),(7.83;0.56),(7.81;0.46),(7.79;0.37),(7.77;0.40),(7.76;0.17),(7.54;0.62),( 7.53;0.61),(7.52;0.61),(7.51;0.60),(7.40;0.54),(7.40;0.60),(7.31;0.61),(7.31;0.54),( 7.14;0.86),(6.87;0.18),(4.26;1.02),(3.97;0.17),(3.95;0.27),(3.94;0.26),(3.35;0.21),( 3.11;66.87),(2.86;0.19),(2.66;0.21),(2.66;0.31),(2.65;0.23),(2.57;0.18),(2.54;0.22), (2.53 ;0.50),(2.50;16.63),(2.49;32.76),(2.49;45.15),(2.48;32.21),(2.48;16.00),(2.32; 0.20),(2.31;0.31),(2.31;0.20),(2.29;0.32),(2.18;0.29),(2.16;0.20),(2.14;3.07),(2.04; 0.18),(1.40;0.24),(1.36;2.52),(1.19;0.64),(1.18;0.60),(1.13;0.19),(1.11;0.22),(1.07; 4.05),(1.06;4.01),(1.03;0.16),(0.01;0.29),(-0.00;6.56),(-0.01;0.29) |
| 46 | 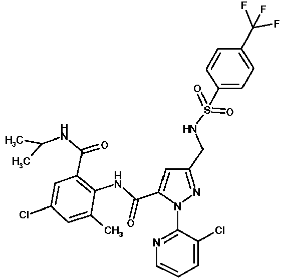 | 3,47 | 669 | (8.42;2.05),(8.41;2.17),(8.41;2.18),(8.40;2.16),(8.39;0.47),(8.38;0.46),(8.37;0.47),( 8.37;0.46),(8.05;2.49),(8.05;3.09),(8.04;2.93),(8.03;2.95),(8.03;3.68),(8.02;3.97),( 8.00;0.89),(7.97;0.24),(7.96;0.43),(7.96;0.45),(7.94;0.45),(7.94;0.45),(7.92;3.85),( 7.89;3.22),(7.88;0.79),(7.86;1.02),(7.84;0.99),(7.53;2.26),(7.52;2.22),(7.51;2.14),( 7.50;2.08),(7.46;0.38),(7.44;0.40),(7.44;0.40),(7.42;0.39),(7.40;2.06),(7.40;2.15),( 7.40;2.47),(7.40;2.30),(7.32;2.39),(7.31;2.18),(7.15;3.28),(6.87;0.70),(6.42;0.67),( 6.40;0.25),(4.19;8.85),(4.07;2.01),(3.98;0.24),(3.97;0.60),(3.95;0.93),(3.93;0.95),( 3.91;0.63),(3.90;0.25),(3.27;0.34),(3.25;0.48),(3.23;0.41),(3.22;0.27),(3.11;6.03),( 2.86;0.34),(2.53;0.49),(2.50;10.41),(2.49;20.76),(2.49;28.77),(2.48;20.60),(2.48;10 .32),(2.30;0.77),(2.18;1.23),(2.14;12.70),(1.36;11.04),(1.19;0.29),(1.16;4.48),(1.15 ;4.39),(1.13;0.40),(1.12;0.37),(1.10;0.26),(1.07;16.00),(1.05;15.90),(0.86;0.23),(0. 84;0.49),(0.83;0.22),(0.83;0.21),(-0.00;1.35) |
| 47 | 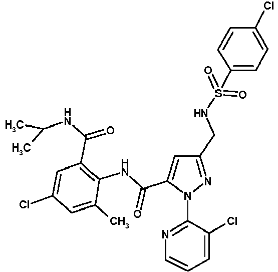 | 3,25 | 635 | (8.43;1.93),(8.43;2.16),(8.42;2.14),(8.42;2.15),(8.40;0.26),(8.40;0.29),(8.39;0.25),( 8.06;2.09),(8.05;2.21),(8.04;2.42),(8.03;2.38),(7.97;0.34),(7.96;0.28),(7.96;0.28),( 7.85;1.00),(7.84;1.26),(7.83;5.57),(7.83;2.29),(7.82;2.13),(7.81;6.40),(7.80;1.16),( 7.79;0.82),(7.62;0.84),(7.61;5.73),(7.61;2.05),(7.59;2.35),(7.59;4.84),(7.58;0.81),( 7.58;0.40),(7.57;0.68),(7.53;2.26),(7.52;2.26),(7.51;2.17),(7.50;2.14),(7.45;0.22),( 7.41;2.03),(7.40;2.12),(7.40;2.46),(7.40;2.33),(7.32;2.36),(7.31;2.17),(7.13;3.33),( 4.14;8.77),(4.03;1.03),(3.99;0.22),(3.97;0.63),(3.96;0.96),(3.94;0.94),(3.92;0.62),( 3.90;0.25),(3.28;0.36),(3.26;0.46),(3.25;0.39),(3.23;0.23),(3.18;0.30),(3.16;0.55),( 3.11;22.16),(2.86;0.61),(2.66;0.27),(2.53;0.70),(2.50;13.66),(2.49;26.76),(2.49;36. 81),(2.48;26.15),(2.48;12.98),(2.31;0.23),(2.31;0.24),(2.30;1.32),(2.15;12.57),(1.1 7;4.36),(1.16;4.25),(1.13;0.68),(1.11;0.67),(1.11;0.31),(1.07;16.00),(1.06;15.68),(0 .86;0.31),(0.84;0.46),(0.83;0.30),(-0.00;1.78) |
| 48 | 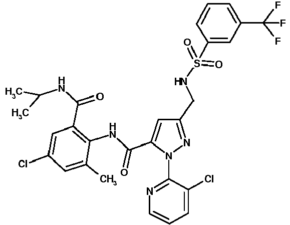 | 3,43 | 669 | (8.42;0.93),(8.42;1.05),(8.41;1.05),(8.41;1.05),(8.13;0.64),(8.11;1.65),(8.05;1.03),( 8.05;1.07),(8.03;1.15),(8.03;1.14),(7.97;0.57),(7.95;0.78),(7.92;0.16),(7.86;0.43),( 7.84;0.46),(7.83;0.69),(7.81;0.98),(7.79;0.49),(7.53;1.09),(7.52;1.09),(7.51;1.04),( 7.50;1.02),(7.40;1.08),(7.40;1.09),(7.40;1.22),(7.40;1.18),(7.32;1.17),(7.31;1.08),( 7.14;1.67),(6.87;1.03),(6.40;0.21),(6.34;0.20),(4.17;4.41),(4.03;0.49),(3.97;0.31),( 3.95;0.46),(3.93;0.46),(3.91;0.32),(3.60;0.26),(3.19;0.16),(3.11;5.61),(2.53;0.25),( 2.51;0.33),(2.50;5.14),(2.49;10.15),(2.49;13.98),(2.48;9.96),(2.48;4.97),(2.30;0.43 ),(2.18;1.84),(2.14;6.19),(1.76;0.31),(1.36;16.00),(1.19;0.26),(1.13;1.19),(1.12;1.1 8),(1.07;7.85),(1.06;7.72),(0.86;0.18),(0.84;0.39),(0.83;0.16),(-0.00;0.69) |
| 49 | 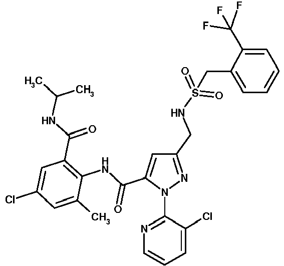 | 2,62 | 683 | (8.44;1.00),(8.44;1.03),(8.43;1.02),(8.42;0.97),(8.07;1.00),(8.07;0.98),(8.05;1.08),( 8.05;0.99),(7.82;0.51),(7.80;0.50),(7.76;0.76),(7.74;0.97),(7.73;0.50),(7.71;1.05),( 7.71;0.74),(7.69;0.75),(7.59;0.43),(7.57;0.66),(7.55;1.16),(7.53;1.05),(7.53;0.97),( 7.51;0.93),(7.40;0.98),(7.40;1.07),(7.31;1.10),(7.30;0.96),(7.24;1.43),(6.87;1.02),( 6.40;0.41),(4.54;3.03),(4.28;3.50),(3.93;0.44),(3.91;0.42),(3.11;22.82),(2.86;0.41), (2.53;0.45),(2.51;0.53),(2.50;7.94),(2.49;15.67),(2.49;21.60),(2.48;15.37),(2.48;7. 68),(2.30;0.73),(2.18;1.77),(2.15;5.77),(1.36;16.00),(1.19;1.87),(1.17;0.47),(1.17;1 .81),(1.13;0.42),(1.12;0.41),(1.05;7.41),(1.04;7.39),(-0.00;0.65) |
| 50 | 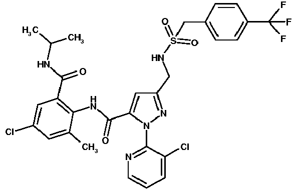 | 2,28 | 683 | (8.45;0.99),(8.44;1.01),(8.44;1.02),(8.43;0.93),(8.08;1.01),(8.08;0.99),(8.06;1.09),( 8.06;0.99),(7.84;0.53),(7.82;0.53),(7.73;1.95),(7.71;2.47),(7.61;1.02),(7.60;1.89),( 7.58;1.22),(7.55;1.09),(7.54;1.04),(7.53;0.98),(7.52;0.95),(7.41;1.04),(7.40;1.14),( 7.31;1.16),(7.31;1.01),(7.24;1.49),(6.87;1.06),(6.40;0.40),(4.47;3.48),(4.38;2.21),( 4.26;3.02),(3.93;0.47),(3.91;0.45),(3.11;53.00),(2.53;0.45),(2.51;0.56),(2.50;7.77), (2.49;15.18),(2.49;20.82),(2.48;14.79),(2.48;7.36),(2.30;1.05),(2.18;1.83),(2.16;5. 99),(1.36;16.00),(1.19;1.55),(1.17;1.70),(1.05;7.68),(1.04;7.54),(-0.00;0.61) |
| 51 | 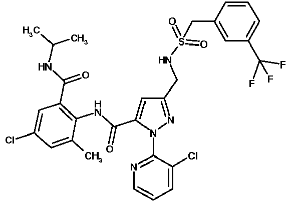 | 3,42 | 683 | (8.44;0.51),(8.44;0.53),(8.43;0.51),(8.43;0.51),(8.08;0.55),(8.07;0.58),(8.06;0.59),( 8.05;0.54),(7.74;0.63),(7.70;0.33),(7.68;0.75),(7.66;0.63),(7.62;0.50),(7.60;0.50),( 7.55;0.62),(7.54;0.56),(7.53;0.56),(7.52;0.52),(7.40;0.56),(7.40;0.61),(7.31;0.61),( 7.30;0.57),(7.23;0.80),(6.87;1.OS),(4.49;2.10),(4.48;0.35),(4.25;0.35),(4.24;1.98),( 3.11;39.96),(2.53;1.56),(2.51;0.77),(2.50;10.02),(2.49;19.40),(2.49;26.51),(2.48;18 .82),(2.48;9.31),(2.30;0.77),(2.18;1.84),(2.15;3.16),(2.06;0.40),(2.04;0.37),(1.39;0. 87),(1.36;16.00),(1.19;0.56),(1.18;1.70),(1.17;0.62),(1.17;1.59),(1.13;0.32),(1.05;4 .10),(1.04;3.97),(-0.00;1.05) |
| 52 | 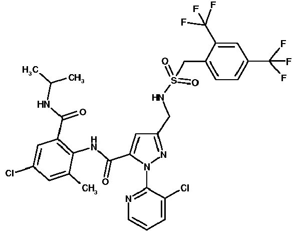 | 3,92 | 751 | (8.44;1.24),(8.44;1.31),(8.43;1.27),(8.43;1.25),(8.11;0.76),(8.08;1.05),(8.08;1.48),( 8.07;1.42),(8.06;1.44),(8.05;1.34),(8.02;1.46),(7.97;1.20),(7.95;0.90),(7.83;0.69),( 7.81;0.69),(7.55;1.40),(7.54;1.39),(7.53;1.28),(7.52;1.22),(7.40;1.33),(7.40;1.45),( 7.31;1.51),(7.30;1.39),(7.25;1.89),(6.87;1.02),(6.40;0.43),(4.65;3.74),(4.59;0.37),( 4.33;4.83),(3.94;0.39),(3.92;0.58),(3.90;0.59),(3.89;0.38),(3.31;0.35),(3.29;0.43),( 3.27;0.35),(3.11;85.64),(2.86;1.12),(2.53;2.14),(2.51;1.10),(2.50;13.76),(2.49;26.6 2),(2.49;36.29),(2.48;25.64),(2.48;12.62),(2.30;1.23),(2.18;1.84),(2.15;7.58),(2.07; 0.42),(1.36;16.00),(1.19;3.75),(1.18;3.69),(1.13;1.08),(1.12;1.09),(1.08;0.33),(1.07 ;0.62),(1.05;9.89),(1.03;9.55),(-0.00;1.13) |
| 53 | 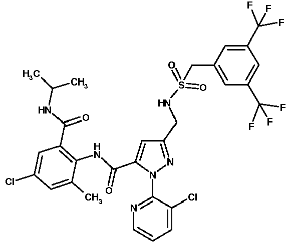 | 3,95 | 751 | (8.43;1.23),(8.43;1.29),(8.42;1.31),(8.42;1.25),(8.08;3.31),(8.08;2.38),(8.07;1.69),( 8.06;1.46),(8.05;1.38),(8.03;1.38),(7.84;0.67),(7.82;0.68),(7.55;1.36),(7.54;1.35),( 7.53;1.26),(7.52;1.22),(7.40;1.30),(7.40;1.33),(7.40;1.48),(7.31;1.51),(7.31;1.34),( 7.23;1.93),(6.87;1.06),(6.40;0.33),(4.65;4.68),(4.31;0.44),(4.29;4.89),(3.95;0.40),( 3.93;0.59),(3.91;0.57),(3.89;0.39),(3.12;19.42),(2.87;0.47),(2.53;0.70),(2.51;0.33), (2.50;4.61),(2.49;8.99),(2.49;12.28),(2.48;8.72),(2.48;4.34),(2.18;1.87),(2.16;7.61) ,(2.07;0.42),(1.37;16.00),(1.19;1.61),(1.17;1.43),(1.13;0.48),(1.12;0.47),(1.06;9.74 ),(1.04;9.58),(-0.00;0.40) |
| 54 | 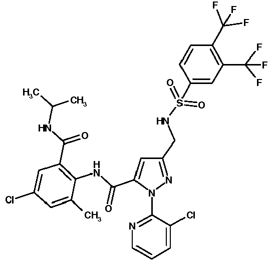 | 4 | 737 | (8.40;2.23),(8.39;2.27),(8.38;2.40),(8.38;2.34),(8.31;2.57),(8.31;3.53),(8.30;1.96),( 8.28;2.73),(8.28;1.80),(8.26;0.35),(8.22;0.38),(8.20;2.98),(8.18;1.99),(8.17;0.38),( 8.03;2.03),(8.03;1.97),(8.01;2.23),(8.01;2.04),(7.86;0.99),(7.84;0.97),(7.53;2.14),( 7.51;2.09),(7.51;1.99),(7.49;1.91),(7.40;2.15),(7.40;2.40),(7.32;2.39),(7.31;2.14),( 7.14;3.14),(6.87;1.04),(6.40;0.41),(4.27;8.30),(4.20;0.40),(3.96;0.64),(3.95;0.98),( 3.93;0.94),(3.91;0.63),(3.29;0.40),(3.27;0.37),(3.12;45.65),(3.04;0.39),(2.87;0.60), (2.53;1.90),(2.51;1.01),(2.50;12.65),(2.49;24.51),(2.49;33.42),(2.48;23.74),(2.48;1 1.77),(2.30;0.56),(2.18;1.85),(2.16;0.33),(2.13;12.21),(2.04;0.65),(1.36;16.00),(1.1 9;3.42),(1.17;3.19),(1.13 ;0.65),(1.12;0.63),(1.09;0.46),(1.07;15.56),(1.05;15.36),(-0.00;0.99)" |
| 55 | 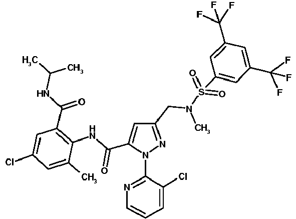 | 4,5 | 751 | (10.027;1.76),(8.412;2.10),(8.408;2.21),(8.401;2.26),(8.397;2.19),(8.350;4.70),(8.3 13;1.94),(8.045;2.11),(8.041;2.16),(8.025;2.37),(8.021;2.26),(7.809;0.83),(7.790;0. 82),(7.542;2.41),(7.530;2.34),(7.522;2.25),(7.510;2.22),(7.403;1.84),(7.402;1.88),( 7.397;2.12),(7.396;1.97),(7.304;2.12),(7.299;1.93),(7.203;2.29),(4.559;7.07),(3.95 5 ;0.59),(3.939;0.88),(3.920;0.86),(3.903;0.59),(3.117;123.19),(2.875;15.99),(2.527 ;0.86),(2.511;0.58),(2.506;0.83),(2.498;11.27),(2.493;23.13),(2.488;32.43),(2.484; 22.67),(2.479;10.86),(2.146;11.38),(1.364;0.75),(1.060;16.00),(1.044;15.82),(-0.000;0.50) |
| 56 | 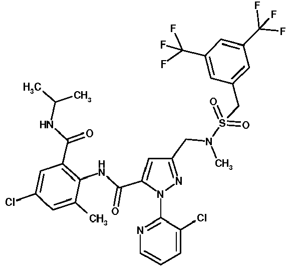 | 4,37 | 765 | (10.060;1.09),(8.446;1.41),(8.442;1.51),(8.434;1.54),(8.430;1.48),(8.140;3.02),(8.0 91;1.49),(8.087;1.53),(8.071;2.81),(8.067;2.14),(7.795;0.57),(7.776;0.58),(7.562;1. 66),(7.550;1.60),(7.542;1.55),(7.530;1.55),(7.405;1.24),(7.403;1.28),(7.398;1.43),( 7.397;1.34),(7.300;1.45),(7.294;1.31),(7.259;1.51),(6.873;0.89),(6.405;0.47),(4.76 2;4.77),(4.422;4.54),(3.934;0.38),(3.917;0.59),(3.898;0.58),(3.882;0.39),(3.118;12 3.30),(2.824;10.81),(2.527;0.87),(2.511;0.56),(2.506;0.81),(2.498;11.01),(2.493;22 .59),(2.488;31.67),(2.484;22.09),(2.479;10.51),(2.183;1.50),(2.158;7.62),(1.364;16 .00),(1.045;10.82),(1.029;10.70) |
| 57 | 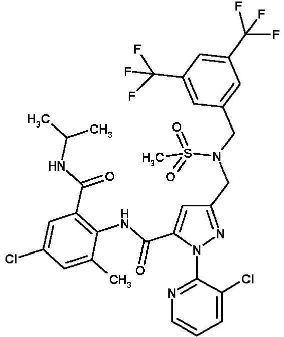 | 4,26 | 765 | (10.025;1.71),(8.415;2.08),(8.412;2.15),(8.404;2.22),(8.400;2.22),(8.058;2.20),(8.0 54;2.04),(8.038;2.33),(8.034;2.28),(7.975;0.77),(7.941;4.38),(7.891;1.86),(7.808;0. 81),(7.790;0.77),(7.540;2.55),(7.528;2.41),(7.520;2.27),(7.508;2.16),(7.406;1.92),( 7.400;2.07),(7.309;2.18),(7.302;1.95),(7.224;2.20),(5.689;0.97),(4.635;5.70),(4.52 1;6.95),(4.047;0.58),(4.029;0.54),(3.952;0.61),(3.935;0.88),(3.917;0.95),(3.900;0.6 0),(3.609;0.37),(3.602;0.47),(3.352;0.36),(3.206;0.34),(3.189;0.47),(3.174;0.68),(3 .112;1092.94),(3.093;24.71),(3.043;0.36),(2.666;0.64),(2.662;1.51),(2.657;2.00),(2 .652;1.40),(2.647;0.67),(2.558;0.43),(2.526;1.93),(2.510;4.94),(2.505;7.03),(2.497; 106.03),(2.493;219.75),(2.488;310.47),(2.483;217.21),(2.478;104.28),(2.324;0.68) ,(2.319;1.34),(2.315;1.88),(2.310;1.26),(2.305;0.68),(2.140;11.24),(2.040;1.67),(1. 974;2.71),(1.901;0.40),(1.771;0.34),(1.762;0.72),(1.419;0.39),(1.404;1.27),(1.363; 1.67),(1.245;0.53),(1.195;0.75),(1.177;1.62),(1.159;0.72),(1.059;16.00),(1.042;15. 78),(0.895;0.49),(-0.000;8.56) |
| 58 | 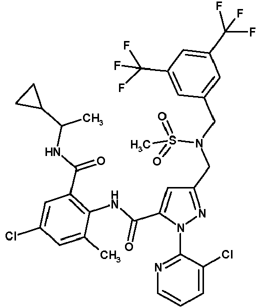 | 4,54 | 791 | (10.032;2.34),(8.417;3.05),(8.413;3.24),(8.405;3.35),(8.401;3.22),(8.059;3.10),(8.0 55;3.13),(8.038;3.52),(8.035;3.24),(7.943;6.51),(7.894;3.71),(7.876;1.34),(7.541;3. 51),(7.529;3.36),(7.521;3.26),(7.509;3.19),(7.415;2.66),(7.414;2.73),(7.409;3.06),( 7.314;3.11),(7.309;2.79),(7.226;3.31),(4.632;8.35),(4.518;10.12),(3.392;0.64),(3.3 72;1.26),(3.355;1.29),(3.335;0.67),(3.115;162.24),(3.092;26.49),(2.510;0.95),(2.50 6;1.40),(2.497;19.29),(2.493;39.54),(2.488;55.40),(2.483;38.66),(2.479;18.44),(2.1 42;16.00),(1.404;1.95),(1.092;10.11),(1.076;9.97),(0.885;0.45),(0.877;0.67),(0.865 ;1.30),(0.857;0.83),(0.853;0.88),(0.844;1.35),(0.832;0.77),(0.824;0.55),(0.395;0.49 ),(0.391;0.54),(0.382;1.04),(0.3733:1.01),(0.369;1.00),(0.360;1.15),(0.352;0.66),(0. 348;0.73),(0.338;0.64),(0.299;0.57),(0.288;0.81),(0.285;0.78),(0.278;0.74),(0.275; 1.01),(0.267;1.03),(0.254;1.12),(0.247;0.56),(0.243;0.55),(0.238;0.59),(0.232;0.72) ,(0.225;0.82),(0.215;1.16),(0.203 ;1.44),(0.192;1.13),(0.179;0.54),(0.162;0.72),(0.1 50;1.16),(0.140;1.35),(0.127;1.07),(0.117;0.60),(-0.000;1.51) |
| 59 | 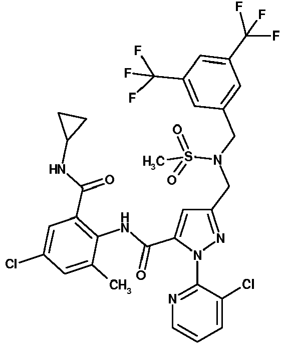 | 4,05 | 763 | (12.543;1.97),(11.209;0.83),(10.018;3.12),(8.425;2.59),(8.414;2.60),(8.084;0.89),( 8.082;0.94),(8.062;3.85),(8.060;3.46),(8.043;3.38),(7.949;7.54),(7.894;3.31),(7.82 9;1.72),(7.542;2.03),(7.532;2.06),(7.522;1.85),(7.512;1.94),(7.400;2.91),(7.293;3.5 2),(7.227;3.58),(4.946;0.83),(4.643;8.95),(4.527;9.55),(3.651;0.91),(3.449;0.87),(3 .410;0.84),(3.389;1.09),(3.364;1.00),(3.35 8;0.99),(3.284;1.22),(3.262;1.15),(3.239; 1.63),(3.214;1.98),(3.116;1733.15),(3.113;1640.53),(3.091;31.11),(3.053;1.27),(3. 050;1.14),(2.711;1.44),(2.701;1.66),(2.657;3.08),(2.560;1.17),(2.524;3.80),(2.497; 167.94),(2.493;345.71),(2.488;495.50),(2.486;456.28),(2.484;388.70),(2.481;312.4 0),(2.417;1.10),(2.407;0.97),(2.395;0.92),(2.313;2.88),(2.134;16.00),(2.040;1.23),( 1.404;12.37),(1.402;11.71),(1.244;1.46),(1.045;0.81),(0.943;0.98),(0.632;0.91),(0. 614;2.89),(0.601;3.06),(0.475;1.26),(0.457;3.46),(0.451;3.24),(-0.000;15.31),(-0.002;14.21) |
| 60 | 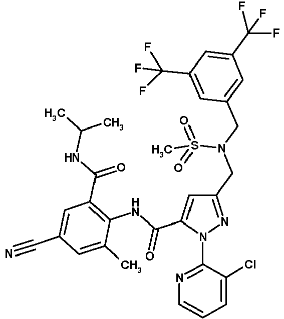 | 3,91 | 757 | (10.330;1.09),(8.418;1.84),(8.414;2.00),(8.406;2.04),(8.402;1.96),(8.061;1.81),(8.0 58;1.90),(8.041;2.07),(8.037;1.99),(7.987;0.54),(7.981;0.59),(7.974;0.51),(7.958;0. 73),(7.941;4.10),(7.888;1.68),(7.791;1.66),(7.724;1.86),(7.720;1.73),(7.543;2.05),( 7.531;1.96),(7.523;1.92),(7.511;1.90),(7.243;3.01),(6.873;0.87),(6.401;0.45),(4.64 0;5.20),(4.531;6.22),(4.368;0.34),(3.961;0.51),(3.945;0.81),(3.926;0.78),(3.910;0.5 6),(3.113;307.41),(3.099;20.73),(3.016;0.92),(2.662;0.42),(2.657;0.67),(2.652;0.44 ),(2.526;0.61),(2.510;1.68),(2.506;2.33),(2.497;33.91),(2.493;70.02),(2.488;98.68), (2.483;69.12),(2.478;33.18),(2.319;0.40),(2.315;0.62),(2.310;0.41),(2.286;0.74),(2. 189;9.57),(2.137;0.81),(1.510;0.47),(1.404;0.55),(1.364;16.00),(1.228;0.65),(1.211 ;0.64),(1.092;1.15),(1.073;14.69),(1.057;14.36),(-0.000;3.83) |
| 61 | 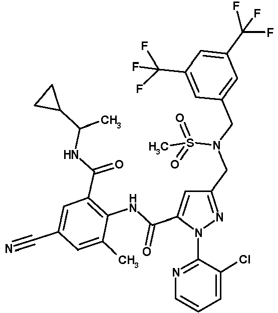 | 4,12 | 783 | (8.419;3.13),(8.416;3.39),(8.408;3.46),(8.404;3.32),(8.062;3.68),(8.058;3.75),(8.04 2;3.68),(8.038;3.46),(7.942;6.69),(7.890;2.76),(7.801;2.73),(7.799;2.97),(7.727;3.2 6),(7.723;3.00),(7.544;3.49),(7.532;3.34),(7.524;3.25),(7.512;3.28),(7.239;5.92),(5 .688;0.54),(4.638;8.59),(4.528;10.47),(3.401;0.67),(3.381;1.31),(3.364;1.35),(3.34 4;0.67),(3.119;211.31),(3.098;29.20),(3.010;1.23),(2.511;1.05),(2.506;1.47),(2.498 ;22.54),(2.494;46.59),(2.489;65.64),(2.484;45.93),(2.480;22.04),(2.287;1.18),(2.19 0;16.00),(2.136;1.15),(1.404;5.15),(1.364;2.27),(1.132;0.83),(1.115;1.12),(1.106;1 0.41),(1.089;10.33),(0.899;0.53),(0.891;0.71),(0.878;1.38),(0.871;0.84),(0.866;0.9 2),(0.858;1.51),(0.846;0.81),(0.838;0.57),(0.403;0.61),(0.395;1.10),(0.386;1.06),(0 .382;1.11),(0.373;1.25),(0.365;0.72),(0.361;0.83),(0.351;0.74),(0.317;0.58),(0.306; 0.83),(0.303;0.82),(0.292;1.04),(0.286;1.09),(0.272;1.17),(0.264;0.58),(0.251;0.85) ,(0.240;0.89),(0.230;1.22),(0.217;1.50),(0.206;1.24),(0.193;0.56),(0.177;0.78),(0.1 64;1.25),(0.154;1.43),(0.141;1.16),(0.132;0.67),(0.000;1.48) |
| 62 | 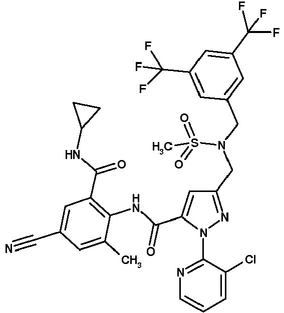 | 3,64 | 755 | (10.329;1.95),(8.431;3.12),(8.427;3.40),(8.419;3.43),(8.415;3.28),(8.283;0.47),(8.2 78;0.53),(8.260;0.50),(8.217;0.76),(8.214;1.03),(8.208;1.17),(8.197;1.54),(8.193;1. 18),(8.071;2.94),(8.067;3.08),(8.051;3.38),(8.047;3.17),(7.995;0.88),(7.986;0.47),( 7.949;6.60),(7.890;2.98),(7.789;2.78),(7.704;2.89),(7.701;2.71),(7.548;3.37),(7.53 7;3.16),(7.528;3.13),(7.516;3.05),(7.507;0.47),(7.269;1.20),(7.251;5.42),(4.672;1.1 1),(4.648;8.69),(4.570;1.43),(4.539;9.71),(3.115;585.74),(3.109;56.53),(3.039;1.04 ),(2.743;0.60),(2.734;0.89),(2.725 ;1.26),(2.716;1.25),(2.707;0.94),(2.701;0.89),(2. 697;0.72),(2.690;0.64),(2.662;0.74),(2.657;1.07),(2.653;0.71),(2.527;1.10),(2.510; 2.86),(2.506;4.13),(2.498;56.93),(2.493;116.98),(2.488;164.48),(2.484;114.57),(2. 479;54.47),(2.320;0.67),(2.315;0.98),(2.310;0.68),(2.283;1.31),(2.184;16.00),(2.14 6;1.22),(2.040;0.96),(1.974;0.49),(1.894;1.96),(1.404;2.56),(1.363;0.67),(0.650;0.8 3),(0.637;2.22),(0.631;2.95),(0.619;2.90),(0.613;2.35),(0.602;1.08),(0.496;1.30),(0 .486;3.12),(0.479;2.87),(0.470;2.41),(0.458;0.78),(-0.000;5.29) |
| 63 | 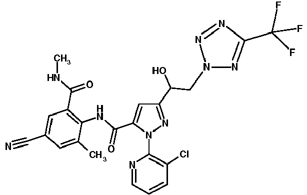 | 2,01 / 2,27 | 575 | |
| 64 | 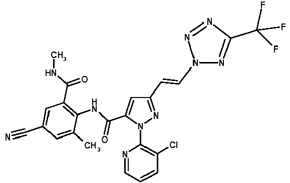 | 3,17 / 3,49 | 557 | (8.496;0.37),(8.494;0.38),(8.488;0.37),(8.486;0.37),(8.410;1.59),(8.386;2.20),(8.38 3;0.97),(8.378;0.97),(8.375;0.96),(8.369;0.83),(8.368;0.87),(8.366;0.89),(8.365;0.8 6),(8.355;0.59),(8.331;0.66),(8.011;0.96),(8.008;1.21),(8.005;0.42),(7.997;1.00),(7 .995;1.22),(7.992;0.43),(7.819;1.32),(7.795;1.20),(7.782;0.53),(7.775;0.34),(7.774; 0.35),(7.772;0.38),(7.771;0.38),(7.758;0.49),(7.750;0.79),(7.748;0.99),(7.747;0.97) ,(7.745;0.81),(7.663;0.30),(7.662;0.33),(7.660;0.31),(7.527;0.40),(7.519;0.39),(7.5 13;0.39),(7.505;0.37),(7.468;1.07),(7.460;1.30),(7.455;0.99),(7.447;1.06),(7.430;2. 62),(6.974;0.67),(6.973;0.67),(4.064;0.67),(4.053;0.67),(3.757;9.31),(3.653;0.31),( 3.642;0.78),(3.631;0.35),(3.483;0.30),(2.823;0.64),(2.817;0.64),(2.646;0.46),(2.63 8;0.46),(2.222;0.90),(2.221;1.42),(2.220;0.86),(2.198;2.12),(2.177;0.30),(1.974;5.2 9),(1.973;6.30),(1.951;3.45),(1.947;6.63),(1.943;10.02),(1.939;6.85),(1.935;3.31),( 1.812;0.33),(1.801;0.97),(1.795;0.29),(1.790;0.33),(1.386;13.50),(1.216;0.93),(1.2 04;1.74),(1.199;0.46),(1.192;0.93),(-0.000;7.90) |
| 65 | 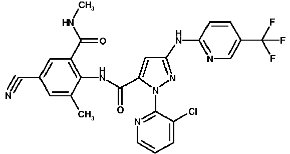 | 3,71 | 597 | (10.549;3.82),(10.396;4.84),(8.540;2.80),(8.483;2.94),(8.479;3.07),(8.471;3.12),(8. 468;2.95),(8.357;0.70),(8.348;1.72),(8.337;1.67),(8.326;0.62),(8.144;2.95),(8.140; 2.79),(8.123;3.22),(8.120;2.91),(7.975;1.73),(7.969;1.65),(7.953;1.88),(7.947;1.74) ,(7.866;3.54),(7.863;3.70),(7.761;4.03),(7.756;3.58),(7.569;2.95),(7.557;2.90),(7.5 49;2.79),(7.537;2.77),(7.494;3.97),(7.371;2.43),(7.349;2.29),(4.057;0.69),(4.039;1. 82),(4.021;1.86),(4.003;0.72),(3.652;0.39),(3.618;0.41),(3.559;0.50),(3.552;0.52),( 3.546;0.53),(3.434;1.29),(3.312;2504.39),(2.700;10.34),(2.689;10.41),(2.679;1.79) ,(2.674;2.62),(2.670;3.33),(2.665;2.44),(2.660;1.31),(2.628;0.47),(2.619;0.54),(2.5 39;4.05),(2.523;15.97),(2.509;188.46),(2.505;339.59),(2.500;433.23),(2.496;296.1 1),(2.492;138.38),(2.332;2.10),(2.327;2.83),(2.323;1.99),(2.318;0.89),(2.231;16.00 ),(2.085;2.72),(2.069;2.88),(1.987;7.32),(1.908;0.49),(1.237;0.69),(1.193;2.05),(1. 175;4.04),(1.157;2.03),(0.890;1.13),(-0.000;6.89) |
| 66 | 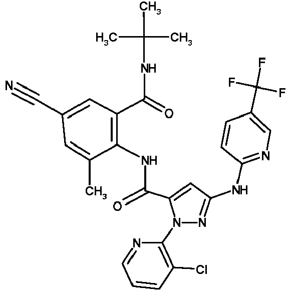 | 2,84 | 555 | (10.417;1.62),(8.507;0.67),(8.474;0.62),(8.470;0.69),(8.462;0.67),(8.458;0.63),(8.1 45;0.60),(8.141;0.61),(8.125;0.69),(8.121;0.62),(7.972;0.37),(7.967;0.39),(7.950;0. 39),(7.944;0.43),(7.831;0.81),(7.828;0.81),(7.758;0.94),(7.706;0.87),(7.702;0.82),( 7.569;0.63),(7.557;0.64),(7.549;0.62),(7.537;0.94),(7.533;1.03),(7.353;0.55),(7.33 1;0.53),(4.039;0.38),(4.021;0.41),(3.381;0.44),(3.305;691.37),(3.282;14.43),(2.678 ;0.42),(2.674;0.78),(2.669;1.02),(2.664;0.74),(2.539;1.56),(2.522;4.28),(2.509;57.3 7),(2.505;107.43),(2.500;140.94),(2.496;98.10),(2.491;46.94),(2.332;0.68),(2.327; 0.93),(2.322;0.67),(2.229;3.47),(2.085;0.55),(2.069;1.91),(1.987;1.75),(1.285;;16.0 0),(1.193;0.49),(1.175;0.95),(1.157;0.52),(1.046;0.34),(1.030;0.33),(0.008;0.54),(-0.000;12.25),(-0.009;0.46) |
| 67 | 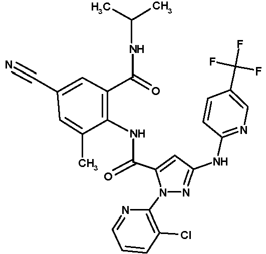 | 3,31 | 493 | (10.486;2.72),(10.400;3.40),(8.527;1.91),(8.524;1.91),(8.522;1.84),(8.470;2.23),(8. 467;2.32),(8.459;2.40),(8.455 ;2.25),(8.175;1.44),(8.156;1.48),(8.132;2.24),(8.128; 2.22),(8.112;2.49),(8.108;2.26),(7.994;0.84),(7.989;0.84),(7.974;1.21),(7.967;1.19) ,(7.951;1.26),(7.945;1.25),(7.859;2.32),(7.857;2.46),(7.854;2.56),(7.784;0.53),(7.7 79;0.50),(7.763;0.79),(7.758;0.75),(7.728;2.75),(7.725;2.54),(7.675;1.57),(7.654;1. 07),(7.565;2.33),(7.553;2.27),(7.544;2.26),(7.533;2.97),(7.527;3.14),(7.360;1.68),( 7.338;1.60),(4.039;1.09),(4.021;1.10),(4.003;0.39),(3.957;0.63),(3.940;0.95),(3.92 2;0.92),(3.905;0.62),(3.305;407.19),(2.678;0.44),(2.674;0.86),(2.669;1.10),(2.664; 0.80),(2.660;0.43),(2.539;1.33),(2.522;4.99),(2.509;62.36),(2.505;115.15),(2.500;1 49.52),(2.496;102.45),(2.491;48.17),(2.332;0.78),(2.327;1.02),(2.322;0.73),(2.237; 11.04),(2.069;1.78),(1.987;4.83),(1.245;3.18),(1.193;1.42),(1.175;2.84),(1.157;1.3 9),(1.080;16.00),(1.063;15.77),(-0.000;5.36) |
| 65 | 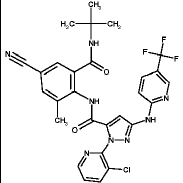 | 3.71 | 597,2 | (10.42;1.62), (8.51;0.67), (8.47;0.62), (8.47;0.69), (8.46;0.67), (8.46;0.63), (8.15;0.60), (8.14;0.61), (8.13;0.69), (8.12;0.62), (7.97;0.37), (7.97;0.39), (7.95;0.39), (7.94;0.43), (7.83;0.81), (7.83;0.81), (7.76;0.94), (7.71;0.87), (7.70;0.82), (7.57;0.63), (7.56;0.64), (7.55;0.62), (7.54;0.94), (7.53;1.03), (7.35;0.55), (7.33;0.53), (4.04;0.38), (4.02;0.41), (3.38;0.44), (3.31;691.37), (3.28;14.43), (2.68;0.42), (2.67;0.78), (2.67;1.02), (2.66;0.74), (2.54;1.56), (2.52;4.28), (2.51;57.37), (2.50;107.43), (2.50;140.94), (2.50;98.10), (2.49;46.94), (2.33;0.68), (2.33;0.93), (2.32;0.67), (2.23;3.47), (2.08;0.55), (2.07;1.91), (1.99;1.75), (1.29;16.00), (1.19;0.49), (1.17;0.95), (1.16;0.52), (1.05;0.34), (1.03;0.33), (0.01;0.54), (0.00;12.25), (-0.01;0.46) |
| 66 | 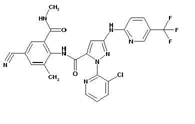 | 2.84 | 555,1 | (10.55;3.82), (10.40;4.84), (8.54;2.80), (8.48;2.94), (8.48;3.07), (8.47;3.12), (8.47;2.95), (8.36;0.70), (8.35;1.72), (8.34;1.67), (8.33;0.62), (8.14;2.95), (8.14;2.79), (8.12;3.22), (8.12;2.91), (7.98;1.73), (7.97;1.65), (7.95;1.88), (7.95;1.74), (7.87;3.54), (7.86;3.70), (7.76;4.03), (7.76;3.58), (7.57;2.95), (7.56;2.90), (7.55;2.79), (7.54;2.77), (7.49;3.97), (7.37;2.43), (7.35;2.29), (4.06;0.69), (4.04;1.82), (4.02;1.86), (4.00;0.72), (3.67;0.35), (3.65;0.39), (3.62;0.41), (3.56;0.50), (3.55;0.52), (3.55;0.53), (3.43;1.29), (3.31;2504.39), (2.70;10.34), (2.69;10.41), (2.68;1.79), (2.67;2.62), (2.67;3.33), (2.67;2.44), (2.66;1.31), (2.63;0.47), (2.62;0.54), (2.54;4.05), (2.52;15.97), (2.51;188.46), (2.51;339.59), (2.50;433.23), (2.50;296.11), (2.49;138.38), (2.33;2.10), (2.33;2.83), (2.32;1.99), (2.32;0.89), (2.23;16.00), (2.08;2.72), (2.07;2.88), (1.99;7.32), (1.91;0.49), (1.24;0.69), (1.19;2.05), (1.17;4.04), (1.16;2.03), (0.89;1.13), (0.00;6.89) |
| 67 | 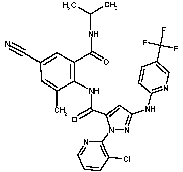 | 3.31 | 583,1 | (10.49;2.72), (10.40;3.40), (8.53;1.91), (8.52;1.91), (8.52;1.84), (8.47;2.23), (8.47;2.32), (8.46;2.40), (8.45;2.25), (8.18;1.44), (8.16;1.48), (8.13;2.24), (8.13;2.22), (8.11;2.49), (8.11;2.26), (7.99;0.84), (7.99;0.84), (7.97;1.21), (7.97;1.19), (7.95;1.26), (7.95;1.25), (7.86;2.32), (7.86;2.46), (7.85;2.56), (7.78;0.53), (7.78;0.50), (7.76;0.79), (7.76;0.75), (7.73;2.75), (7.72;2.54), (7.68;1.57), (7.65;1.07), (7.56;2.33), (7.55;2.27), (7.54;2.26), (7.53;2.97), (7.53;3.14), (7.36;1.68), (7.34;1.60), (4.06;0.37), (4.04;1.09), (4.02;1.10), (4.00;0.39), (3.96;0.63), (3.94;0.95), (3.92;0.92), (3.91;0.62), (3.30;407.19), (2.68;0.44), (2.67;0.86), (2.67;1.10), (2.66;0.80), (2.66;0.43), (2.54;1.33), (2.52;4.99), (2.51;62.36), (2.50;115.15). (2.50;149.52), (2.50;102.45), (2.49;48.17), (2.34;0.37), (2.33;0.78), (2.33;1.02), (2.32;0.73), (2.24;11.04), (2.07;1.78), (1.99;4.83), (1.25;3.18), (1.19;1.42), (1.17;2.84), (1.16;1.39), (1.08;16.00), (1.06;15.77), (0.00;5.36) |
| 68 | 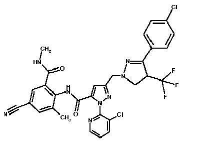 | 3.66 | 655,1 | (10.16;2.23), (8.47;3.24), (8.46;3.13), (8.45;3.24), (8.45;3.02), (7.99;3.07), (7.99;2.92), (7.97;3.37), (7.97;3.23), (7.74;3.92), (7.73;4.86), (7.72;5.87), (7.70;7.23), (7.70;4.98), (7.50;3.48), (7.49;3.40), (7.48;3.17), (7.47;3.19), (7.43;8.19), (7.43;2.47), (7.41;2.41), (7.41;6.43), (7.08;0.39), (7.06;8.17), (7.04;0.99), (7.03;1.15), (7.02;1.12), (5.52;0.51), (5.45;1.90), (4.55;2.61), (4.51;5.94). (4.46;5.86), (4.44;0.92), (4.44;0.98), (4.42;3.22), (4.42;1.43), (4.41;1.04), (4.39;0.98), (4.39;0.82), (4.37;0.32), (4.07;0.53), (4.05;0.47), (3.89;2.06), (3.88;2.02), (3.86;2.29), (3.85;2.24), (3.48;1.94), (3.47;0.47), (3.45;3.29), (3.43;1.53), (3.34;0.35), (3.32;0.49), (3.31;0.46), (2.94;0.33), (2.83;0.35), (2.82;0.38), (2.78;0.96), (2.77;16.00), (2.76;15.62), (2.73;1.46), (2.68;0.34), (2.62;0.39), (2.60;0.36), (2.58;0.40), (2.58;0.37), (2.55;0.42), (2.53;0.42), (2.52;0.42), (2.51;0.38), (2.50;0.44), (2.47;0.67), (2.46;0.77), (2.46;0.75), (2.45;0.50), (2.43;0.54), (2.42;0.48), (2.40;0.50), (2.40;0.56), (2.38;0.57), (2.38;0.55), (2.36;0.66), (2.30;0.94), (2.28;0.96), (2.20;24.46), (2.14;2463.28), (2.12;5.91), (2.11;5.80), (2.11;6.05), (2.10;4.22), (2.09;2.45), (2.07;0.97), (2.07;0.96), (2.05;0.97), (2.04;1.07), (2.01;1.76), (1.96;481.38), (1.96;44.49), (1.95;265.53), (1.95;491.65); (1.94;697.21), (1.93;475.63), (1.93;239.61), (1.79;2.48), (1.78;1.34), (1.77;2.58), (1.77;3.85), (1.76;2.51), (1.76;1.25), (1.39;1.02), (1.27;1.58), (1.22;0.53), (1.20;1.01), (1.19;0.52), (1.13;0.59), (1.11;1.06), (1.10;0.48), (0.91;2.89), (0.88;0.35), (0.15;0.33), (0.01;3.88), (0.00;79.00), (-0.01;2.79), (-0.15;0.37) |
| 69 | 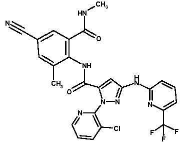 | 2.89 | 555,1 | (10.62;0.92), (10.29;1.27), (8.48;0.72), (8.48;0.78), (8.47;0.80), (8.47;0.73), (8.37;0.42), (8.36;0.45), (8.15;0.72), (8.15;0.69), (8.13;0.78), (8.13;0.71), (7.90;0.36), (7.88;0.61), (7.86;1.31), (7.78;0.96), (7.77;0.89), (7.63;0.62), (7.61;0.56), (7.57;0.74), (7.56;0.72), (7.55;0.67), (7.54;0.67), (7.27;1.45), (7.25;0.84), (4.04;0.43), (4.02;0.42), (3.43;0.36), (3.41;0.41), (3.30;559.69), (3.28;4.39), (2.71;2.60), (2.70;2.60), (2.68;0.47), (2.67;0.74), (2.67;0.94), (2.66;0.74), (2.54;2.17), (2.52;4.91), (2.51;52.99), (2.50;95.66), (2.50;122.72), (2.50;84.65), (2.49;40.48), (2.33;0.58), (2.33;0.75), (2.32;0.56), (2.23;3.86), (2.07;16.00), (1.99;1.63), (1.19;0.47), (1.17;0.95), (1.16;0.48), (0.01;0.76), (0.00;14.81), (-0.01;0.59) |
| 70 | 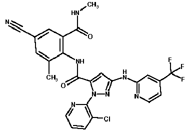 | 2.76 | 555,1 | (10.55;4.54), (10.25;5.08), (8.47;6.02), (8.47;4.83), (8.46;7.29), (8.46;4.74), (8.34;1.93), (8.33;1.90), (8.15;2.61), (8.13;2.77), (8.13;2.69), (7.86;3.98), (7.76;4.37), (7.56;3.10), (7.55;7.06), (7.54;2.96), (7.53;2.47), (7.47;4.39), (7.32;0.33), (7.11;2.79), (7.10;2.71), (4.06;0.37), (4.04;1.03), (4.02;1.16), (4.00;0.43), (3.71;0.41), (3.58;0.38), (3.56;0.41), (3.51;0.52), (3.50;0.57), (3.48;0.87), (3.45;1.05), (3.32;3880.78), (3.23;3.74), (3.18;1.90), (3.15;1.41), (3.14;1.34), (3.11;1.10), (3.10;1.08), (3.09;1.03), (3.07;0.88), (3.05;0.84), (3.01;0.71), (3.00;0.72), (2.99;0.68), (2.96;0.64), (2.92;0.55), (2.90;0.54), (2.87;0.55), (2.84;0.53), (2.83;0.49), (2.80;0.51), (2.75;0.56), (2.71;10.73), (2.69;11.29), (2.68;2.26), (2.67;3.42), (2.67;4.17), (2.67;3.15), (2.64;0.58), (2.58;1.25), (2.54;8.31), (2.52;18.72), (2.51;228.93), (2.51;420.15), (2.50;544.13), (2.50;380.45), (2.49;185.40), (2.41;1.26), (2.40;1.07), (2.33;3.19), (2.33;4.05), (2.32;3.01), (2.31;0.74), (2.30;0.68), (2.28;0.71), (2.24;16.00), (2.20;0.71), (2.18;0.68), (2.16;0.60), (2.12;0.44), (2.07;2.43), (2.05;0.55), (2.00;0.78), (1.99;5.11), (1.96;0.41), (1.91;0.55), (1.88;0.40), (1.87;0.34), (1.82;0.33), (1.65;0.37), (1.49;0.38), (1.43;0.35), (1.33;0.33), (1.29;0.37), (1.24;1.78), (1.19;1.47), (1.18;2.81), (1.16;1.51), (1.02;0.32), (0.89;0.38), (0.87;0.34), (0.85;0.34), (0.00;12.92) |
| 71 | 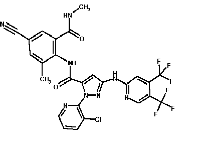 | 3.5 | 623,0 | (18.86;1.01), (17.46;0.96), (17.01;1.02), (15.71;0.95), (14.61;1.05), (13.00;1.03), (11.91;1.00), (10.93;3.03), (10.58;2.80), (8.76;3.72), (8.49;2.38), (8.48;2.07), (8.47;1.92), (8.35;1.36), (8.34;1.59), (8.18;0.92), (8.16;2.12), (8.14;1.96), (7.87;2.80), (7.76;3.12), (7.75;0.99), (7.71;1.11), (7.58;2.38), (7.57;2.78), (7.56;2.69), (7.55;2.06), (7.43;0.93), (5.86;0.94), (4.97;1.08), (4.52;0.99), (4.48;0.96), (4.43;1.07), (4.42;1.00), (4.41;0.99), (4.32;0.99), (4.20;1.11), (4.18;1.12), (4.13;1.10), (4.12;1.09), (4.09;1.09), (4.04;1.05), (4.03;1.02), (4.00;0.96), (3.94;1.09), (3.92;1.57), (3.90;1.31), (3.88;1.43), (3.86;1.49), (3.84;1.51), (3.81;1.61), (3.77;1.56), (3.76;1.68), (3.74;1.65), (3.70;1.63), (3.68;1.67), (3.65;1.89), (3.64;1.99), (3.63;2.16), (3.62;2.09), (3.57;2.37), (3.56;2.47), (3.54;2.72), (3.49;3.73), (3.46;4.33), (3.45;4.70), (3.44;4.56), (3.30;9797.66), (3.28;116.36), (3.18;1.04), (3.05;1.03), (3.01;0.98), (2.97;1.23), (2.89;1.24), (2.84;1.03), (2.78;1.29), (2.77;1.22), (2.76;1.35), (2.75;1.58), (2.74;1.79), (2.70;8.90), (2.70;8.25), (2.69;8.62), (2.68;7.04), (2.67;11.13), (2.67;14.20), (2.66;10.83), (2.54;33.75), (2.51;808.06), (2.50;1441.06), (2.50;1834.67), (2.50;1264.22), (2.49;600.71), (2.33;9.02), (2.33;11.60), (2.32;8.09), (2.23;10.45), (2.08;1.04), (2.07;16.00), (1.24;1.87), (0.89;1.00), (0.00;41.23) |
| 72 | 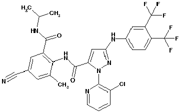 | 3.91 | 650,2 | |
| 73 | 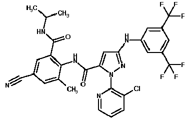 | 4.08 | 650,2 | |
| 74 | 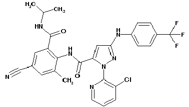 | 3.7 | 582,1 | (10.46;2.01), (9.55;2.34), (8.47;1.84), (8.47;1.94), (8.46;1.98), (8.46;1.92), (8.25;1.18), (8.24;1.21), (8.14;1.45), (8.13;1.47), (8.12;1.57), (8.12;1.50), (7.88;1.82), (7.88;1.89), (7.74;1.97), (7.74;1.90), (7.58;16.00), (7.56;1.62), (7.55;1.50), (7.55;1.47), (7.54;1.45), (6.90;2.40), (4.03;0.34), (4.02;0.35), (3.95;0.54), (3.94;0.80), (3.93;0.78), (3.91;0.53), (3.38;0.38), (3.35;482.12), (3.33;1.69), (2.62;0.33), (2.62;0.47), (2.61;0.34), (2.52;0.73), (2.52;0.95), (2.52;1.02), (2.51;24.51), (2.51;53.11), (2.50;73.79), (2.50;52.16), (2.50;23.35), (2.39;0.32), (2.39;0.44), (2.24;8.36), (2.08;0.65), (1.99;1.61), (1.26;0.44), (1.23;1.08), (1.19;0.46), (1.17;0.94), (1.16;0.50), (1.07;12.75), (1.06;12.70), (0.01;0.52), (0.00;18.11), (-0.01;0.48) |
| 75 | 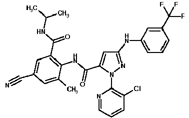 | 3.66 | 582,1 | |
| 76 | 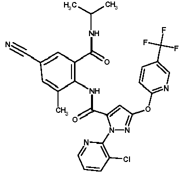 | 3.4 | 584,1 | (8.72;1.96), (8.67;0.37), (8.47;2.09), (8.47;2.32), (8.46;2.26), (8.46;2.35), (8.40;0.37), (8.40;0.37), (8.37;1.38), (8.36;1.37), (8.35;1.40), (8.35;1.40), (8.13;1.32), (8.12;1.38), (8.05;0.39), (7.81;0.35), (7.76;0.70), (7.58;1.20), (7.57;1.25), (7.57;1.23), (7.56;1.15), (7.44;1.34), (7.43;1.33), (6.87;0.46), (4.03;0.53), (4.02;0.54), (3.93;0.69), (3.92;1.04), (3.91;1.09), (3.90;0.72), (3.80;0.89), (3.45;0.63), (3.44;0.72), (3.44;0.74), (3.43;0.81), (3.36;296.43), (3.36;339.87), (3.33;3.15), (3.29;0.34), (3.28;0.38), (2.62;0.64), (2.62;0.88), (2.61;0.62), (2.54;0.54), (2.52;1.70), (2.52;2.28), (2.52;2.82), (2.51;45.87), (2.51;96.06), (2.50;130.59), (2.50;92.75), (2.50;41.57), (2.39;0.58), (2.39;0.80), (2.38;0.57), (2.37;0.50), (2.21;3.38), (2.18;0.90), (2.08;8.16), (2.06;0.36), (1.99;2.30), (1.76;0.34), (1.65;0.45), (1.64;0.44), (1.46;0.37), (1.35;5.10), (1.26;0.56), (1.24;1.86), (1.19;0.74), (1.17;1.36), (1.16;4.29), (1.15;3.69), (1.07;1.74), (1.06;16.00), (1.06;4.61), (1.05;15.74), (0.01;1.30), (0.00;34.71), (-0.01;0.91) |
| 77 | 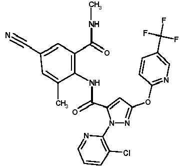 | 2.89 | 555,9 | (10.51;4.01), (8.74;3.43), (8.74;3.46), (8.74;3.52), (8.73;3.58), (8.72;0.55), (8.49;4.25), (8.49;4.61), (8.48;4.68), (8.48;4.54), (8.42;0.57), (8.42;0.63), (8.41;0.67), (8.41;0.68), (8.37;4.57), (8.37;4.19), (8.35;3.44), (8.35;2.82), (8.22;0.54), (8.22;0.56), (8.20;0.60), (8.20;0.56), (8.17;3.12), (8.16;3.15), (8.15;3.48), (8.14;3.33), (8.03;0.32), (7.97;0.54), (7.97;0.63), (7.97;0.63), (7.97;0.53), (7.88;3.32), (7.76;3.87), (7.75;3.80), (7.60;3.22), (7.59;3.19), (7.58;3.12), (7.57;3.32), (7.56;0.67), (7.55;0.57), (7.54;0.55), (7.50;0.53), (7.48;0.76), (7.46;3.51), (7.44;3.37), (7.28;2.22), (7.19;3.52), (6.87;0.79), (6.82;0.50), (6.64;0.45), (4.06;0.70), (4.04;2.10), (4.02;2.12), (4.00;0.73), (3.80;1.77), (3.75;4.28), (3.60;0.40), (3.48;0.32), (3.45;0.49), (3.44;0.49), (3.43;0.52), (3.40;0.94), (3.39;1.16), (3.33;1664.42), (2.68;12.71), (2.67;13.48), (2.54;1.97), (2.52;6.88), (2.51;109.35), (2.51;217.70), (2.50;288.68), (2.50;206.24), (2.49;98.56), (2.37;0.92), (2.34;0.65), (2.33;1.36), (2.33;1.85), (2.32;1.31), (2.32;0.62), (2.22;16.00), (2.18;1.33), (2.07;7.26), (1.99;9.43), (1.94;2.79), (1.91;0.68), (1.76;0.36), (1.40;0.71), (1.36;10.48), (1.30;0.63), (1.26;1.05), (1.24;3.32), (1.19;2.75), (1.17;5.45), (1.16;2.66), (0.85;0.48), (0.01;2.21), (0.00;54.87), (-0.01;1.98) |

### Analytische Methoden

Die Bestimmung der in der voranstehenden Tabelle und den Herstellungsbeispielen angegebenen logP Werten erfolgte gemäß EEC Directive 79/831 Annex V.A8 durch HPLC (High Performance Liquid Chromatography) an reversed-phase Säulen (C 18), mit nachfolgenden Methoden:
Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.

Die Kalibrierung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die lambda-maX Werte wurden an Hand der UV-Spektren von 200 nm bis 400 nm in den Maxima der chromatographischen Signale ermittelt.

Die MH⁺-Signale wurden mit einem Agilent MSD-System mit ESI und positiver oder negativer Ionisation bestimmt.

Die NMR-Spektren wurden mit einem Bruker Avance 400, ausgestattet mit einem Durchflussprobenkopf (60 µl Volumen), bestimmt. Als Lösungsmittel wurde d₆-DMSO verwendet, wobei als Referenz Tetramethylsilan (0.00 ppm) eingesetzt wurde. Die Beispiele in obiger Tabelle wurden in d₆-DMSO als Lösungsmittel aufgenommen, mit Ausnahme des Beispiels Nr. 64, welches in CD₃CN als Lösungsmittel aufgenommen wurde. Die Meßtemperatur beträgt 303K, falls d₆-DMSO als Lösungsmittel verwendet wird, und 298K, falls CD₃CN als Lösungsmittel verwendet wird.

In Einzelfällen wurden die Proben mit einem Bruker Avance II 600 oder III 600 bestimmt.

### Anwendungsbeispiele

### Beispiel 1

### Myzus-Test (MYZUPE Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung verwischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 80 % bei einer Aufwandmenge von 100g/ha: 32, 63

Bei diesem Test zeigen z. B. die folgenden Verbindungen der Herstellungsbeispiele Wirkung von 90 % bei einer Aufwandmenge von 100g/ha: 15, 21, 33

### Beispiel 2

### Phaedon -Test (PHAECO Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100 % bei einer Aufwandmenge von 500g/ha: 19, 20

Bei diesem Test zeigen z. B. die folgenden Verbindungen der Herstellungsbeispiele Wirkung von 83 % bei einer Aufwandmenge von 100g/ha : 1

Bei diesem Test zeigen z. B. die folgenden Verbindungen der Herstellungsbeispiele Wirkung von 100 % bei einer Aufwandmenge von 100g/ha : 3,10,11,13,14,15,17,18,21,22, 64,65,67, 68, 71, 73, 74, 76, 77

### Beispiel 3

### Spodoptera frugiperda -Test (SPODFR Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung verwischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 500g/ha : 19, 20

Bei diesem Test zeigen z. B. die folgenden Verbindungen der Herstellungsbeispiele Wirkung von 83 % bei einer Aufwandmenge von 100g/ha: 4, 46, 52

Bei diesem Test zeigen z. B. die folgenden Verbindungen der Herstellungsbeispiele Wirkung von 100 % bei einer Aufwandmenge von 100g/ha: 1,10,11,12,14,17,21,22,33,55,56,59, 63, 64, 65, 66, 67, 68, 71, 73, 74, 76, 77

Bei diesem Test zeigen z. B. die folgenden Verbindungen der Herstellungsbeispiele Wirkung von 100 % bei einer Aufwandmenge von 20g/ha: 15

### Beispiel 4

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung verwischt man 10 mg Wirkstoff mit 0,5 ml Lösungsmittel und verdünnt das Konzentrat mit Lösungsmittel auf die gewünschte Konzentration.

Die Wirkstofflösung wird in das Abdomen *(Boophilus microplus)* injiziert, die Tiere werden in Schalen überführt und in einem klimatisierten Raum aufbewahrt.

Nach 7 Tagen wird die Wirkung in % bestimmt. Die Wirkungskontrolle erfolgt auf Ablage fertiler Eier. Dabei bedeutet 100%, dass keine Zecke fertile Eier gelegt hat.

Bei diesem Test zeigen z.B. die folgenden Verbindungen der Herstellungsbeispiele eine Wirkung von 100% bei einer Aufwandmenge von 20µg / Tier: 15, 22, 63

### Beispiel 5

### Lucilia cuprina-Test (LUCICU)

| | |
|---|---|
| Lösungsmittel: | Dimethylsulfoxid |

Zur Herstellung einer zweckmäßigen Wirkstoffzubereitung verwischt man 10 mg Wirkstoff mit 0,5 ml Dimethylsulfoxid und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit ca 20 *Lucilia cuprina* Larven besetzt.

Nach 48h wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 90% bei einer Aufwandmenge von 100ppm: 11, 12

Bei diesem Test zeigen z.B. die folgenden Verbindungen der Herstellungsbeispiele eine Wirkung von 100% bei einer Aufwandmenge von 100ppm: 15, 21, 22

### Beispiel 6

### Phaedon cochleariae -Test (PHAECO Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

### Spodoptera frugiperda -Test (SPODFR Spritzbehandlung)

| | |
|---|---|
| Lösungsmittel: | 78,0 Gewichtsteile Aceton |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele überlegene Wirksamkeit gegenüber dem Stand der Technik: siehe Tabelle

| Substanz | Struktur | Objekt | Konzentration | % Wirkung |
|---|---|---|---|---|
| Beispiel 18 erfindungsgemäß | 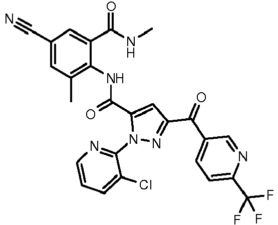 | PHAECO | 100g/ha | 100 7d |
| | | SPODFR | 100g/ha | 100 7d |
| Nr. 1 bekannt WO 2007/144100 | 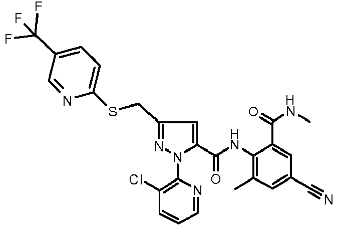 | PHAECO | 100 g/ha | 0 7d |
| | | SPODFR | 100 g/ha | 0 7d |

## Patentansprüche

1. Verbindungen der allgemeinen Formel (I), 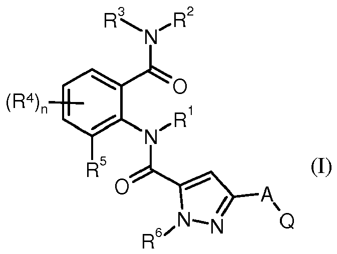 in welcher
R¹ für Wasserstoff, Amino, Hydroxy oder für jeweils gegebenenfalls einfach oder mehrfach, gleich oder verschieden substituiertes C₁-C₆-Alhyl, C₂-C₆-Alkenyl, C₂-C₆-Alkinyl oder C₃-C₆-Cyclo-alkyl steht, wobei die Substituenten unabhängig voneinander ausgewählt sein können aus Halogen, Cyano, Nitro, Hydroxy, C₁-C₄-Alkoxy, C₁-C₄-Alkylthio, C₁-C₄-Alkylsulfinyl, C₁-C₄-Alkylsulfonyl, (C₁-C₄-Alkoxy)carbonyl, C₁-C₄-Alkylamino, Di-(C₁-C₄-alkyl)amino, C₃-C₆-Cycloalkylamino oder (C₁-C₄-Alkyl)C₃-C₆-cycloalkylamino,
R² für Wasserstoff, C₁-C₆-Alkyl, C₂-C₆-Alkenyl, C₂-C₆-Alkinyl, C₃-C₆-Cycloalkyl, C₁-C₄-Alkoxy, C₁-C₄-Alkylamino, Di-(C₁-C₄-alk-yl)amino, C₃-C₆-Cycloalkylamino, C₁-C₆-Alhoxycarbonyl oder C₁-C₆-Alkylcarbonyl steht,
R³ für Wasserstoff oder für jeweils gegebenenfalls einfach oder mehrfach, gleich oder verschieden substituiertes C₁-C₆-Alkyl, C₁-C₆-Alhoxy, C₂-C₆-Alkenyl, C₂-C₆-Alkinyl steht, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₁-C₆-Alkoxycarbonyl, C₁-C₆-Alhylcarbonyl oder C₃-C₆-Trialkylsilyl,
R³ weiterhin für jeweils gegebenenfalls einfach oder mehrfach, gleich oder verschieden substituiertes C₁-C₆-Alkyl, C₁-C₆-Alkoxy, C₂-C₆-Alkenyl, C₂-C₆-Alkinyl steht, wobei die Substituenten unabhängig voneinander ausgewählt sein können aus Amino, C₃-C₆-Cycloalkylamino oder einem 5- oder 6-gliedrigen heteroaromatischen Ring,
R³ ebenfalls weiterhin für C₃-C₁₂-Cycloalkyl, C₃-C₁₂-Cycloalkyl-C₁-C₆-Alkyl und C₄-C₁₂-Bicycloalkyl steht, wobei die Substituenten unabhängig voneinander ausgewählt sein können aus Halogen, Cyano, Nitro, Hydroxy, Amino, C₁-C₆-Alkyl, C₃-C₆-Cycloalkyl, C₃-C₆-Cycloalkylamino, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoxinlino-C₁-C₄-alkyl, C₁-C₄-Alkylsulfoximino-C₂-C₅-alkylcarbonyl, C₁-C₆-Alhoxycarbonyl, C₁-C₆-Alkylcarbonyl, C₃-C₆-Trialkylsilyl oder einem 5- oder 6-gliedrigen heteroaromatischen Ring,
R² und R³ miteinander über zwei bis sechs Kohlenstoffatome verbunden sein können und einen Ring ausbilden, der gegebenenfalls zusätzlich ein weiteres Stickstoff-, Schwefel- oder Sauerstoffatom enthält und gegebenenfalls einfach bis vierfach mit C₁-C₂-Alkyl, Halogen, Cyano, Amino oder C₁-C₂-Alkoxy substituiert sein kann,
R², R³ weiterhin gemeinsam für =S(C₁-C₄-Alkyl)₂, =S(O)(C₁-C₄-Alkyl)₂, stehen,
R⁴ für Wasserstoff, Halogen, Cyano, Nitro, Hydroxy, Amino, (C=O)OH, OCN, SCN, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Alkoxy-C₁-C₄-alkyl, C₁-C₄-Haloalkoxy, SF₅, C₁-C₄-Alkylthio, C₁-C₄-Alkylthio-C₁-C₄-alkyl, C₁-C₄-Alkylsulfinyl, C₁-C₄-Alkylsulfonyl, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, N-Methoxy-N-methylamino, C₁-C₄-Alkoxycarbonyl, C₁-C₄-Alkoxycarbonyl-C₁-C₄-alkyl, C₁-C₄-Alkylcarbonyl, C₁-C₄-Alkylcarbonyloxy, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₃-C₆-Cycloalhylamino, (C₁-C₄-Alkoxy)imino, (C₁-C₄-Alkyl)(C₁-C₄-Alkoxy)imino, (C₁-C₄-Haloalkyl)(C₁-C₄-Alkoxy)imino, C₁-C₄-Alkylsulfonylamino, Aminosulfonyl, C₁-C₄-Alkylaminosulfonyl, C₁-C₄-Dialkylaminosulfonyl, C₁-C₄-Alkylsulfoximino, C₃-C₆-Trialkylsilyl steht oder für einen 3- bis 6-gliedrigen gesättigten, teilgesättigten oder aromatischen Ring steht, der gegebenenfalls ein bis drei Heteroatome aus der Reihe O, S oder N enthatlen kann und der gegebenenfalls einfach oder mehrfach, gleich oder verschieden substituiert ist mit Halogen, Cyano, Nitro, Hydroxy, Amino, Carboxy, C₁-C₄-Alkyl, C₁-C₄-Haloalkyl, C₂-C₆-Alkenyl, C₂-C₆-Haloalkenyl, C₂-C₆-Alkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, C₁-C₄-Alkoxycarbonyl, C₁-C₄-Alkylcarbonyl, C₁-C₄-Alkylcarbonyloxy, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₃-C₆-Cycloalkylamino, C₁-C₄-Alkylsulfonylamino, Aminosulfonyl, C₁-C₄-Alkylaminosulfonyl oder C₁-C₄-Dialkylaminosulfonyl,
zwei R⁴ über benachbarte Kohlenstoffatome einen Ring ausbilden, der für -(CH₂)₃-, -(CH₂)₄-, -(CH₂)₅-, -(CH=CH-)₂-, -OCH₂O-, -O(CH₂)₂O-,-OCF₂O-, -(CF₂)₂O-, -O(CF₂)₂O-, -(CH=CH-CH=N)-oder -(CH=CH-N=CH)- steht, oder
zwei R⁴ weiterhin über benachbarte Kohlenstoffatome die folgenden anellierten Ringe ausbilden, die gegebenenfalls einfach oder mehrfach, gleich oder verschieden substituiert sind, wobei die Substituenten unabhängig voneinander ausgewählt sein können aus Wasserstoff, Cyano, C₁-C₆-Alkyl, C₃-C₆-Cycloalkyl, C₁-C₆-Haloalhyl, C₃-C₆-Halocycloalkyl, Halogen, C₁-C₆-Alkoxy, C₁-C₄-Alkylthio(C₁-C₆-alkyl), C₁-C₄-Alkylsulfinyl(C₁-C₆-alkyl), C₁-C₄-Alkylsulfonyl(C₁-C₆-alkyl), C₁-C₄-Alkylamino, Di-(C₁-C₄-alkyl)amino oder C₃-C₆-Cycloalkylamino, 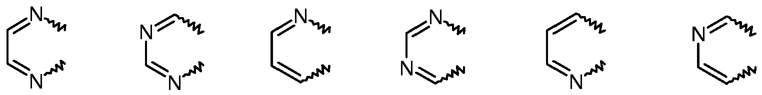 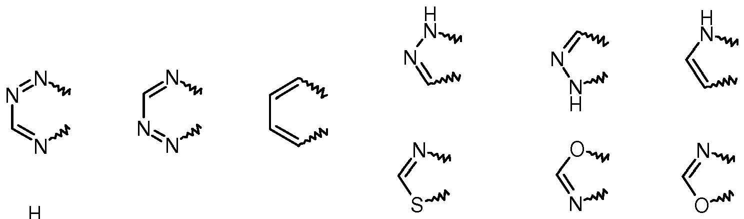 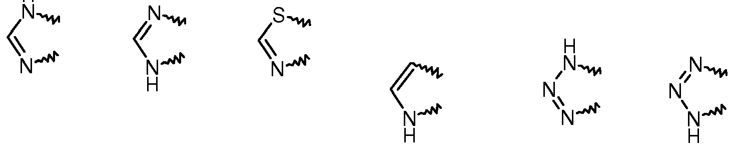 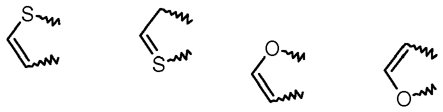
n für 0 bis 3 steht,
R⁵ für C₁-C₆-Alkyl, C₃-C₆-Cycloalkyl, C₁-C₆-Haloalkyl, C₁-C₆-Halocycloalkyl, C₂-C₆-Alkenyl, C₂-C₆-Haloalhenyl, C₂-C₆-Alkinyl, C₂-C₆-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkyl-sulfinyl, C₁-C₄-Haloalkylsulfonyl, Halogen, Cyano, Nitro oder C₃-C₆-Trialkylsilyl steht,
A für -C(=O)-, -CH₂(CO)-, CH₂CH(OH)-, -C(=O)-C(=O)-, -CH=CH-, -C≡C-, Isoxazolin, Imidazolidon, -CH₂NHSO₂CH₂-, -CH₂NMeSO₂CH₂-, CH₂N(SO₂Me)CH₂-, -(CO)CH₂-,-CHCl-, -CCl₂-, -CHF-, -CF₂- steht,
Qz für einen 3- bis 4-gliedrigen, teilweise gesättigten oder gesättigten, oder für einen 5 bis 6-gliedrigen, teilweise gesättigten, gesättigten oder aromatischen Ring steht oder für ein 6 bis 10-gliedriges bizyklisches Ringsystem steht,
wobei der Ring oder das bizyklische Ringsystem gegebenenfalls 1-3 Heteroatome aus der Reihe N, S, O enthalten kann,
wobei der Ring oder das bizyklische Ringsystem gegebenenfalls einfach oder mehrfach, gleich oder verschieden substituiert ist, und wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂NH₂, NO₂, OH, C₁-C₄-Alkoxy, C₁-C₄-Haloalkoxy, C₁-C₄-Alkylthio, C₁-C₄-Alkylsulfonyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alkoxy)carbonyl, (C₁-C₆-Alkyl)aminocarbonyl, Di-(C₁-C₄-alkyl)aminocarbonyl, (=O) oder (=O)₂,
Q für Phenyl steht, welches einfach oder mehrfach durch R¹⁰ substituiert ist, oder für einen 5-oder 6-gliedrigen, teilweise gesättigten oder gesättigten heterozyklischen oder heteroaromatischen Ring, oder ein aromatisches 8-, 9- oder 10-gliedriges annelliertes heterobicyclisches Ringsystem steht, wobei der Ring oder das Ringsystem gegebenenfalls einfach oder mehrfach, gleich oder verschieden durch R¹⁰ substituiert ist,
R¹⁰ für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, CO₂H, CO₂NH₂, NO₂, OH, C₁-C₄-Alkoxy, C₁-C₄-Haloalkoxy, C₁-C₄-Alkylthio, C₁-C₄-Alkylsulfonyl, C₁-C₄-Alkylsulfonyl, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₃-C₆-Cycloalkylamino, (C₁-C₆-Alkyl)carbonyl, (C₁-C₆-Alhoxy)carbonyl, (C₁-C₆-Alkyl)aminocarbonyl, Di-(C₁-C₄-alkyl)aminocarbonyl, Tri-(C₁-C₂)alkylsilyl, (C₁-C₄-Alkyl)(C₁-C₄-Alkoxy)imino oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy, C₁-C₄-Haloalkoxy substituiert sein können,
wobei R¹⁰ nicht für Wasserstoff steht, falls A für -R⁸-C(=O)-R⁸ steht und Q für Phenyl steht,
R⁸ für linear oder verzweigtes -(C₁-C₆-alkylen)- steht oder für eine direkte Bindung steht,
wobei mehrere R⁸ unabhängig voneinander für linear oder verzweigtes-(C₁-C₆-alkylen)- oder für eine direkte Bindung stehen,
R⁶ für Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₃-C₆-Cycloalkoxy oder 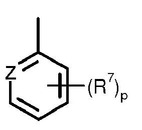 steht,
R⁷ unabhängig voneinander für Wasserstoff, C₁-C₆-Alhyl, C₃-C₆-Cycloalkyl, C₁-C₆-Haloalkyl, Halogen, Cyano, Nitro, C₁-C₄-Alkoxy, C₁-C₄-Haloalkoxy, C₁-C₄-Alkylthio oder C₁-C₄-Haloalkylthio steht,
p für 0 bis 4 steht,
Z für N, CH, CF, CCl, CBr oder CI steht,
die Verbindungen der allgemeinen Formel (I) außerdem N-Oxide und Salze umfassen.

2. Verbindungen der allgemeinen Formel (I) gemäß Anspruch 1,
in welcher
R¹ für Wasserstoff, C₁-C₆-Alkyl, C₂-C₆-Alkenyl, C₂-C₆-Alkinyl, C₃-C₆-Cycloalkyl, Cyano(C₁-C₆-alkyl), C₁-C₆-Haloalkyl, C₂-C₆-Haloalkenyl, C₂-C₆-Haloalkinyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl oder C₁-C₄-Alkylsulfonyl-C₁-C₄-alkyl steht,
R² steht für Wasserstoff oder C₁-C₆-Alhyl.
R³ steht für Wasserstoff oder für jeweils gegebenenfalls einfach oder mehrfach, gleich oder schieden substituiertes C₁-C₆-Alkyl, C₁-C₆-Alkoxy, C₂-C₆-Alkenyl, C₂-C₆-Alkinyl, wobei die Substituenten unabhängig voneinander ausgewählt sein können aus Halogen, Cyano, Nitro Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ steht weiterhin für C₃-C₁₂-Cycloalkyl und C₄-C₁₀-Bicycloalkyl, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkyl-sulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R⁴ für Wasserstoff, C₁-C₄-Alkyl, C₁-C₄-Haloalkyl, Halogen, Cyano, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio Nitro, Hydroxy, Amino, (C=O)OH, C₁-C₄-Alkylsulfonyloxy, C₁-C₄-Haloalkylthio, C₁-C₄-Haloalkylsulfinyl, C₁-C₄-Haloalkylsulfonyl, C₁-C₄-Alkylamino, Di-(C₁-C₄-alkyl)amino, C₁-C₄-Alkylcarbonylamino, C₁-C₄-Alkoxycarbonyl, C₁-C₄-Alkylcarbonyl, Aminocarbonyl, C₁-C₄-Alkylaminocarbonyl, C₁-C₄-Dialkylaminocarbonyl, Aminothiocarbonyl, C₁-C₄-Alkylaminothiocarbonyl, C₁-C₄-Dialkylaminothiocarbonyl, C₁-C₄-Alkylsulfonylamino, Aminosulfonyl, C₁-C₄-Alkylaminosulfonyl oder C₁-C₄-Dialkylaminosulfonyl steht,
zwei benachbarte Reste R⁴ ebenfalls für -(CH₂)₃-, -(CH₂)₄-, -(CH₂)₅-, -(CH=CH-)₂-, -OCH₂O-,-O(CH₂)₂O-,-OCF₂O-, -(CF₂)₂O-, -O(CF₂)₂O-, -(CH=CH-CH=N)- oder -(CH=CH-N=CH)-stehen,
R⁵ für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkyl-sulfinyl, C₁-C₄-Haloalkylsulfonyl, Halogen, Cyano, Nitro oder C₃-C₆-Trialkylsilyl steht,
A für -C(=O)-, -CH₂(CO)-, CH₂CH(OH)-, -C(=O)-C(=O)-, -CH=CH-, -C≡C-, Isoxazolin, Imidazolidon, -CH₂NHSO₂CH₂-, -CH₂NMeSO₂CH₂-, CH₂N(SO₂Me)CH₂-, -(CO)CH₂-, CHCl-, -CCl₂-, -CHF-, -CF₂- steht,
Qz für einen 3- bis 4-gliedrigen, teilweise gesättigten oder gesättigten, oder für einen 5 bis 6-gliedrigen, teilweise gesättigten, gesättigten oder aromatischen Ring steht,wobei der Ring gegebenenfalls 1-3 Heteroatome aus der Reihe N,S,O enthalten kann,
wobei der Ring gegebenenfalls einfach oder mehrfach, gleich oder verschieden substituiert ist, und wobei die Substituenten unabhängig voneinander ausgewählt sein können aus Wasserstoff, C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl,
R⁶ für C₁-C₆-Alkyl steht oder für den Rest 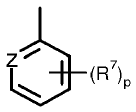 steht,
R⁶ weiterhin für C₃-C₆-Cycloalkoxy steht,
R⁷ unabhängig voneinander für Wasserstoff, Halogen, Cyano, C₁-C₄-Alkyl, C₁-C₄-Alkoxy, C₁-C₄-Haloalkyl, C₁-C₄-Haloalkoxy, C₁-C₄-Haloalkylsulfonyl oder (C₁-C₄-Alkyl)C₁-C₄-Alkoxyimino steht,
p für 1, 2 oder 3 steht,
Z für N, CH, CF, CCl, CBr oder CI steht,
R⁸ für linear oder verzweigtes -(C₁-C₄-alkylen)-oder für eine direkte Bindung steht
R¹⁰ für Wasserstoff, C₁-C₃-Alkyl, C₁-C₃-Haloalkyl, C₁-C₂-Alkoxy, Halogen, Cyano, Hydroxy, Nitro, C₁-C₂-Haloalkoxy oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₆-Alkyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, NO₂, OH, C₁-C₄-Alkoxy oder C₁-C₄-Haloalkoxy substituiert sein können,
wobei R¹⁰ nicht für Wasserstoff steht, falls A für -R⁸-C(=O)-R⁸ steht und Q für Phenyl steht,
Q für einfach oder mehrfach durch R¹⁰ substituiertes Phenyl oder für einen 5-oder 6-gliedrigen, teilweise gesättigten oder gesättigten heterozyklischen oder heteroaromatischen Ring oder ein aromatisches 8-, 9- oder 10-gliedriges annelliertes heterobicyclisches Ringsystem steht, wobei die Heteroatome ausgewählt sein können aus der Reihe N, S, O, wobei der Ring oder das Ringsystem gegebenenfalls einfach oder mehrfach, gleich oder verschieden durch R¹⁰ substituiert ist.

3. Verbindungen der allgemeinen Formel (I) gemäß Anspruch 1,
in welcher
R¹ für Wasserstoff, Methyl, Cyclopropyl, Cyanomethyl, Methoxymethyl, Methylthiomethyl, Methylsulfinylmethyl oder Methylsulfonylmethyl steht,
R² für Wasserstoff oder Methyl steht,
R³ steht für Wasserstoff oder für jeweils gegebenenfalls einfach oder mehrfach, gleich oder verschieden substituiertes C₁-C₆-Alkyl, C₁-C₆-Alkoxy, wobei die Substituenten unabhängig voneinander ausgewählt sein können aus Halogen, Cyano, Nitro, Hydroxy, C₁-C₆-Alkyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R³ steht weiterhin für gegebenenfalls einfach oder mehrfach, gleich oder verschieden substituiertes C₃-C₆-Cycloalkyl, wobei die Substituenten unabhängig voneinander ausgewählt sein können aus Halogen, Cyano, Nitro, Hydroxy, C₁-C₆-Alhyl, C₃-C₆-Cycloalkyl, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Alkylsulfimino, C₁-C₄-Alkylsulfimino-C₁-C₄-alkyl, C₁-C₄-Alkylsulfimino-C₂-C₅-alkylcarbonyl, C₁-C₄-Alkylsulfoximino, C₁-C₄-Alkylsulfoximino-C₁-C₄-alkyl, C₁-C₄-Alkylsulfoximino-C₂-C₅-alkylcarbonyl, C₂-C₆-Alkoxycarbonyl, C₂-C₆-Alkylcarbonyl oder C₃-C₆-Trialkylsilyl,
R⁴ für Wasserstoff, C₁-C₄-Alkyl, C₁-C₂-Haloalkyl, Halogen, Cyano oder C₁-C₂-Haloalkoxy steht
zwei benachbarte Reste R⁴ für -(CH₂)₄-, -(CH=CH-)₂-, -O(CH₂)₂O-, -O(CF₂)₂O-, -(CH=CH-CH=N)-oder -(CH=CH-N=CH)- stehen,
R⁵ für C₁-C₄-Alkyl, C₃-C₆-Cycloalkyl, C₁-C₄-Haloalkyl, C₁-C₆-Halocycloalkyl, C₂-C₆-Alkenyl, C₂-C₄-Haloalkenyl, C₂-C₄-Alkinyl, C₂-C₄-Haloalkinyl, C₁-C₄-Alkoxy, C₁-C₄-Haloalkoxy, Fluor, Chlor, Brom, Iod, Cyano, Nitro oder C₃-C₆-Trialkylsilyl steht,
A für -C(=O)-, -CH₂(CO)-, CH₂CH(OH)-, -C(=O)-C(=O)-, -CH=CH-, -C≡C-, Isoxazolin, Imidazolidon, -CH₂NHSO₂CH₂-, -CH₂NMeSO₂CH₂-, CH₂N(SO₂Me)CH₂-, -(CO)CH₂-, CHCl-, -CCl₂-, -CHF-, -CF₂- steht,
Qz für einen 3- bis 4-gliedrigen, teilweise gesättigten oder gesättigten, oder für einen 5 bis 6 gliedrigen, teilweise gesättigten, gesättigten oder aromatischen Ring steht, wobei der Ring gegebenenfalls 1-2 Heteroatome aus der Reihe N,S,O enthalten kann, wobei der Ring gegebenenfalls einfach oder mehrfach, gleich oder verschieden substituiert ist, und wobei die Substituenten unabhängig voneinander ausgewählt sein können aus Wasserstoff, C₁-C₆-Alhyl, C₂-C₆-Alkenyl, C₂-C₆-Alkinyl, C₃-C₆-Cycloalkyl, C₁-C₆-Haloalkyl, C₂-C₆-Haloalkenyl, C₂-C₆-Haloalkinyl, C₃-C₆-Halocycloalkyl, Halogen, CN, OH, C₁-C₄-Alkoxy, C₁-C₄-Haloalkoxy, C₁-C₄-Alkylthio, C₁-C₄-Alkylsulfinyl, C₁-C₄-Alkylsulfonyl, C₁-C₄-Haloalkylthio, C₁-C₄-Haloalkylsulfinyl, C₁-C₄-Haloalkylsulfonyl,
R⁶ für Methyl steht oder für den Rest 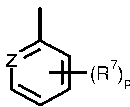 steht,
R⁷ unabhängig voneinander für Wasserstoff, Halogen oder C₁-C₄-Haloalkyl steht,
p für 1 oder 2 steht,
Z für N, CH, CF, CCl oder CBr steht,
R⁸ für Methylen, Ethylen, Propylen, iso-Propylen, n-Butylen, sec-Butylen oder -iso-Butylen oder eine direkte Bindung steht
R¹⁰ für Wasserstoff, C₁-C₃-Alkyl, C₁-C₃-Haloalkyl, C₁-C₂-Alkoxy, Halogen, Cyano, Hydroxy, C₁-C₂-Haloalkoxy oder für Phenyl oder einen 5- oder 6-gliedrigen heteroaromatischen Ring steht, wobei Phenyl oder der Ring gegebenenfalls einfach oder mehrfach, gleich oder verschieden mit C₁-C₃-Alkyl, C₂-C₄-Alkenyl, C₂-C₄-Alkinyl, C₃-C₆-Cycloalkyl, C₁-C₃-Haloalkyl, C₂-C₄-Haloalkenyl, C₂-C₄-Haloalkinyl, C₃-C₆-Halocycloalkyl, Halogen, Cyano, C₁-C₄-Alkoxy, C₁-C₄-Haloalkoxy substituiert sein können,
wobei R¹⁰ nicht für Wasserstoff steht, falls A für -R⁸-C(=O)-R⁸ steht und Q für Phenyl steht,
Q für einfach oder mehrfach durch R¹⁰ substituiertes Phenyl oder für einen gegebenenfalls einfach oder mehrfach durch R¹⁰ substituierten 5- oder 6-gliedrigen heteroaromatischen Ring der Reihe Q-1 bis Q-53 und Q-58 bis Q-59, Q62 bis Q63, einen aromatisches 9-gliedriges annelliertes heterobicyclisches Ringsystem Q-54 bis Q-56 sowie für ein 5-gliedrigen heterocyclischen Ring Q-60 bis Q-61 steht,

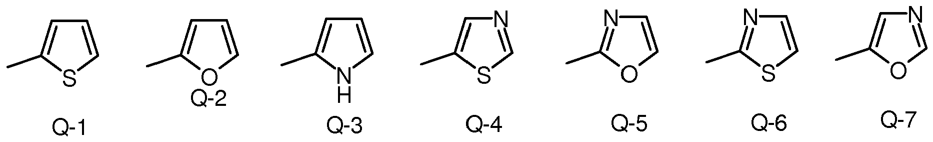

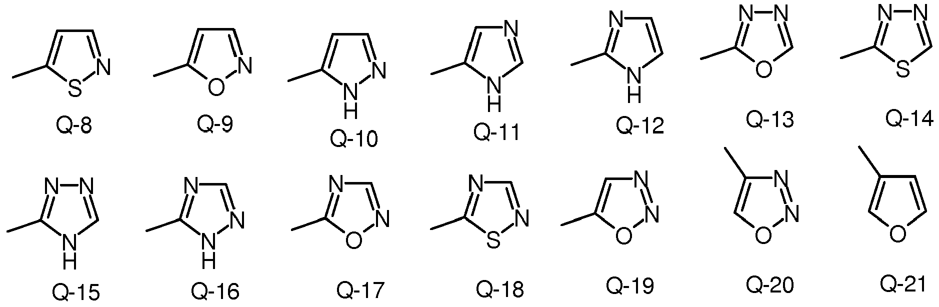

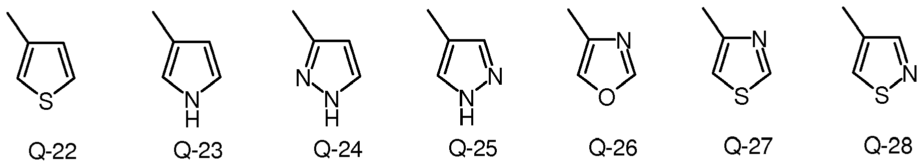

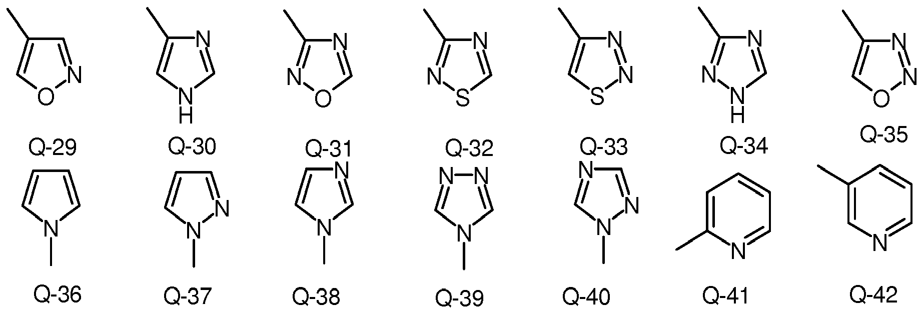

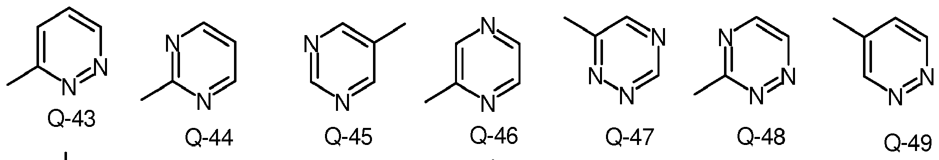

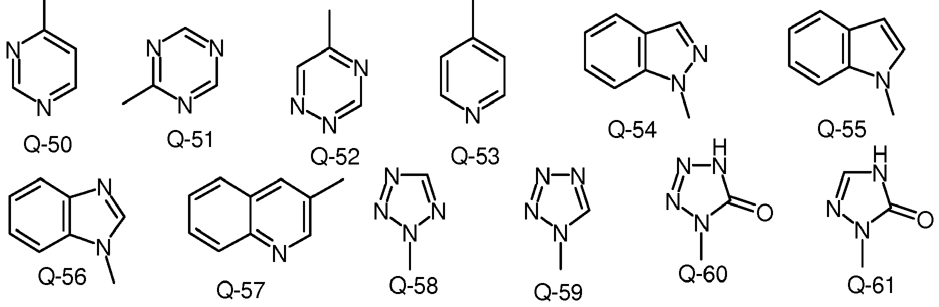

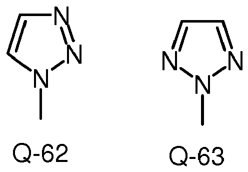

4. Mischungen von Verbindungen der allgemeinen Formel (I) gemäß einem der Ansprüche 1 bis 3, in welchen Q für Q62 und Q63 steht, wobei das Verhältnis einer Verbindung der Formel (I), in welchen Q für Q62 steht, zu einer Verbindung der Formel (I), in welchen Q für Q63 steht, 60:40 bis 99:1 beträgt.

5. Mischungen von Verbindungen der allgemeinen Formel (I) gemäß einem der Ansprüche 1 bis 3, in welchen Q für Q58 und Q59 steht, wobei das Verhältnis einer Verbindung der Formel (I), in welchen Q für Q58 steht, zu einer Verbindung der Formel (I), in welchen Q für Q59 steht, 60:40 bis 99:1 beträgt.

6. Verfahren zur Herstellung von Verbindungen der allgemeinen Formel (I) gemäß einem der Ansprüche 1 bis 3, **dadurch gekennzeichnet, dass** man
(A) Aniline der Formel (II) 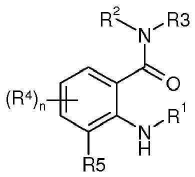 in welcher R¹, R², R³, R⁴, R⁵ und n die oben angegebenen Bedeutungen haben, beispielsweise mit einer Carbonsäure der Formel (III) 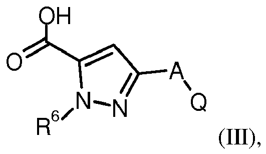 wobei
Q, A und R⁶ die oben angegebenen Bedeutungen haben, in Gegenwart eines Kondensationsmittels umsetzt; oder
(B) Aniline der Formel (II) 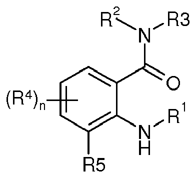 in welcher R¹, R², R³, R⁴, R⁵ und n die oben angegebenen Bedeutungen haben beispielsweise mit Carbonsäurechloriden der Formel (IIIA) 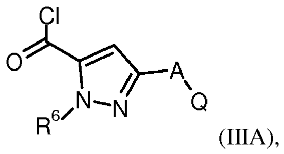 wobei
Q, A und R⁶ die oben angegebenen Bedeutungen haben,
in Gegenwart eines Säurebindungsmittels umsetzt; oder
(C) zur Synthese von Anthranilamiden der Formel (I), in welcher R¹ für Wasserstoff steht, beispielsweise Benzoxazinone der Formel (IIIB) 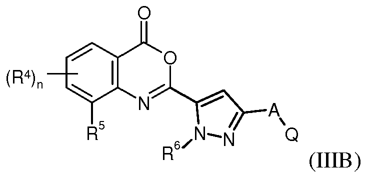 in welcher R⁴, R⁵, R⁶, A, Q und n die oben angegebenen Bedeutungen haben, mit einem Amin der Formel (IIIC) 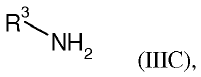 in welcher R³ die oben angegebenen Bedeutungen hat,
in Gegenwart eines Verdünnungsmittels umsetzt zu erfindungsgemäßen Verbindungen der Formel (I).

7. Zusammensetzungen enthaltend mindestens eine Verbindung der Formel (I) oder eine Mischung von Verbindungen der Formel (I) gemäß einem der Ansprüche 1 bis 5 sowie mindestens ein Salz der Formel (XXIV) 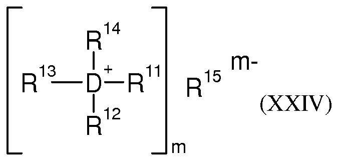 in welcher
D für Stickstoff oder Phosphor steht,
R¹¹, R¹² , R¹³ and R¹⁴ unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
m für 1, 2, 3 oder 4 steht,
R¹⁵ für ein anorganisches oder organisches Anion steht.

8. Zusammensetzungen enthaltend mindestens eine Verbindung der Formel (I) oder eine Mischung von Verbindungen der Formel (I) gemäß einem der Ansprüche 1 bis 5 sowie mindestens einen Penetrationsförderer der Formel (XXV)
R-O-(-AO)_{V-}R' (XXV),
in welcher
R für geradkettiges oder verzweigtes Alkyl mit 4 bis 20 Kohlenstoffatomen steht,
R' für Wasserstoff, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
V für Zahlen von 2 bis 30 steht.

9. Agrochemische Zusammensetzungen enthaltend mindestens eine Verbindung der Formel (I) oder eine Mischung von Verbindungen der Formel (I) gemäß einem der Ansprüche 1 bis 5 oder eine Zusammensetzung gemäß Anspruch 7 oder 8, sowie Streckmittel und/oder oberflächenaktive Stoffe.

10. Verfahren zur Herstellung agrochemischer Zusammensetzungen, **dadurch gekennzeichnet, dass** mindestens eine Verbindung der allgemeinen Formel (I) oder eine Mischung von Verbindungen der allgemeinen Formel (I) gemäß einem der Ansprüche 1 bis 5 oder eine Zusammensetzung gemäß Anspruch 7 oder 8 mit Streckmitteln und/oder oberflächenaktiven Stoffen gemischt wird.

11. Verwendung einer Verbindung der allgemeinen Formel (I) oder einer Mischung von Verbindungen gemäß einem der Ansprüche 1 bis 5 oder einer Zusammensetzung gemäß einem der Ansprüche 7 bis 9 zur Bekämpfung tierischer Schädlinge, ausgenommen zur therapeutischen Behandlung des tierischen und menschlichen Körpers.

12. Verfahren zur Bekämpfung tierischer Schädlinge ausgenommen zur therapeutischen Behandlung des tierischen und menschlichen Körpers, **dadurch gekennzeichnet, dass** man eine Verbindung der allgemeinen Formel (I) oder eine Mischung von Verbindungen der allgemeinen Formel (I) gemäß einem der Ansprüche 1 bis 5 oder eine Zusammensetzung gemäß einem der Ansprüche 7 bis 9 auf tierische Schädlinge und/oder phytopathogene Pilze und/oder deren Lebensraum und/oder Saatgut einwirken lässt.

## Claims

1. Compounds of the general formula (I), 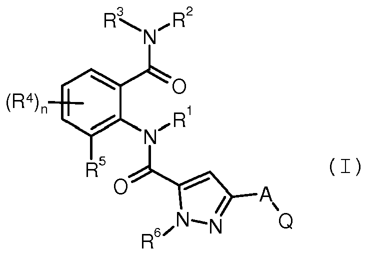 in which
R¹ represents hydrogen, amino, hydroxyl or represents C₁-C₆-alkyl, C₂-C₆-alkenyl, C₂-C₆-alkynyl or C₃-C₆-cycloalkyl, each of which is optionally mono- or polysubstituted by identical or different substituents, where the substituents independently of one another may be selected from the group consisting of halogen, cyano, nitro, hydroxyl, C₁-C₄-alkoxy, C₁-C₄-alkylthio, C₁-C₄-alkylsulphinyl, C₁-C₄-alkylsulphonyl, (C₁-C₄-alkoxy)carbonyl, C₁-C₄-alkylamino, di-(C₁-C₄-alkyl)amino, C₃-C₆-cycloalkylamino and (C₁-C₄-alkyl)-C₃-C₆-cycloalkylamino,
R² represents hydrogen, C₁-C₆-alkyl, C₂-C₆-alkenyl, C₂-C₆-alkynyl, C₃-C₆-cycloalkyl, C₁-C₄-alkoxy, C₁-C₄-alkylamino, di-(C₁-C₄-alkyl)amino, C₃-C₆-cycloalkylamino, C₁-C₆-alkoxycarbonyl or C₁-C₆-alkylcarbonyl,
R³ represents hydrogen or represents in each case optionally mono- or polysubstituted C₁-C₆-alkyl, C₁-C₆-alkoxy, C₂-C₆-alkenyl, C₂-C₆-alkynyl, where the substituents are identical or different and independently of one another may be selected from the group consisting of halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₁-C₆-alkoxycarbonyl, C₁-C₆-alkylcarbonyl and C₃-C₆-trialkylsilyl,
R³ furthermore represents in each case optionally mono- or polysubstituted C₁-C₆-alkyl, C₁-C₆-alkoxy, C₂-C₆-alkenyl, C₂-C₆-alkynyl, where the substituents are identical or different and independently of one another may be selected from the group consisting of amino, C₃-C₆-cycloalkylamino and a 5- or 6-membered heteroaromatic ring,
R³ likewise furthermore represents C₃-C₁₂-cycloalkyl, C₃-C₁₂-cycloalkyl-C₁-C₆-alkyl and C₄-C₁₂-bicycloalkyl, where the substituents independently of one another may be selected from the group consisting of halogen, cyano, nitro, hydroxyl, amino, C₁-C₆-alkyl, C₃-C₆-cycloalkyl, C₃-C₆-cycloalkylamino, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₁-C₆-alkoxycarbonyl, C₁-C₆-alkylcarbonyl, C₃-C₆-trialkylsilyl and a 5- or 6-membered heteroaromatic ring,
R² and R³ may be joined to one another via two to six carbon atoms and form a ring which optionally additionally contains a further nitrogen, sulphur or oxygen atom and may optionally be mono- to tetrasubstituted by C₁-C₂-alkyl, halogen, cyano, amino or C₁-C₂-alkoxy,
R², R³ furthermore together represent =S(C₁-C₄-alkyl)₂, =S (O) (C₁-C₄-alkyl)₂,
R⁴ represents hydrogen, halogen, cyano, nitro, hydroxyl, amino, (C=O)OH, OCN, SCN, C₁-C₄-alkyl, C₁-C₄-haloalkyl, C₂-C₆-alkenyl, C₂-C₆-haloalkenyl, C₂-C₆-alkynyl, C₁-C₄-alkoxy, C₁-C₄-alkoxy-C₁-C₄-alkyl, C₁-C₄-haloalkoxy, SF₅, C₁-C₄-alkylthio, C₁-C₄-alkylthio-C₁-C₄-alkyl, C₁-C₄-alkylsulphinyl, C₁-C₄-alkylsulphonyl, C₁-C₄-alkylsulphonyloxy, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₁-C₄-alkylcarbonylamino, N-methoxy-N-methylamino, C₁-C₄-alkoxycarbonyl, C₁-C₄-alkoxycarbonyl-C₁-C₄-alkyl, C₁-C₄-alkylcarbonyl, C₁-C₄-alkylcarbonyloxy, aminocarbonyl, C₁-C₄-alkylaminocarbonyl, C₁-C₄-dialkylaminocarbonyl, aminothiocarbonyl, C₁-C₄-alkylaminothiocarbonyl, C₁-C₄-dialkylaminothiocarbonyl, C₃-C₆-cycloalkylamino, (C₁-C₄-alkoxy) imino, (C₁-C₄-alkyl) (C₁-C₄-alkoxy)imino, (C₁-C₄-haloalkyl)(C₁-C₄-alkoxy)imino, C₁-C₄-alkylsulphonylamino, aminosulphonyl, C₁-C₄-alkylaminosulphonyl, C₁-C₄-dialkylaminosulphonyl, C₁-C₄-alkylsulphoximino, C₃-C₆-trialkylsilyl or represents a 3- to 6-membered saturated, partially saturated or aromatic ring which may optionally contain one to three heteroatoms from the group consisting of O, S and N and which is optionally mono- or polysubstituted by identical or different substituents from the group consisting of halogen, cyano, nitro, hydroxyl, amino, carboxyl, C₁-C₄-alkyl, C₁-C₄-haloalkyl, C₂-C₆-alkenyl, C₂-C₆-haloalkenyl, C₂-C₆-alkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphonyloxy, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₁-C₄-alkylcarbonylamino, C₁-C₄-alkoxycarbonyl, C₁-C₄-alkylcarbonyl, C₁-C₄-alkylcarbonyloxy, aminocarbonyl, C₁-C₄-alkylaminocarbonyl, C₁-C₄-dialkylaminocarbonyl, aminothiocarbonyl, C₁-C₄-alkylaminothiocarbonyl, C₁-C₄-dialkylaminothiocarbonyl, C₃-C₆-cycloalkylamino, C₁-C₄-alkylsulphonylamino, aminosulphonyl, C₁-C₄-alkylaminosulphonyl and C₁-C₄-dialkylaminosulphonyl,
two radicals R⁴ form, via adjacent carbon atoms, a ring which represents -(CH₂)₃-, -(CH₂)₄-,-(CH₂)₅-, -(CH=CH-)₂-, -OCH₂O-, -O(CH₂)₂O-,-OCF₂O-, -(CF₂)₂O-, -O(CF₂)₂O-, -(CH=CH-CH=N)-or -(CH=CH-N=CH)-, or
two radicals R⁴ furthermore form, via adjacent carbon atoms, the fused rings below which are optionally mono- or polysubstituted by identical or different substituents, where the substituents independently of one another may be selected from the group consisting of hydrogen, cyano, C₁-C₆-alkyl, C₃-C₆-cycloalkyl, C₁-C₆-haloalkyl, C₃-C₆-halocycloalkyl, halogen, C₁-C₆-alkoxy, C₁-C₄-alkylthio (C₁-C₆-alkyl), C₁-C₄-alkylsulphinyl(C₁-C₆-alkyl), C₁-C₄-alkylsulphonyl (C₁-C₆-alkyl), C₁-C₄-alkylamino, di-(C₁-C₄-alkyl) amino and C₃-C₆-cycloalkylamino, 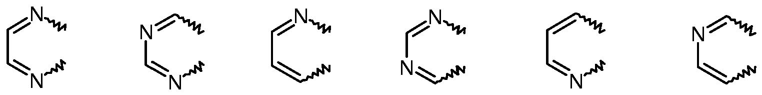 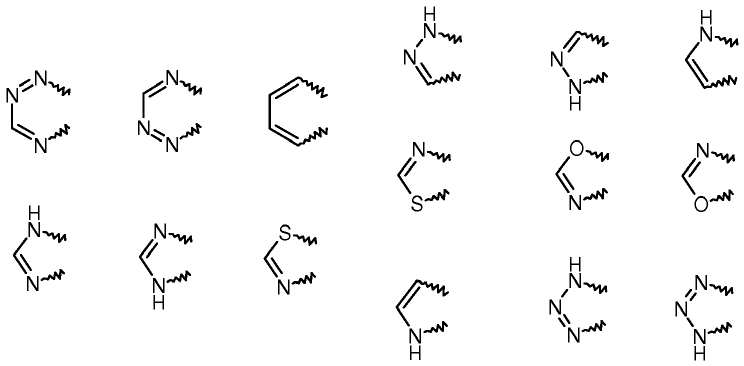 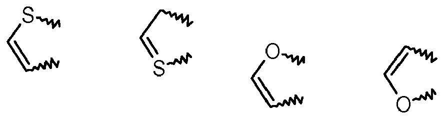
n represents 0 to 3,
R⁵ represents C₁-C₆-alkyl, C₃-C₆-cycloalkyl, C₁-C₆-haloalkyl, C₁-C₆-halocycloalkyl, C₂-C₆-alkenyl, C₂-C₆-haloalkenyl, C₂-C₆-alkynyl, C₂-C₆-haloalkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, halogen, cyano, nitro or C₃-C₆-trialkylsilyl,
A represents -C(=O)-, -CH₂(CO)-, CH₂CH(OH)-,-C(=O)-C(=O)-, -CH=CH-, -C≡C-, isoxazoline, imidazolidone, -CH2NHSO2CH2-, -CH₂NMeSO₂CH₂-, CH₂N(SO₂Me)CH₂-, -(CO)CH₂-, -CHCl-, -CCl₂-,-CHF-, -CF₂-,
Qz represents a 3- or 4-membered partially saturated or saturated or a 5- or 6-membered partially saturated, saturated or aromatic ring or represents a 6- to 10-membered bicyclic ring system,
where the ring or the bicyclic ring system may optionally contain 1-3 heteroatoms from the group consisting of N, S, O,
where the ring or the bicyclic ring system is optionally mono- or polysubstituted by identical or different substituents, and where the substituents independently of one another may be selected from the group consisting of hydrogen, C₁-C₆-alkyl, C₂-C₆-alkenyl, C₂-C₆-alkynyl, C₃-C₆-Cycloalkyl, C₁-C₆-haloalkyl, C₂-C₆-haloalkenyl, C₂-C₆-haloalkynyl, C₃-C₆-halocycloalkyl, halogen, CN, CO₂NH₂, NO₂, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₃-C₆-cycloalkylamino, (C₁-C₆-alkyl)carbonyl, (C₁-C₆-alkoxy)carbonyl, (C₁-C₆-alkyl)aminocarbonyl, di-(C₁-C₄-alkyl)aminocarbonyl, (=O) or (=O)₂,
Q represents phenyl which is mono- or polysubstituted by R¹⁰, or represents a 5-or 6-membered partially saturated or saturated heterocyclic or heteroaromatic ring or an aromatic 8-, 9- or 10-membered fused heterobicyclic ring system, where the ring or the ring system is optionally mono- or polysubstituted by identical or different R¹⁰,
R¹⁰ represents hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, CO₂H, CO₂NH₂, NO₂, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₃-C₆-cycloalkylamino, (C₁-C₆-alkyl)carbonyl, (C₁-C₆-alkoxy)carbonyl, (C₁-C₆-alkyl)aminocarbonyl, di-(C₁-C₄-alkyl)aminocarbonyl, tri-(C₁-C₂)alkylsilyl, (C₁-C₄-alkyl)(C₁-C₄-alkoxy)imino or represents phenyl or a 5- or 6-membered heteroaromatic ring, where phenyl or the ring may optionally be mono- or polysubstituted by identical or different substituents from the group consisting of C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, OH, C₁₃C₄-alkoxy, C₁-C₄-haloalkoxy,
where R¹⁰ does not represent hydrogen if A represents -R⁸-C(=O)-R⁸ and Q represents phenyl,
R⁸ represents straight-chain or branched -(C₁-C₆-alkylene)- or represents a direct bond,
where a plurality of R⁸ radicals independently of one another represent straight-chain or branched - (C₁-C₆-alkylene)- or represent a direct bond,
R⁶ represents hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₃-₆-cycloalkoxy or 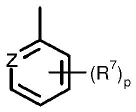
R⁷ independently represents hydrogen, C₁-C₆-alkyl, C₃-C₆-cyloalkyl, C₁-C₆-haloalkyl, halogen, cyano, nitro, C₁-C₄-alkoxy, C₁-C₄-haloalkoxy, C₁-C₄-alkylthio or C₁-C₄-haloalkylthio,
p represents 0 to 4,
Z represents N, CH, CF, CC1, CBr or CI,
the compounds of the general formula (I) furthermore comprise N-oxides and salts.

2. Compounds of the general formula (I) according to Claim 1
in which
R¹ represents hydrogen, C₁-C₆-alkyl, C₂-C₆-alkenyl, C₂-C₆-alkynyl, C₃-C₆-cycloalkyl, cyano (C₁-C₆-alkyl), C₁-C₆-haloalkyl, C₂-C₆-haloalkenyl, C₂-C₆-haloalkynyl, C₁-C₄-alkoxy-C₁-C₄-alkyl, C₁-C₄-alkylthio-C₁-C₄-alkyl, C₁-C₄-alkylsulphinyl-C₁-C₄-alkyl or C₁-C₄-alkylsulphonyl-C₁-C₄-alkyl,
R² represents hydrogen or C₁-C₆-alkyl.
R³ represents hydrogen or represents in each case optionally mono- or polysubstituted C₁-C₆-alkyl, C₁-C₆-alkoxy, C₂-C₆-alkenyl, C₂-C₆-alkynyl, where the substituents are identical or different and independently of one another may be selected from the group consisting of halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl and C₃-C₆-trialkylsilyl,
R³ furthermore represents C₃-C₁₂-cycloalkyl or C₄-C₁₀bicycloalkyl, where the substituents independently of one another may be selected from the group consisting of halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylkylsulphoximino-C₂-C₅-alkylcarbonyl, C₂-C₆-alkoxycarbonyl, C₂-C₆-alkylcarbonyl and C₃-C₆-trialkylsilyl,
R⁴ represents hydrogen, C₁-C₄-alkyl, C₁-C₄-haloalkyl, halogen, cyano, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, nitro, hydroxyl, amino, (C=O)OH, C₁-C₄-alkylsulphonyloxy, C₁-C₄-haloalkylthio, C₁-C₄-haloalkylsulphinyl, C₁-C₄-haloalkylsulphonyl, C₁-C₄-alkylamino, di-(C₁-C₄-alkyl)amino, C₁-C₄-alkylcarbonylamino, C₁-C₄-alkoxycarbonyl, C₁-C₄-alkylcarbonyl, aminocarbonyl, C₁-C₄-alkylaminocarbonyl, C₁-C₄-dialkylaminocarbonyl, aminothiocarbonyl, C₁-C₄-alkylaminothiocarbonyl, C₁-C₄-dialkylaminothiocarbonyl, C₁-C₄-alkylsulphonylamino, aminosulphonyl, C₁-C₄-alkylaminosulphonyl or C₁-C₄-dialkylaminosulphonyl,
two adjacent radicals R⁴ likewise represent-(CH2)₃-, -(CH₂)₄-, -(CH₂)₅-, -(CH=CH-)₂-,-OCH₂O-, -O(CH₂)₂O-, -OCF₂O-, -(CF₂)₂O-,-O(CF₂)₂O-, -(CH=CH-CH=N)- or -(CH=CH-N=CH)-,
R⁵ represents C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl, halogen, cyano, nitro or C₃-C₆-trialkylsilyl,
A represents -C(=O)-, -CH₂(CO)-, CH₂CH(OH)-,-C(=O)-C(=O)-, -CH=CH-, -C≡C-, isoxazoline, imidazolidone, -CH2NHSO2CH2-, -CH₂NMeSO₂CH₂-, CH₂N(SO₂Me)CH₂-, -(CO)CH₂- , -CHCl-, -CCl₂-,-CHF-, -CF₂-,
Qz represents a 3- or 4-membered partially saturated or saturated ring or represents a 5- or 6-membered partially saturated, saturated or aromatic ring, where the ring may optionally contain 1-3 heteroatoms from the group consisting of N, S, O, where the ring is optionally mono- or polysubstituted by identical or different substituents and where the substituents independently of one another may be selected from the group consisting of hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl,
R⁶ represents C₁-C₆-alkyl or represents the radical 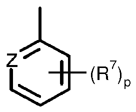
R⁶ furthermore represents C₃-C₆-cycloalkoxy,
R⁷ independently represents hydrogen, halogen, cyano, C₁-C₄-alkyl, C₁-C₄-alkoxy, C₁-C₄-haloalkyl, C₁-C₄-haloalkoxy, C₁-C₄-haloalkylsulphonyl or (C₁-C₄-alkyl)-C₁-C₄-alkoxyimino,
p represents 1, 2 or 3,
Z represents N, CH, CF, CC1, CBr or CI,
R⁸ represents straight-chain or branched -(C₁-C₄-alkylene)- or represents a direct bond
R¹⁰ represents hydrogen, C₁-C₃-alkyl, C₁-C₃-haloalkyl, C₁-C₂-alkoxy, halogen, cyano, hydroxyl, nitro, C₁-C₂-haloalkoxy or represents phenyl or a 5- or 6-membered heteroaromatic ring, where phenyl or the ring may optionally be mono- or polysubstituted by identical or different substituents from the group consisting of C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, NO₂, OH, C₁-C₄-alkoxy and C₁-C₄-haloalkoxy, where R¹⁰ does not represent hydrogen if A represents -R⁸-C(=O)-R⁸ and Q represents phenyl,
Q represents phenyl which is mono- or polysubstituted by R¹⁰ or represents a 5- or 6-membered partially saturated or saturated heterocyclic or heteroaromatic ring or an aromatic 8-, 9- or 10-membered fused heterobicyclic ring system, where the heteroatoms may be selected from the group consisting of N, S, O, where the ring or the ring system is optionally mono- or polysubstituted by identical or different R¹⁰.

3. Compounds of the general formula (I) according to Claim 1
in which
R¹ represents hydrogen, methyl, cyclopropyl, cyanomethyl, methoxymethyl, methylthiomethyl, methylsulphinylmethyl or methylsulphonylmethyl,
R² represents hydrogen or methyl,
R³ represents hydrogen or represents in each case optionally mono- or polysubstituted C₁-C₆-alkyl, C₁-C₆-alkoxy, where the substituents are identical or different and independently of one another may be selected from the group consisting of halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl and C₃-C₆-trialkylsilyl,
R³ furthermore represents optionally mono- or polysubstituted C₃-C₆-cycloalkyl, where the substituents are identical or different and independently of one another may be selected from the group consisting of halogen, cyano, nitro, hydroxyl, C₁-C₆-alkyl, C₃-C₆-cycloalkyl, C₁-C₄-alkoxy, C₁-C₄-haloalkoxy, C₁-C₄-alkylthio, C₁-C₄-alkylsulphinyl, C₁-C₄-alkylsulphonyl, C₁-C₄-alkylsulphimino, C₁-C₄-alkylsulphimino-C₁-C₄-alkyl, C₁-C₄-alkylsulphimino-C₂-C₅-alkylcarbonyl, C₁-C₄-alkylsulphoximino, C₁-C₄-alkylsulphoximino-C₁-C₄-alkyl, C₁-C₄-alkylsulphoximino-C₂-C₅-alkylcarbonyl, C₂-C₆-alkoxycarbonyl, C₂-C₆-alkylcarbonyl and C₃-C₆-trialkylsilyl,
R⁴ represents hydrogen, C₁-C₄-alkyl, C₁-C₂-haloalkyl, halogen, cyano or C₁-C₂-haloalkoxy,
two adjacent radicals R⁴ represent -(CH₂)₄-,-(CH=CH-)₂-, -O(CH₂)₂O-, -O(CF₂)₂O-, -(CH=CH-CH=N)- or - (CH=CH-N=CH) -,
R⁵ represents C₁-C₄-alkyl, C₃-C₆-cycloalkyl, C₁-C₄-haloalkyl, C₁-C₆-halocycloalkyl, C₂-C₆-alkenyl, C₂-C₄-haloalkenyl, C₂-C₄-alkynyl, C₂-C₄-haloalkynyl, C₁-C₄-alkoxy, C₁-C₄-haloalkoxy, fluorine, chlorine, bromine, iodine, cyano, nitro or C₃-C₆-trialkylsilyl,
A represents -C(=O)-, -CH₂(CO)-, CH₂CH(OH)-,-C(=O)-C(=O)-, -CH=CH-, -C≡C-, isoxazoline, imidazolidone, -CH2NHSO2CH2-, -CH₂NMeSO₂CH₂-, CH₂N(SO₂Me)CH₂-, -(CO)CH₂-, -CHCl-, -CCl₂-,-CHF-, -CF₂-,
Qz represents a 3- or 4-membered partially saturated or saturated ring or represents a 5- or 6-membered partially saturated, saturated or aromatic ring, where the ring may optionally contain 1-2 heteroatoms from the group consisting of N, S, O+, where the ring is optionally mono- or polysubstituted by identical or different substituents and where the substituents independently of one another may be selected from the group consisting of hydrogen, C₁-C₆-alkyl, C₂-C₆-alkenyl, C₂-C₆-alkynyl, C₃-C₆-cycloalkyl, C₁-C₆-haloalkyl, C₂-C₆-haloalkenyl, C₂-C₆-haloalkynyl, C₃-C₆-halocycloalkyl, halogen, CN, OH, C₁-C₄-alkoxy, C₁-C₄-haloalkoxy, C₁-C₄-alkylthio, C₁-C₄-alkylsulphinyl, C₁-C₄-alkylsulphonyl, C₁-C₄-haloalkylthio, C₁-C₄-haloalkylsulphinyl, C₁-C₄-haloalkylsulphonyl,
R⁶ represents methyl or represents the radical 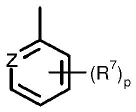
R⁷ independently represents hydrogen, halogen or C₁-C₄-haloalkyl,
p represents 1 or 2,
Z represents N, CH, CF, CC1 or CBr,
R⁸ represents methylene, ethylene, propylene, isopropylene, n-butylene, sec-butylene or isobutylene or a direct bond
R¹⁰ represents hydrogen, C₁-C₃-alkyl, C₁-C₃-haloalkyl, C₁-C₂-alkoxy, halogen, cyano, hydroxyl, C₁-C₂-haloalkoxy or represents phenyl or a 5- or 6-membered heteroaromatic ring, where phenyl or the ring may optionally be mono- or polysubstituted by identical or different substituents from the group consisting of C₁-C₃-alkyl, C₂-C₄-alkenyl, C₂-C₄-alkynyl, C₃-C₆-cycloalkyl, C₁-C₃-haloalkyl, C₂-C₄-haloalkenyl, C₂-C₄-haloalkynyl, C₃-C₆-halocycloalkyl, halogen, cyano, C₁-C₄-alkoxy, C₁-C₄-haloalkoxy,
where R¹⁰ does not represent hydrogen if A represents -R⁸-C(=O)-R⁸ and Q represents phenyl,
Q represents phenyl which is mono- or polysubstituted by R¹⁰ or represents a 5- or 6-membered heteroaromatic ring from the group consisting of Q-1 to Q-53 and Q-58 to Q-59, Q62 to Q63 which is optionally mono- or polysubstituted by R¹⁰, an aromatic 9-membered fused heterobicyclic ring system Q-54 to Q-56 or represents a 5-membered heterocyclic ring Q-60 to Q-61,

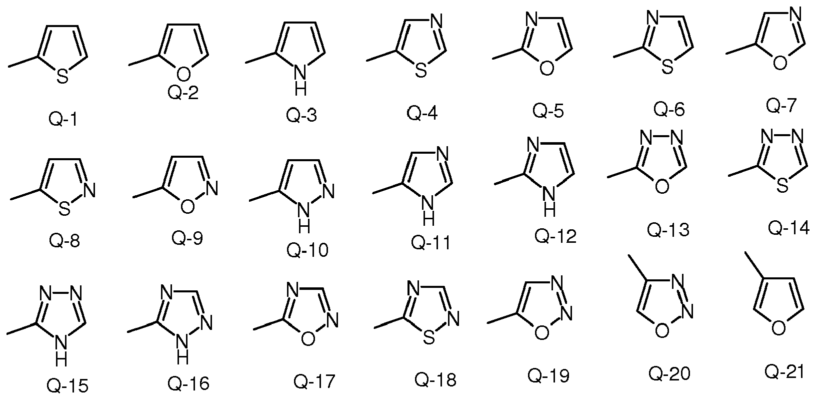

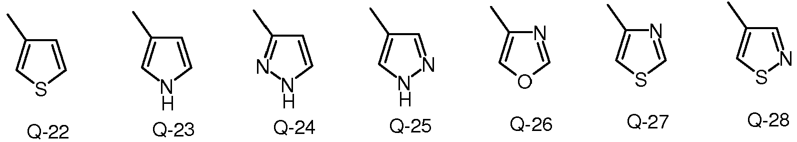

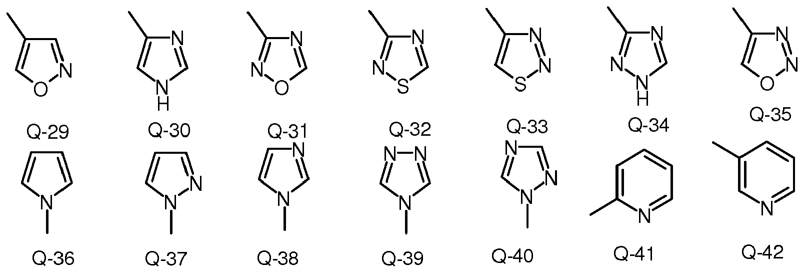

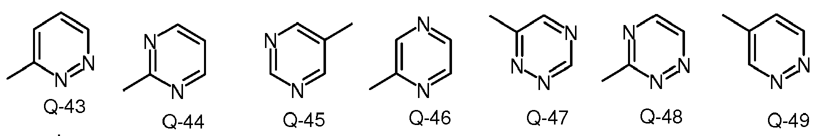

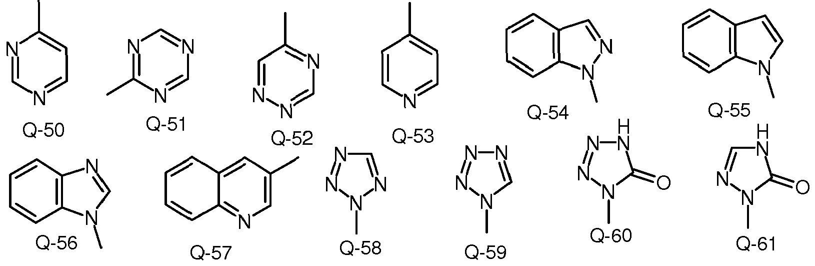

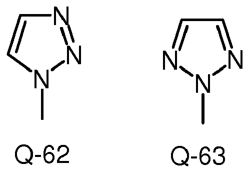

4. Mixtures of compounds of the general formula (I) according to any of Claims 1 to 3, in which Q is Q62 and Q63, the ratio of a compound of the formula (I) in which Q is Q62 to a compound of the formula (I) in which Q is Q63 being 60:40 to 99:1.

5. Mixtures of compounds of the general formula (I) according to any of Claims 1 to 3, in which Q is Q58 and Q59, the ratio of a compound of the formula (I) in which Q is Q58 to a compound of the formula (I) in which Q is Q59 being 60:40 to 99:1.

6. Process for preparing compounds of the general formula (I) according to any of Claims 1 to 3, **characterized in that**
(A) anilines of the formula (II) 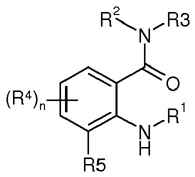 in which R¹, R², R³, R⁴, R⁵ and n have the meanings given above
are reacted, for example, with a carboxylic acid of the formula (III) 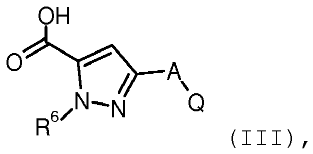 where
Q, A and R⁶ have the meanings given above,
in the presence of a condensing agent; or
(B) anilines of the formula (II) 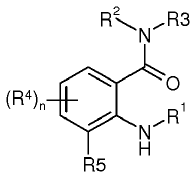 in which R¹, R², R³, R⁴, R⁵ and n have the meanings given above
are reacted, for example, with carbonyl chlorides of the formula (IIIA) 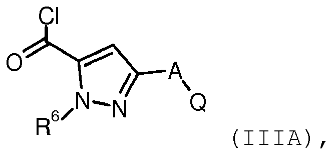 where
Q, A and R⁶ have the meanings given above,
in the presence of an acid-binding agent; or
(C) for the synthesis of anthranilimides of the formula (I) in which R¹ represents hydrogen,
for example benzoxazinones of the formula (IIIB) 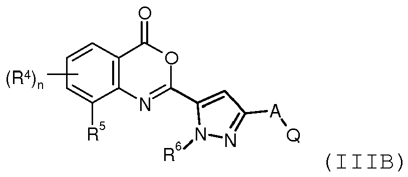 in which R⁴, R⁵, R⁶, A, Q and n have the meanings given above
are reacted with an amine of the formula (IIIC) 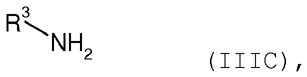 in which R³ has the meanings given above in the presence of a diluent to give compounds of the formula (I) according to the invention.

7. Compositions, comprising at least one compound of the formula (I) or a mixture of compounds of the formula (I) according to any of Claims 1 to 5 and at least one salt of the formula (XXIV) 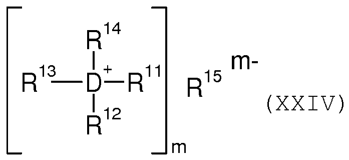 in which
D represents nitrogen or phosphorus,
R¹¹, R¹², R¹³ and R¹⁴ independently of one another represent hydrogen or in each case optionally substituted C₁-C₈-alkyl or mono- or polyunsaturated, optionally substituted C₁-C₈-alkylene where the substituents may be selected from the group consisting of halogen, nitro and cyano,
m represents 1, 2, 3 or 4,
R¹⁵ represents an inorganic or organic anion,

8. Compositions comprising at least one compound of the formula (I) or a mixture of compounds of the formula (I) according to any of Claims 1 to 5 and at least one penetrant of the formula (XXV)
R-O-(-AO)_{V-}R' (XXV)
in which
R represents straight-chain or branched alkyl having 4 to 20 carbon atoms
R' represents hydrogen, methyl, ethyl, n-propyl, isopropyl, n-butyl, isobutyl, tert-butyl, n-pentyl or n-hexyl,
AO represents an ethylene oxide radical, a propylene oxide radical, a butylene oxide radical or represents mixtures of ethylene oxide and propylene oxide radicals or butylene oxide radicals and
V represents a number from 2 to 30.

9. Agrochemical compositions comprising at least one compound of the formula (I) or a mixture of compounds of the formula (I) according to any of Claims 1 to 5 or a composition according to Claim 7 or 8, and also extenders and/or surfactants.

10. Process for producing agrochemical compositions, **characterized in that** at least one compound of the general formula (I) or a mixture of compounds of the general formula (I) according to any of Claims 1 to 5 or a composition according to Claim 7 or 8 is mixed with extenders and/or surfactants.

11. Use of a compound of the general formula (I) or of a mixture of compounds according to any of Claims 1 to 5 or of a composition according to any of Claims 7 to 9 for controlling animal pests, with the exception being for the therapeutic treatment of the animal and human body.

12. Method for controlling animal pests with the exception being for the therapeutic treatment of the animal and human body, **characterized in that** a compound of the general formula (I) or a mixture of compounds of the general formula (I) according to any of Claims 1 to 5 or a composition according to any of Claims 7 to 9 is applied to animal pests and/or phytopathogenic fungi and/or their habitat and/or seed.

## Revendications

1. Composés de formule générale (I), 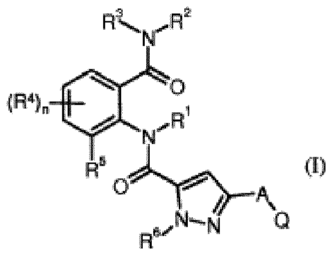 dans laquelle
R¹ représente un atome d'hydrogène, un groupe amino, hydroxy, ou un groupe alkyle en C₁-C₆, alcényle en C₂-C₆, alcynyle en C₂-C₆ ou cycloalkyle en C₃-C₆, chacun portant éventuellement un ou plusieurs substituants identiques ou différents, les substituants pouvant être choisis indépendamment les uns des autres parmi halogéno, cyano, nitro, hydroxy, alcoxy en C₁-C₄, alkyl(C₁-C₄)thio, alkyl(C₁-C₄)sulfinyle, alkyl(C₁-C₄)sulfonyle, (alcoxy(C₁-C₄))carbonyle, alkyl(C₁-C₄)amino, dialkyl(C₁-C₄)amino, cycloalkyl(C₃-C₆)amino ou (alkyl(C₁-C₄))-cycloalkyl(C₃-C₆)amino,
R² représente un atome d'hydrogène, un groupe alkyle en C₁-C₆, alcényle en C₂-C₆, alcynyle en C₂-C₆, cycloalkyle en C₃-C₆, alcoxy en C₁-C₄, alkyl(C₁-C₄)amino, di-(alkyl(C₁-C₄))amino, cycloalkyl(C₃-C₆)amino, alcoxy(C₁-C₆)carbonyle ou alkyl(C₁-C₆)carbonyle,
R³ représente un atome d'hydrogène ou un groupe alkyle en C₁-C₆, alcoxy en C₁-C₆, alcényle en C₂-C₆, alcynyle en C₂-C₆ chacun portant éventuellement un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)-sulfimino-alkylcarbonyle(C₂-C₅), alkyl(C₁-C₄)-sulfoximino, alkyl(C₁-C₄)sulfoximino-alkyle(C₁-C₄), alkyl(C₁-C₄)sufoximino-alkylcarbonyle(C₂-C₅), alcoxy(C₁-C₆)carbonyle, alkyl(C₁-C₆)carbonyle ou trialkyl(C₃-C₆)silyle,
R³ représente en outre un groupe alkyle en C₁-C₆, alcoxy en C₁-C₆, alcényle en C₂-C₆, alcynyle en C₂-C₆ chacun portant éventuellement un ou plusieurs substituants identiques ou différents, les substituants pouvant être choisis indépendamment les uns des autres parmi amino, cycloalkyl(C₃-C₆)-amino ou un cycle hétéroaromatique à 5 ou 6 chaînons,
R³ représente en outre également un groupe cycloalkyle en C₃-C₁₂, cycloalkyl(C₃-C₁₂)-alkyle(C₁-C₆) et bicycloalkyle en C₄-C₁₂, les substituants pouvant être choisis indépendamment les uns des autres parmi halogéno, cyano, nitro, hydroxy, amino, alkyle en C₁-C₆, cycloalkyle en C₃-C₆, cycloalkyl(C₃-C₆)amino, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)sulfimino-alkyl-carbonyle(C₂-C₅), alkyl(C₁-C₄)sulfoximino, alkyl(C₁-C₄)sulfoximino-alkyle(C₁-C₄), alkyl(C₁-C₄)-sulfoximino-alkylcarbonyle(C₂-C₅), alcoxy(C₁-C₆)-carbonyle, alkyl(C₁-C₆)carbonyle, trialkyl(C₃-C₆)-silyle ou un cycle hétéroaromatique à 5 ou 6 chaînons,
R² et R³ peuvent être liés l'un à l'autre par deux à six atomes de carbone et former un cycle qui contient éventuellement en plus un autre atome d'azote, de soufre ou d'oxygène et peut éventuellement être une à quatre fois substitué par alkyle en C₁-C₂, halogéno, cyano, amino ou alcoxy en C₁-C₂,
R², R³ en outre représentent ensemble =S(alkyle(C₁-C₄))₂, =S(O)(alkyle(C₁-C₄))₂,
R⁴ représente un atome d'hydrogène ou d'halogène, un groupe cyano, nitro, hydroxy, amino, (C=O)OH, OCN, SCN, alkyle en C₁-C₄, halogénoalkyle(C₁-C₄), alcényle en C₂-C₆, halogénoalcényle(C₂-C₆), alcynyle en C₂-C₆, alcoxy en C₁-C₄, alcoxy(C₁-C₄)-alkyle(C₁-C₄), halogénoalcoxy(C₁-C₄), SF₅, alkyl(C₁-C₄)thio, alkyl(C₁-C₄)thio-alkyle(C₁-C₄), alkyl(C₁-C₄)sulfinyle, alkyl(C₁-C₄)sulfonyle, alkyl(C₁-C₄)sulfonyloxy, halogénoalkyl(C₁-C₄)thio, halogénoalkyl(C₁-C₄)sulfinyle, halogéno-alkyl(C₁-C₄)sulfonyle, alkyl(C₁-C₄)amino, di-(alkyl(C₁-C₄))amino, alkyl(C₁-C₄)carbonylamino, N-méthoxy-N-méthylamino, alcoxy(C₁-C₄)carbonyle, alcoxy (C₁-C₄)carbonyl-alkyle(C₁-C₄), alkyl(C₁-C₄)-carbonyle, alkyl(C₁-C₄)carbonyloxy, aminocarbonyle, alkyl(C₁-C₄)aminocarbonyle, dialkyl(C₁-C₄)amino-carbonyle, aminothiocarbonyle, alkyl(C₁-C₄)amino-thiocarbonyle, dialkyl(C₁-C₄)aminothiocarbonyle, cycloalkyl(C₃-C₆)amino, (alcoxy(C₁-C₄))imino, (alkyl(C₁-C₄))(alcoxy(C₁-C₄))imino, (halogéno-alkyl(C₁-C₄))(alcoxy(C₁-C₄))imino, alkyl(C₁-C₄)-sulfonylamino, aminosulfonyle, alkyl(C₁-C₄)amino-sulfonyle, dialkyl(C₁-C₄)aminosulfonyle, alkyl(C₁-C₄)sulfoximino, trialkyl(C₃-C₆)silyle ou un cycle saturé, partiellement saturé ou aromatique à 3 à 6 chaînons, qui peut éventuellement contenir un à trois hétéroatomes choisies dans la série constituée par O, S et N et qui porte éventuellement un ou plusieurs substituants identiques ou différents halogéno, cyano, nitro, hydroxy, amino, carboxy, alkyle en C₁-C₄, halogénoalkyle(C₁-C₄), alcényle en C₂-C₆, halogénoalcényle(C₂-C₆), alcynyle en C₂-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfonyloxy, halogénoalkyl(C₁-C₄)thio, halogénoalkyl(C₁-C₄)sulfinyle, halogéno-alkyl(C₁-C₄)sulfonyle, alkyl(C₁-C₄)amino, di-(alkyl(C₁-C₄))amino, alkyl(C₁-C₄)carbonylamino, alcoxy(C₁-C₄)carbonyle, alkyl(C₁-C₄)carbonyle, alkyl(C₁-C₄)carbonyloxy, aminocarbonyle, alkyl(C₁-C₄)aminocarbonyle, dialkyl(C₁-C₄)amino-carbonyle, aminothiocarbonyle, alkyl(C₁-C₄)amino-thiocarbonyle, dialkyl(C₁-C₄)aminothiocarbonyle, cycloalkyl(C₃-C₆)amino, alkyl(C₁-C₄)sulfonylamino, aminosulfonyle, alkyl(C₁-C₄)aminosulfonyle ou dialkyl(C₁-C₄)aminosulfonyle,
deux radicaux R⁴ forment avec les atomes de carbone voisins un cycle qui représente -(CH₂)₃-, -(CH₂)₄-, -(CH₂)₅-, -(CH=CH-)₂-, -OCH₂O-, -O(CH₂)₂O-, -OCF₂O-, -(CF₂)₂O-, -O(CF₂)₂O-, -(CH=CH-CH=N)- ou -(CH=CH-N=CH)-, ou
deux radicaux R⁴ en outre forment avec les atomes de carbone voisins les cycles condensés suivants, qui portent éventuellement un ou plusieurs substituants identiques ou différents, les substituants pouvant être choisis indépendamment les uns des autres parmi un atome d'hydrogène, des groupes cyano, alkyle en C₁-C₆, cycloalkyle en C₃-C₆, halogénoalkyle(C₁-C₆), halogénocyclo-alkyle(C₃-C₆), halogéno, alcoxy en C₁-C₆, alkyl(C₁-C₄)thio (alkyle(C₁-C₆)), alkyl(C₁-C₄)-sulfinyl(alkyle(C₁-C₆)), alkyl(C₁-C₄)sulfonyl-(alkyle(C₁-C₆)), alkyl(C₁-C₄)amino, di-(alkyl(C₁-C₄))amino ou cycloalkyl(C₃-C₆)amino, 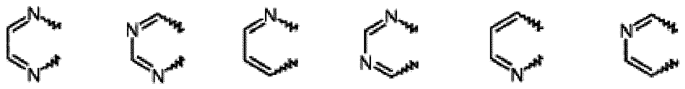 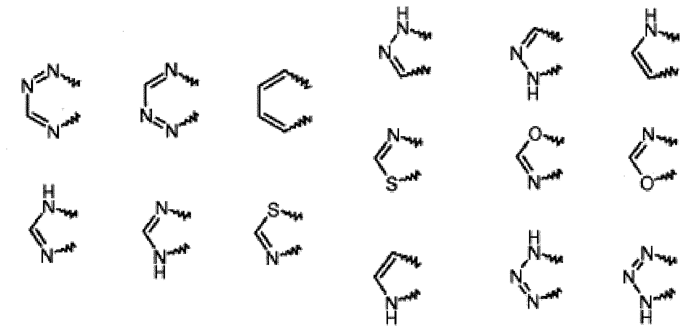 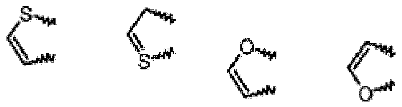
n représente 0 à 3
R⁵ représente un groupe alkyle en C₁-C₆, cycloalkyle en C₃-C₆, halogénoalkyle(C₁-C₆), halogéno-cyloalkyle(C₁-C₆), alcényle en C₂-C₆, halogénoalcényle(C₂-C₆), alcynyle en C₂-C₆, halogénoalcynyle(C₂-C₆), alcoxy en C₁-C₄, halogénoalcoxy(C₁-C₄), alkyl(C₁-C₄)thio, alkyl(C₁-C₄)sulfinyle, alkyl(C₁-C₄)sulfonyle, halogénoalkyl(C₁-C₄)thio, halogénoalkyl(C₁-C₄)-sulfinyle, halogénoalkyl(C₁-C₄)sulfonyle, un atome d'halogène, un groupe cyano, nitro ou trialkyl(C₃-C₆)silyle,
A représente -C(=O)-, -CH₂(CO)-, CH₂CH(OH)-, -C(=O)-C(=O)-, -CH=CH-, -C≡ºC-, isoxazoline, imidazolidone, -CH₂NHSO₂CH₂-, -CH₂NMeSO₂CH₂-, CH₂N(SO₂Me)CH₂-, -(CO)CH₂-, -CHCl-, -CCl₂-, -CHF-, -CF₂-,
Q_{z} représente un cycle à 3 ou 4 chaînons, saturé ou partiellement saturé, ou un cycle à 5 ou 6 chaînons, saturé, partiellement saturé ou aromatique, ou un système cyclique bicyclique à 6 à 10 chaînons, le cycle ou le système cyclique bicyclique pouvant éventuellement contenir 1-3 hétéroatomes choisis dans la série constituée par N, S, O, le cycle ou le système cyclique bicyclique portant éventuellement un ou plusieurs substituants, identiques ou différents, et les substituants pouvant être choisis indépendamment les uns des autres parmi un atome d'hydrogène, des groupes alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogénocycloalkyle(C₃-C₆), halogéno, CN, CO₂NH₂, NO₂, OH, alcoxy en C₁-C₄, halogénoalcoxy(C₁-C₄), alkyl(C₁-C₄)thio, alkyl(C₁-C₄)sulfinyle, alkyl(C₁-C₄)sulfonyle, halogénoalkyl(C₁-C₄)thio, halogénoalkyl(C₁-C₄)sulfinyle, halogéno-alkyl(C₁-C₄)sulfonyle, alkyl(C₁-C₄)amino, di-(alkyl(C₁-C₄))amino, cycloalkyl(C₃-C₆)amino, (alkyl(C₁-C₆))carbonyle, (alcoxy(C₁-C₆))carbonyle, (alkyl(C₁-C₆))aminocarbonyle, di-(alkyl(C₁-C₄))-aminocarbonyle, (=O) ou (=O)₂,
Q représente un cycle phényle qui est une ou plusieurs fois substitué par R¹⁰, ou un cycle hétérocyclique saturé ou partiellement saturé ou hétéroaromatique à 5 ou 6 chaînons, ou un système cyclique hétérobicyclique aromatique condensé à 8, 9 ou 10 chaînons, le cycle ou le système cyclique portant éventuellement un ou plusieurs substituants R¹⁰ identiques ou différents,
R¹⁰ représente un atome d'hydrogène, un groupe alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogénocycloalkyle(C₃-C₆), un atome d'halogène, un groupe CN, CO₂H, CO₂NH₂, NO₂, OH, alcoxy en C₁-C₄, halogénoalcoxy(C₁-C₄), alkyl(C₁-C₄)thio, alkyl(C₁-C₄)sulfinyle, alkyl(C₁-C₄)sulfonyle, halogénoalkyl(C₁-C₄)thio, halogénoalkyl(C₁-C₄)-sulfinyle, halogénoalkyl(C₁-C₄)sulfonyle, alkyl(C₁-C₄)amino, di-(alkyl(C₁-C₄))amino, cycloalkyl(C₃-C₆)amino, (alkyl(C₁-C₆))carbonyle, (alcoxy(C₁-C₆))carbonyle, (alkyl(C₁-C₆))amino-carbonyle, di-(alkyl(C₁-C₄))aminocarbonyle, tri-(alkyl(C₁-C₂))silyle, (alkyl(C₁-C₄))(alcoxy(C₁-C₄))-imino ou représente un groupe phényle ou un cycle hétéroaromatique à 5 ou 6 chaînons, le groupe phényle ou le cycle pouvant éventuellement porter un ou plusieurs substituants identiques ou différents alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogéno-cycloalkyle(C₃-C₆), halogéno, CN, NO₂, OH, alcoxy en C₁-C₄, halogénoalcoxy(C₁-C₄),
R¹⁰ ne représentant pas un atome d'hydrogène lorsque A représente -R⁸-C(=O)-R⁸ et Q représente un groupe phényle,
R⁸ représentant un groupe -(alkylène(C₁-C₆))-linéaire ou ramifié ou une liaison directe, plusieurs radicaux R⁸ représentant indépendamment les uns des autres un groupe -(alkylène(C₁-C₆))-linéaire ou ramifié ou une liaison directe,
R⁶ représente un atome d'hydrogène, un groupe alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), cycloalcoxy en C₃-C₆ ou 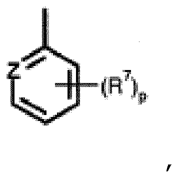
R⁷ représente chaque fois indépendamment un atome d'hydrogène, un groupe alkyle en C₁-C₆, cycloalkyle en C₃-C₆, halogénoalkyle(C₁-C₆), un atome d'halogène, un groupe cyano, nitro, alcoxy en C₁-C₄, halogénoalcoxy(C₁-C₄), alkyl(C₁-C₄)thio ou halogénoalkyl(C₁-C₄)thio,
p représente 0 à 4,
Z représente N, CH, CF, CCl, CBr ou CI,
les composés de formule générale (I) comprennent en outre les N-oxydes et sels.

2. Composés de formule générale (I) selon la revendication 1,
dans lesquels
R¹ représente un atome d'hydrogène, un groupe alkyle en C₁-C₆, alcényle en C₂-C₆, alcynyle en C₂-C₆, cycloalkyle en C₃-C₆, cyano(alkyle(C₁-C₆)), halogénoalkyle(C₁-C₆), halogénoalcényle(C₂-C₆), halogénoalcynyle(C₂-C₆), alcoxy(C₁-C₄)-alkyle(C₁-C₄), alkyl(C₁-C₄)thio-alkyle(C₁-C₄), alkyl(C₁-C₄)sulfinyl-alkyle(C₁-C₄) ou alkyl(C₁-C₄)-sulfonyl-alkyle(C₁-C₄),
R² représente un atome d'hydrogène ou un groupe alkyle en C₁-C₆,
R³ représente un atome d'hydrogène ou un groupe alkyle en C₁-C₆, alcoxy en C₁-C₆, alcényle en C₂-C₆, alcynyle en C₂-C₆ chacun portant éventuellement un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)-sulfimino-alkylcarbonyle(C₂-C₅), alkyl(C₁-C₄)-sulfoximino, alkyl(C₁-C₄)sulfoximino-alkyle(C₁-C₄), alkyl(C₁-C₄)suifoximino-alkylcarbonyle(C₂-C₅), alcoxycarbonyle(C₂-C₆), alkylcarbonyle(C₂-C₆) ou trialkyl(C₃-C₆)silyle,
R³ représente en outre un groupe cycloalkyle en C₃-C₁₂ et bicycloalkyle en C₄-C₁₀, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy (C₁-C₄), alkyl (C₁-C₄) thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)sulfimino-alkyl-carbonyle(C₂-C₅), alkyl(C₁-C₄)sulfoximino, alkyl(C₁-C₄)sulfoximino-alkyle(C₁-C₄), alkyl(C₁-C₄)-sulfoximino-alkylcarbonyle(C₂-C₅), alcoxy-carbonyle(C₂-C₆), alkylcarbonyle(C₂-C₆) ou trialkyl(C₃-C₆)silyle,
R⁴ représente un atome d'hydrogène, un groupe alkyle en C₁-C₄, halogénoalkyle(C₁-C₄), un atome d'halogène, un groupe cyano, alcoxy en C₁-C₄, halogénoalcoxy(C₁-C₄), alkyl(C₁-C₄)thio, alkyl(C₁-C₄)sulfinyle, alkyl(C₁-C₄)sulfonyle, halogénoalkyl(C₁-C₄)thio, nitro, hydroxy, amino, (C=O)OH, alkyl(C₁-C₄)sulfonyloxy, halogéno-alkyl(C₁-C₄)thio, halogénoalkyl(C₁-C₄)sulfinyle, halogénoalkyl(C₁-C₄)sulfonyle, alkyl(C₁-C₄)amino, di-(alkyl(C₁-C₄))amino, alkyl(C₁-C₄)carbonylamino, alcoxy(C₁-C₄)carbonyle, alkyl(C₁-C₄)carbonyle, aminocarbonyle, alkyl(C₁-C₄)aminocarbonyle, dialkyl(C₁-C₄)aminocarbonyle, aminothiocarbonyle, alkyl(C₁-C₄)aminothiocarbonyle, dialkyl(C₁-C₄) amino-thiocarbonyle, alkyl(C₁-C₄)sulfonylamino, amino-sulfonyle, alkyl(C₁-C₄)aminosulfonyle ou dialkyl(C₁-C₄)aminosulfonyle,
deux radicaux R⁴ voisins représentent également -(CH₂)₃-, -(CH₂)₄)-, -(CH₂)₅-, -(CH=CH-)₂-, -OCH₂O-, -O(CH₂)₂O-, -OCF₂O-, -(CF₂)₂O-, -O(CF₂)₂O-, -(CH=CH-CH=N)- ou -(CH=CH-N=CH)-,
R⁵ représente un groupe alkyle en C₁-C₄, cycloalkyle en C₁-C₆, halogénoalkyle (C₁-C₄), halogéno-cycloalkyle(C₁-C₆), alcényle en C₂-C₆, halogénoalcényle(C₂-C₄), alcynyle en C₂-C₄, halogénoalcynyle (C₂-C₄), alcoxy en C₁-C₄, halogénoalcoxy(C₁-C₄), alkyl(C₁-C₄)thio, alkyl(C₁-C₄)sulfinyle, alkyl(C₁-C₄)sulfonyle, halogénoalkyl(C₁-C₄)thio, halogénoalkyl(C₁-C₄)-sulfinyle, halogénoalkyl(C₁-C₄)sulfonyle, un atome d'halogène, un groupe cyano, nitro ou trialkyl(C₃-C₆)silyle.
A représente -C(=O)-, -CH₂(CO)-, CH₂CH(OH)-, -C(=O)-C(=O)-, -CH=CH-, -C≡ºC-, isoxazoline, imidazolidone, -CH₂NHSO₂CH₂-, -CH₂NMeSO₂CH₂-, CH₂N(SO₂Me)CH₂-, -(CO)CH₂-, CHCl-, -CCl₂-, -CHF-,-CF₂-,
Q_{z} représente un cycle à 3 ou 4 chaînons, saturé ou partiellement saturé, ou un cycle à 5 ou 6 chaînons, saturé, partiellement saturé ou aromatique, le cycle pouvant éventuellement contenir 1-3 hétéroatomes choisis dans la série constituée par N, S, O,
le cycle portant éventuellement un ou plusieurs substituants, identiques ou différents, et les substituants pouvant être choisis indépendamment les uns des autres parmi un atome d'hydrogène, des groupes alkyle en C₁-C₆, alcényle en C₂-C₆, alcynyle en C₂-C₆, cycloalkyle en C₃-C₆, halogéno-alkyle(C₁-C₆), halogénoalcényle(C₂-C₆), halogéno-alcynyle(C₂-C₆), halogénocycloalkyle(C₃-C₆), halogéno, CN, OH, alcoxy en C₁-C₄, halogénoalcoxy(C₁-C₄), alkyl(C₁-C₄)thio, alkyl(C₁-C₄)-sulfinyle, alkyl(C₁-C₄)sulfonyle, halogéno-alkyl(C₁-C₄)thio, halogénoalkyl(C₁-C₄)sulfinyle, halogénoalkyl(C₁-C₄)sulfonyle,
R⁶ représente un groupe alkyle en C₁-C₆ ou le radical 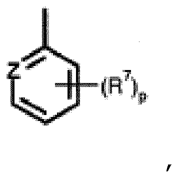
R⁶ représente en outre un groupe cycloalcoxy en C₃-C₆,
R⁷ représente chaque fois indépendamment un atome d'hydrogène, un atome d'halogène, un groupe cyano, alkyle en C₁-C₄, alcoxy en C₁-C₄, halogéno-alkyle(C₁-C₄), halogénoalcoxy(C₁-C₄), halogéno-alkyl(C₁-C₄)sulfonyle ou (alkyl(C₁-C₄))-alcoxy(C₁-C₄) imino,
p représente 1, 2 ou 3,
Z représente N, CH, CF, CCl, CBr ou CI,
R⁸ représente un groupe -(alkylène(C₁-C₄))- linéaire ou ramifié ou une liaison directe,
R¹⁰ représente un atome d'hydrogène, un groupe alkyle en C₁-C₃, halogénoalkyle(C₁-C₃), alcoxy en C₁-C₂, un atome d'halogène, un groupe cyano, hydroxy, nitro, halogénoalcoxy (C₁-C₂) ou phényle ou un cycle hétéroaromatique à 5 ou 6 chaînons, le groupe phényle ou le cycle pouvant éventuellement porter un ou plusieurs substituants identiques ou différents alkyle en C₁-C₆, alcényle en C₂-C₆, alcynyle en C₂-C₆, cycloalkyle en C₃-C₆, halogénoalkyle(C₁-C₆), halogénoalcényle(C₂-C₆), halogénoalcynyle(C₂-C₆), halogénocycloalkyle(C₃-C₆), halogéno, CN, NO₂, OH, alcoxy en C₁-C₄ ou halogénoalcoxy(C₁-C₄),
R¹⁰ ne représentant pas un atome d'hydrogène lorsque A représente -R⁸-C(=O)-R⁸ et Q représente un groupe phényle,
Q représente un cycle phényle une ou plusieurs fois substitué par R¹⁰, ou un cycle hétérocyclique saturé ou partiellement saturé ou hétéroaromatique à 5 ou 6 chaînons, ou un système cyclique hétérobicyclique aromatique condensé à 8, 9 ou 10 chaînons, les hétéroatomes pouvant être choisis dans la série N, S, O, le cycle ou le système cyclique portant éventuellement un ou plusieurs substituants R¹⁰ identiques ou différents.

3. Composés de formule générale (I) selon la revendication 1,
dans lesquels
R¹ représente un atome d'hydrogène, le groupe méthyle, cyclopropyle, cyanométhyle, méthoxy-méthyle, méthylthiométhyle, méthylsulfinylméthyle ou méthylsulfonylméthyle,
R² représente un atome d'hydrogène ou le groupe méthyle,
R³ représente un atome d'hydrogène ou un groupe alkyle en C₁-C₆, alcoxy en C₁-C₆, chacun portant éventuellement un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)sulfimino-alkyl-carbonyle(C₂-C₅), alkyl(C₁-C₄)sulfoximino, alkyl(C₁-C₄)sulfoximino-alkyle(C₁-C₄), alkyl(C₁-C₄)-sulfoximino-alkylcarbonyle(C₂-C₅), alcoxy-carbonyle(C₂-C₆), alkylcarbonyle(C₂-C₆) ou trialkyl(C₃-C₆)silyle,
R³ représente en outre un groupe cycloalkyle en C₃-C₆ portant éventuellement un ou plusieurs substituants identiques ou différents, les substituants pouvant être choisis indépendamment les uns des autres parmi halogéno, cyano, nitro, hydroxy, alkyle en C₁-C₆, cycloalkyle en C₃-C₆, alcoxy en C₁-C₄, halogénoalcoxy(C₁-C₄), alkyl(C₁-C₄)thio, alkyl(C₁-C₄)sulfinyle, alkyl(C₁-C₄)sulfonyle, alkyl(C₁-C₄)sulfimino, alkyl(C₁-C₄)sulfimino-alkyle(C₁-C₄), alkyl(C₁-C₄)-suifimino-alkylcarbonyle(C₂-C₅), alkyl(C₁-C₄)-sulfoximino, alkyl(C₁-C₄)sulfoximino-alkyle(C₁-C₄), alkyl(C₁-C₄)sulfoximino-alkylcarbonyle(C₂-C₅), alcoxycarbonyle(C₂-C₆), alkylcarbonyle(C₂-C₆) ou trialkyl(C₃-C₆)silyle,
R⁴ représente un atome d'hydrogène, un groupe alkyle en C₁-C₄, halogénoalkyle(C₁-C₂), un atome d'halogène, un groupe cyano ou halogénoalcoxy (C₁-C₂),
deux radicaux R⁴ voisins représentent -(CH₂)₄-, -(CH=CH-)₂-, -O(CH₂)₂O-, -O(CF₂)₂O-, -(CH=CH-CH=N)-ou -(CH=CH-N=CH)-,
R⁵ représente un groupe alkyle en C₁-C₄, cycloalkyle en C₃-C₆, halogénoalkyle (C₁-C₄), halogéno-cycloalkyle(C₁-C₆), alcényle en C₂-C₆, halogénoalcényle(C₂-C₄), alcynyle en C₂-C₄, halogénoalcynyle(C₂-C₄), alcoxy en C₁-C₄, halogénoalcoxy(C₁-C₄), un atome de fluor, de chlore, de brome, d'iode, un groupe cyano, nitro ou trialkyl(C₃-C₆)silyle.
A représente -C(=O)-, -CH₂(CO)-, CH₂CH(OH)-, -C(=O)-C(=O)-, -CH=CH-, -C≡ºC-, isoxazoline, imidazolidone, -CH₂NHSO₂CH₂-, -CH₂NMeSO₂CH₂-, CH₂N(SO₂Me)CH₂-, -(CO)CH₂-, CHCl-, -CCl₂-, -CHF-, -CF₂-,
Q_{z} représente un cycle à 3 ou 4 chaînons, saturé ou partiellement saturé, ou un cycle à 5 ou 6 chaînons, saturé, partiellement saturé ou aromatique, le cycle pouvant éventuellement contenir 1-2 hétéroatomes choisis dans la série constituée par N, S, O,
le cycle portant éventuellement un ou plusieurs substituants, identiques ou différents, et les substituants pouvant être choisis indépendamment les uns des autres parmi un atome d'hydrogène, des groupes alkyle en C₁-C₆, alcényle en C₂-C₆, alcynyle en C₂-C₆, cycloalkyle en C₃-C₆, halogéno-alkyle(C₁-C₆), halogénoalcényle(C₂-C₆), halogéno-alcynyle(C₂-C₆), halogénocycloalkyle(C₃-C₆), halogéno, CN, OH, alcoxy en C₁-C₄, halogénoalcoxy(C₁-C₄), alkyl(C₁-C₄) thio, alkyl(C₁-C₄)-sulfinyle, alkyl(C₁-C₄)sulfonyle, halogéno-alkyl(C₁-C₄)thio, halogénoalkyl(C₁-C₄)sulfinyle, halogénoalkyl(C₁-C₄)sulfonyle,
R⁶ représente le groupe méthyle ou le radical 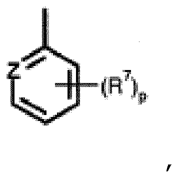
R⁷ représente chaque fois indépendamment un atome d'hydrogène, un atome d'halogène ou un groupe halogénoalkyle(C₁-C₄),
p représente 1 ou 2,
Z représente N, CH, CF, CCl ou CBr,
R⁸ représente le groupe méthylène, éthylène, propylène, isopropylène, n-butylène, sec-butylène, ou isobutylène ou une liaison directe,
R¹⁰ représente un atome d'hydrogène, un groupe alkyle en C₁-C₃, halogénoalkyle(C₁-C₃), alcoxy en C₁-C₂, un atome d'halogène, un groupe cyano, hydroxy, halogénoalcoxy (C₁-C₂) ou phényle ou un cycle hétéroaromatique à 5 ou 6 chaînons, le groupe phényle ou le cycle pouvant éventuellement porter un ou plusieurs substituants identiques ou différents alkyle en C₁-C₃, alcényle en C₂-C₄, alcynyle en C₂-C₄, cycloalkyle en C₃-C₆, halogénoalkyle(C₁-C₃), halogénoalcényle(C₂-C₄), halogénoalcynyle(C₂-C₄), halogénocycloalkyle(C₃-C₆), halogéno, cyano, alcoxy en C₁-C₄, halogéno-alcoxy(C₁-C₄),
R¹⁰ ne représentant pas un atome d'hydrogène lorsque A représente -R⁸-C(=O)-R⁸ et Q représente un groupe phényle,
Q représente un cycle phényle une ou plusieurs fois substitué par R¹⁰, ou un cycle hétéroaromatique à 5 ou 6 chaînons, de la série Q-1 à Q-53 et Q-58 et Q-59, Q-62 et Q-63, éventuellement une ou plusieurs fois substitué par R¹⁰, un système cyclique hétérobicyclique aromatique condensé à 9 chaînons Q-54 à Q-56 ainsi qu'un cycle hétérocyclique à 5 chaînons Q-60 et Q-61,

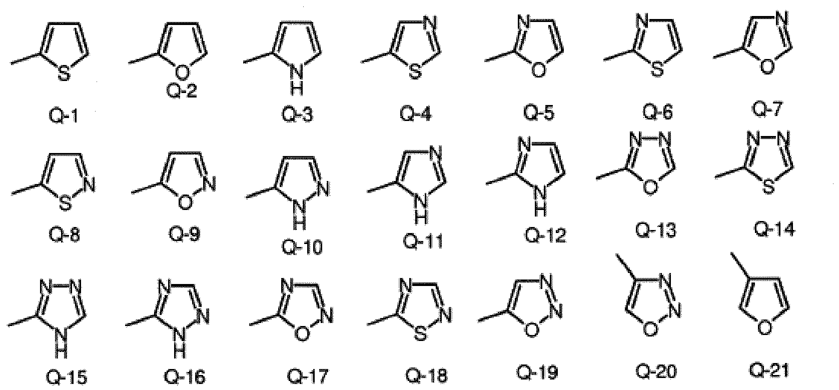

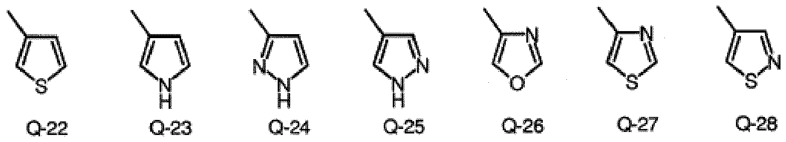

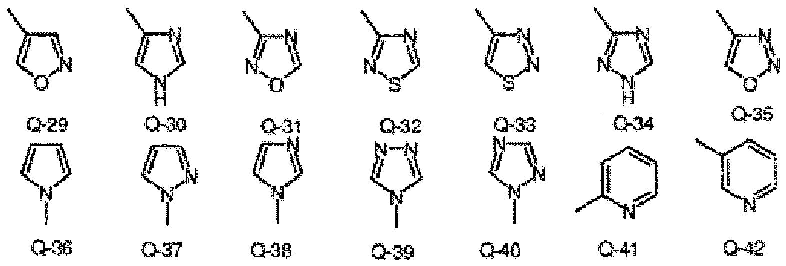

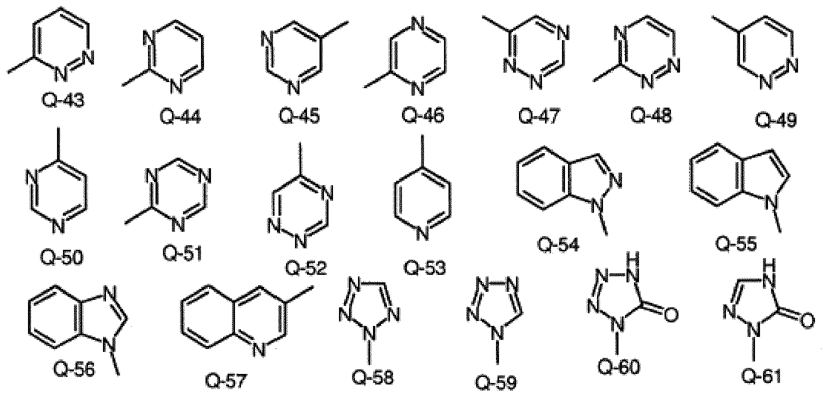

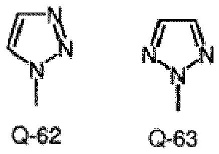

4. Mélanges de composés de formule générale (I) selon l'une quelconque des revendications 1 à 3, dans lesquels Q représente Q62 et Q63, le rapport d'un composé de formule (I), dans lequel Q représente Q62, à un composé de formule (I), dans lequel Q représente Q63, valant de 60:40 à 99:1.

5. Mélanges de composés de formule générale (I) selon l'une quelconque des revendications 1 à 3, dans lesquels Q représente Q58 et Q59, le rapport d'un composé de formule (I), dans lequel Q représente Q58, à un composé de formule (I), dans lequel Q représente Q59, valant de 60:40 à 99:1.

6. Procédé pour la préparation de composés de formule générale (I) selon l'une quelconque des revendications 1 à 3, **caractérisé en ce qu'**on fait réagir
(A) des anilines de formule (II) 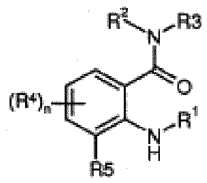 dans laquelle R¹, R², R³, R⁴, R⁵ et n ont les significations indiquées plus haut,
par exemple avec un acide carboxylique de formule (III) 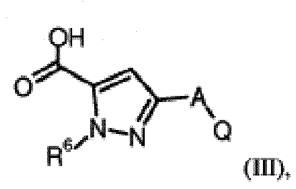 dans laquelle Q, A et R⁶ ont les significations indiquées plus haut,
en présence d'un agent de condensation ; ou
(B) des anilines de formule (II) 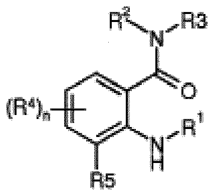 dans laquelle R¹, R², R³, R⁴, R⁵ et n ont les significations indiquées plus haut,
par exemple avec des chlorures d'acyle de formule (IIIA) 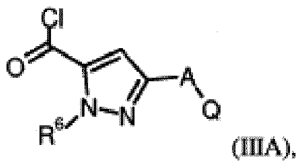 dans laquelle Q, A et R⁶ ont les significations indiquées plus haut,
en présence d'un capteur d'acide ; ou
(C) pour la synthèse d'anthranilamides de formule (I) dans laquelle R¹ représente un atome d'hydrogène, on fait réagir par exemple des benzoxazinones de formule (IIIB) 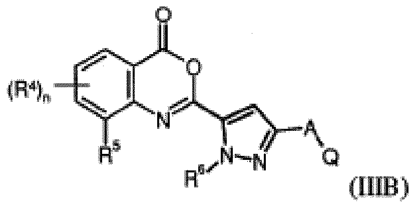 dans laquelle R⁴, R⁵, R⁶, A, Q et n ont les significations indiquées plus haut,
avec une amine de formule (IIIC) 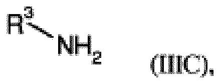 dans laquelle R³ a les significations indiquées plus haut,
en présence d'un diluant, pour aboutir aux composés de formule (I) selon l'invention.

7. Compositions contenant au moins un composé de formule (I) ou un mélange de composés de formule (I) selon l'une quelconque des revendications 1 à 5, ainsi qu'au moins un sel de formule (XXIV) 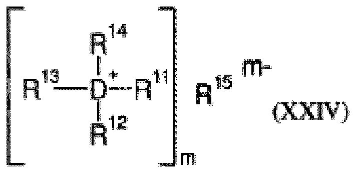 dans laquelle
D représente un atome d'azote ou de phosphore,
R¹¹, R¹², R¹³ et R¹⁴ représentent indépendamment les uns des autres un atome d'hydrogène ou un groupe alkyle en C₁-C₈ chaque fois éventuellement substitué ou alkylène en C₁-C₈ une ou plusieurs fois insaturé, éventuellement substitué, les substituants pouvant être choisis parmi halogéno, nitro et cyano,
m représente 1, 2, 3 ou 4,
R¹⁵ représente un anion organique ou inorganique.

8. Compositions contenant au moins un composé de formule (I) ou un mélange de composés de formule (I) selon l'une quelconque des revendications 1 à 5, ainsi qu'au moins un agent favorisant la pénétration, de formule (XXV)
R-O-(-AO)ᵥ-R' (XXV),
dans laquelle
R représente un groupe alkyle à chaîne droite ou ramifié ayant de 4 à 20 atomes de carbone,
R' représente un atome d'hydrogène, le groupe méthyle, éthyle, n-propyle, isopropyle, n-butyle, isobutyle, tert-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène et
V représente des nombres de 2 à 30.

9. Compositions agrochimiques contenant au moins un composé de formule (I) ou un mélange de composés de formule (I) selon l'une quelconque des revendications 1 à 5 ou une composition selon la revendication 7 ou 8, ainsi que des excipients et/ou des substances tensioactives.

10. Procédé pour la préparation de compositions agrochimiques, **caractérisées en ce qu'**on mélange au moins un composé de formule générale (I) ou un mélange de composés de formule générale (I) selon l'une quelconque des revendications 1 à 5 ou une composition selon la revendication 7 ou 8 avec des excipients et/ou des substances tensioactives.

11. Utilisation d'un composé de formule générale (I) ou d'un mélange de composés selon l'une quelconque des revendications 1 à 5 ou d'une composition selon l'une quelconque des revendications 7 à 9, pour la lutte contre des ravageurs animaux, à l'exclusion du traitement thérapeutique de l'organisme humain ou animal.

12. Procédé pour la lutte contre des ravageurs animaux à l'exclusion du traitement thérapeutique de l'organisme humain ou animal, **caractérisé en ce qu'**on fait agir sur des ravageurs animaux et/ou des champignons phytopathogènes et/ou leur habitat et/ou des semences un composé de formule générale (I) ou un mélange de composés de formule générale (I) selon l'une quelconque des revendications 1 à 5 ou une composition selon l'une quelconque des revendications 7 à 9.
